Europäisches Patentamt

European Patent Office

Office européen des brevets

(19)

(11) Veröffentlichungsnummer: **0 317 511**
**A2**

(12) **EUROPÄISCHE PATENTANMELDUNG**

(21) Anmeldenummer: 88810767.9

(22) Anmeldetag: 09.11.88

(51) Int. Cl.⁴: **C 12 N 5/00**
A 01 H 1/00, C 12 N 15/00,
A 01 N 65/00

(30) Priorität: 18.11.87 US 122109

(43) Veröffentlichungstag der Anmeldung:
24.05.89 Patentblatt 89/21

(84) Benannte Vertragsstaaten:
AT BE CH DE ES FR GB GR IT LI LU NL SE

(71) Anmelder: CIBA-GEIGY AG
Klybeckstrasse 141
CH-4002 Basel (CH)

(72) Erfinder: Rice, Douglas
137 Pinecrest Road
Durham North Carolina 27705 (US)

Carozzi, Nadine
Route 6, Box 348P
Raleigh North Carolina 27612 (US)

Lotstein, Richard
1619 Delaware Street
Durham North Carolina 27705 (US)

de Framond, Annick
2422 West Club Blvd.
Durham North Carolina 27705 (US)

Anderson, David M.
1366 E. Skywood Circle
Altadena California 91001 (US)

Rajasekaran, Kanniah
116C Esperanza Avenue
Sierra Madre California 91024 (US)

Rangan, Thirumale S.
2330 E. Del Mar Bl No. 201
Pasadena California 91107 (US)

Yenofsky, Richard
628 W. Norman Avenue
Arcadia California 91006 (US)

Der Anmelder hat eine Erklärung nach Regel 28 (4) EPÜ (Herausgabe einer Probe nur an einen Sachverständigen) eingereicht.
Eingangsnummer(n) der Hinterlegung(en): ATCC 67329, ATCC 67330, ATCC 40235, ATCC 40486, ATCC 40487.

(54) Insektizide Baumwollpflanzenzellen.

(57) Gegenstand der vorliegenden Erfindung ist ein chimäres Gen, das in den Zellen von Baumwollpflanzen insektizid wirksame Verbindungen exprimiert, die im wesentlichen die Insekten-toxischen Eigenschaften des von *Bacillus thuringiensis* produzierten kristallinen Proteins besitzen.
Die transformierten Zellen werden zu kompletten Pflanzen regeneriert, die toxisch sind gegenüber Insektenlarven aus den Ordnungen *Lepidoptera, Coleoptera* und *Diptera*.

FIG.1

EP 0 317 511 A2

**Beschreibung**

### Insektizide Baumwollpflanzenzellen

Gegenstand der vorliegenden Erfindung ist ein chimäres Gen, das in den Zellen von Baumwollpflanzen insektizid wirksame Verbindungen exprimiert, die im wesentlichen die Insekten-toxischen Eigenschaften des von *Bacillus thuringiensis* produzierten kristallinen Proteins besitzen.

*B. thuringiensis* (im Folgenden mit *Bt* bezeichnet) ist eine Bakterien-Spezies, die ein kristallines Protein produziert, das auch als δ-Endotoxin bezeichnet wird. Technisch gesehen handelt es sich bei diesem kristallinen Protein um ein Protoxin, das in ein Toxin umgewandelt wird, wenn es den Verdauungstrakt von *Lepidoptera-, Coleoptera-* und *Diptera*-Larven (Insekten) passiert.

Das kristalline Protein von *Bt* ist ein potentiell wichtiges Insektizid, das, soweit bekannt, keine schädlichen Auswirkungen auf Menschen, andere Säugetiere, Vögel, Fische oder auf Insekten, - mit Ausnahme der Larven von Lepidopteren, Coleopteren und Dipteren -, hat. Die Aktivität des *Bt* Toxins ist ausserordentlich hoch, sodass bereits wenige Nanogramm dieser Substanz ausreichen, um empfindliche Insektenlarven abzutöten. Weitere Vorteile, die für eine Verwendung des kristallinen Proteins von *Bt* als Insektizid sprechen, sind dessen breites Aktivitätsspektrum gegen *Lepidoptera-, Diptera-* und *Coleoptera*-Larven sowie die offensichtliche Schwierigkeit für diese Larven, Resistenzen gegen das kristalline Protein zu entwickeln. Dies trifft auch dort zu, wo das kristalline Protein in grossem Massstab eingesetzt wird.

Die zuvor erwähnten Insektenlarven stellen ein schwerwiegendes Problem für die Land- und Forstwirtschaft dar, insbesondere was die Kultivierung von Baumwolle betrifft.

Das kristalline Protein entwickelt seine insektizide Wirksamkeit, wenn es auf Pflanzen appliziert wird, die mit Lepidopteren-, Coleopteren- oder Dipterenlarven befallen sind. Zu diesen Pflanzen gehören beispielsweise Brokkoli, Salat und Baumwolle. In Baumwollkulturen stellt insbesondere der Befall mit Lepidopterenlarven ein besonders schwerwiegendes Problem dar.

Bisher wurde das *Bt* Kristallprotein (Protoxin) direkt aus dem Bakterium isoliert und mit Hilfe von Standardmethoden, wie Bestäuben oder Besprühen, auf die Pflanzen appliziert.

Präparate, die das *Bt* Kristallprotein enthalten, werden kommerziell als biologische Insektizide vertrieben.

Als Beispiele seien aufgeführt:

Bactospeine, vertrieben durch Biochem Products Ltd.; Dipel, vertrieben durch Abbot Laboratories; und Thurcide, vertrieben durch Sandoz AG.

Die Tatsache, dass *Bt* das Kristallprotein nur während der Sporulationsphase produziert, stellt einen schwerwiegenden Nachteil dar, was die industrielle Herstellung und Verwendung dieses biologischen Insektizids betrifft.

Im Verlaufe eines industriellen Fertigungsprozesses können solche in Zusammenhang mit einer bestimmten Wachstumsphase stehenden Beschränkungen zu Schwierigkeiten und Zeitverzögerungen während der Herstellung führen.

Hinzu kommt, dass es die bei einem solch komplexen Herstellungsverfahren anfallenden Kosten für ein biologisches Insektizid schwierig machen, mit anderen wirksamen und kommerziell erhältlichen Produkten, die auf definierten chemischen Verbindungen basieren, wie z.B. die Pyrethroid-Derivate, zu konkurrieren.

Ein weiterer Nachteil bei der Verwendung des *Bt* Toxins ist beispielsweise durch die Tatsache gegeben, dass das Protoxin auf der Oberfläche der behandelten Pflanzen verbleibt, wo es nur gegen an der Oberfläche fressende Larven wirkt und wo es durch anhaltende ultraviolette Bestrahlung inaktiviert wird. Diese Inaktivierung dürfte wenigstens eine der Ursachen für die generell fehlende Persistenz des kristallinen Proteins in der Umwelt sein. Dementsprechend ist eine häufige und kostspielige Applikation des kristallinen Proteins notwendig.

Diese und andere Nachteile können dadurch überwunden werden, dass man ein Gen, welches ein *Bt* Kristallprotein oder aber ein Protein kodiert, das im wesentlichen die Insektentoxizitätseigenschaften des *Bt* Kristallproteins aufweist, in Pflanzen einschleust und dort exprimiert.

Die zuvor genannten Nachteile können gemäss der vorliegenden Erfindung dadurch überwunden werden, dass man ein Gen, welches ein *Bt* Kristallprotein oder aber ein Protein kodiert, das im wesentlichen die Insektentoxizitätseigenschaften des *Bt* Kristallproteins aufweist, in Baumwollpflanzen einschleust, dort exprimiert und anschliessend aus den transformierten Protoplasten fertile transgene Baumwollpflanzen regeneriert und diese Insekten-resistenten Baumwollpflanzen kultiviert.

Mit den Verfahren der rekombinanten DNA-Technologie ist es möglich, ein Gen, das für die Produktion eines nützlichen Polypeptids verantwortlich ist, von einer Donorzelle, in welcher das Gen natürlicherweise vorkommt, in eine Empfängerzelle zu transferieren, in welcher das Gen natürlicherweise nicht vorkommt (US Patente 4,237,224 und 4,468,464). Es gibt tatsächlich nur wenige inhärente Grenzen für solche Genübertragungen. So können Gene beispielsweise zwischen Viren, Bakterien, Pflanzen und Tieren übertragen werden. In einigen Fällen ist das transferierte Gen in der Empfängerzelle funktionstüchtig oder kann funktionstüchtig gemacht werden. Wenn die Empfängerzelle eine Pflanzenzelle ist, können ganze Pflanzen aus der Zelle regeneriert werden.

Gene bestehen typischerweise aus DNA-Sequenzabschnitten, die einen Promotor und einen transkribierten Abschnitt enthalten. Der transkribierte Abschnitt enthält normalerweise eine 5′-nichttranslatierte Region, eine kodierende Sequenz und eine 3′-nichttranslatierte Region.

Der Promotor enthält die DNA-Sequenz, die zur Initiation der Transkription notwendig ist, in deren Verlauf die transkribierte Region in mRNA übersetzt wird. Man nimmt an, dass der Promotor in eukaryotischen Zellen einen Abschnitt enthält, der von der RNA-Polymerase erkannt wird, sowie einen weiteren Abschnitt, der die RNA-Polymerase an die für die Initiation der Transkription geeignete Stelle auf der DNA dirigiert. Dieser letztere Abschnitt wird als die TATA-Box bezeichnet. Die TATA-Box befindet sich gewöhnlicherweise etwa 30 Nukleotide stromaufwärts ('up-stream') der Transkriptionsinitiationsstelle.

An den Promotorabschnitt schliesst sich eine Sequenz an, die in mRNA transkribiert, aber nicht in ein Polypeptid translatiert wird. Diese Sequenz stellt die sogenannte 5'-nichttranslatierte Region dar, und man nimmt an, dass sie Sequenzen enthält, die für die Initiation der Translation verantwortlich sind, wie z.B. eine Ribosomenbindungsstelle.

Als kodierenden Abschnitt bezeichnet man diejenige Sequenz, die sich unmittelbar stromabwärts ('downstream') der 5'-nichttranslatierten Region in der DNA oder der entsprechenden RNA befindet. Der kodierende Abschnitt wird entsprechend dem genetischen Kode in Polypeptide translatiert. Beispielsweise besitzt *Bt* ein Gen mit einer kodierenden Sequenz, die in die Aminosäuresequenz des insektiziden kristallinen Proteins translatiert wird.

Dem kodierenden Abschnitt folgt stromabwärts eine Sequenz, von der zwar mRNA transkribiert, aber kein Polypeptid translatiert wird. Diese Sequenz wird die 3'-nichttranslatierte Region genannt, und man nimmt an, dass sie ein Signal enthält, das zur Termination der Transkription führt. In eukaryotischer mRNA liegt in diesem Bereich ein weiteres Signal vor, das die Polyadenylierung des transkribierten mRNA-Strangs bewirkt. Es wird angenommen, dass die Polyadenylierung der mRNA Processing- und Transportfunktionen erfüllt.

Natürliche Gene können als eine Einheit von einer Donorzelle auf eine Empfängerzelle übertragen werden. Es ist jedoch oft vorteilhaft, ein Gen zu konstruieren, das die gewünschte kodierende Region sowie einen Promotor und gegebenenfalls 5'- und 3'-nichttranslatierte Abschnitte enthält, wobei der Promotor sowie die 5'- und 3'-nichttranslatierten Regionen natürlicherweise nicht im selben Gen vorkommen wie der kodierende Abschnitt. Solche Konstrukte werden als chimäre Gene bezeichnet.

Es sind bereits gentechnologische Verfahren beschrieben worden, die zu einer Verbesserung bei der Herstellung kristalliner Proteine geführt haben. Beispielsweise werden in den US Patenten 4,448,885 und 4,467,036 Plasmide beschrieben, die für die Herstellung des kristallinen Proteins in anderen Bakterienstämmen als *Bt* geeignet sind. Diese Verfahren erlauben zwar die Herstellung des kristallinen Proteins, sie beseitigen aber nicht die Nachteile, die bei der kommerziellen Verwendung des kristallinen Proteins als Insektizid auftreten.

Es wurden mittlerweile auch Vorschläge gemacht, *Bt* Toxingene direkt in Pflanzen zu klonieren, um den Pflanzen dadurch die Möglichkeit zu geben, sich selbst zu schützen (Klausner, 1984). In der Europäischen Patentanmeldung 142,924 (Agrigenetics) wird ein Verfahren zur Klonierung von *Bt* Toxingenen in Tabak beschrieben (seite 59) und vorgeschlagen, Baumwolle auf die gleiche Weise zu schützen (Seite 77).

Dieser Vorschlag muss jedoch als rein spekulativ angesehen werden, solange keine Verfahren zur Transformation von Baumwollzellen sowie zur Regeneration ganzer Pflanzen aus diesen Zellen zur Verfügung stehen.

Solche Verfahren sind in der US-Patentanmeldung Nr. 122,200 der Firma Phytogen mit dem Titel "Regeneration und Transformation of Cotton" sowie der US-Patentanmeldung Nr. 122,162 der Firma CIBA GEIGY Corp. mit dem Titel "An Efficient Method for Regenerating Cotton from Cultured Cells" beschrieben. Diese beiden Patentanmeldungen wurden am gleichen Tag hinterlegt wie die vorliegende Anmeldung.

Die Transformationsverfahren aus der Phytogen US-Patentanmeldung Nr. 122,200 sowie die Verfahren zur Regeneration von Baumwollpflanzen in den Phytogen bzw. CIBA-GEIGY US-Patentanmeldungen Nr. 122,200 bzw. Nr. 122,162 sind integrale Bestandteile der vorliegenden Anmeldung.

Es muss daher als eine vordringliche Aufgabe angesehen werden, neue Verfahren für die Herstellung eines *Bt* Kristallproteins oder eines vergleichbaren Proteins, das im wesentlichen die Insektentoxizitätseigenschaften (insektizide Aktivität) des *Bt* Kristallproteins aufweist, in Zellen von Baumwollpflanzen zu entwickeln sowie von Verfahren zur Kontrolle von Insektenlarven, die sich von besagten Baumwollpflanzen ernähren. Unter "Kontrolle" soll im Rahmen dieser Erfindung die Abtötung der Larven oder aber zumindest eine substantielle Reduktion der Nahrungsaufnahme verstanden werden.

Eine weitere vordringliche Aufgabe besteht in der Bereitstellung von Verfahren zum Schutz von Baumwollpflanzen vor Schäden, die durch Schädlinge oder Pathogene verursacht werden. Diese Verfahren beruhen auf der Herstellung eines pestizid wirksamen oder antipathogenen Proteins in der pflanzlichen Zelle, respektive der ganzen Pflanze und zwar in einer Konzentration, die ausreicht, um den Schädling oder das Pathogen effektiv abzutöten oder unter Kontrolle zu halten.

Insbesondere besteht die Aufgabe zur Bereitstellung eines Verfahrens zum Schutz von Baumwollpflanzen gegen Schäden, die von Insekten verursacht werden. Es handelt sich dabei um ein Verfahren, das auf der Herstellung eines *Bt* Kristallproteins oder eines vergleichbaren Proteins, das im wesentlichen die Insektentoxizitätseigenschaften des *Bt* Kristallproteins aufweist, in der pflanzlichen Zelle beruht und zwar in einer Konzentration, die ausreicht, diejenigen Schadinsekten, die sich von diesen Pflanzen ernähren, abzutöten oder aber zumindest unter Kontrolle zu halten.

Eine weitere Aufgabe besteht in der Bereitstellung eines Verfahrens zum Schutz von Baumwollpflanzen vor negativen Einflüssen durch chemische Agentien, wie z.B. Erhöhung der Tolerabilität in bezug auf bestimmte Herbizide, damit diese in Zukunft in Baumwollkulturen breitere Anwendung finden können, mit minimalen Nebenwirkungen für das Oekosystem.

Die vorliegende Erfindung betrifft daher in erster Linie transgene Baumwollzellen, die ein chimäres Gen stabil in das Pflanzengenom integriert enthalten, das zur Expression eines Polypeptides führt, welches im wesentlichen die Insektentoxizitätseigenschaften des *Bt* Kristallproteins aufweist.

Ebenso von der vorliegenden Erfindung umfasst sind transgene Baumwollpflanzen, die aus transgenen Baumwollzellen (Protoplasten) regeneriert werden können und die besagtes chimäres Gen stabil in ihr Genom eingebaut enthalten, das die Pflanze im Zuge der Genexpression unattraktiv und/oder toxisch macht für Insektenlarven, wodurch diese vor Insektenfrass geschützt sind.

Ein weiterer Gegenstand dieser Erfindung betrifft das Vermehrungsgut und die Nachkommenschaft besagter transgener Baumwollpflanzen.

Unter Vermehrungsgut transgener Baumwollpflanzen soll im Rahmen dieser Erfindung jedes beliebige pflanzliche Material verstanden werden, das sich auf sexuellem oder asexuellem Wege bzw. in-vivo oder in-vitro vermehren lässt. Als besonders bevorzugt sind im Rahmen dieser Erfindung in erster Linie Protoplasten, Zellen, Kalli, Gewebe, Organe, Samen, Embryonen, Pollen, Eizellen, Zygoten anzusehen sowie jedes beliebige andere Vermehrungsgut, das von transgenen Baumwollpflanzen erhalten werden kann. Ebenso umfasst von der vorliegenden Erfindung ist die Nachkommenschaft besagter transformierter Baumwollzellen oder -pflanzen. Diese umfasst auch Mutanten und Varianten, die mit Hilfe bekannter Verfahren, wie beispielsweise durch Zellfusionen oder Mutantenselektion, gewonnen werden können und die noch die charakteristischen Eigenschaften des Ausgangsmaterials aufweisen, welche diese aufgrund der Transformation mit exogener DNA erhalten hat.

Wie aus der nachfolgenden detaillierten Beschreibung der Erfindung ersichtlich wird, konnten diese und andere Aufgaben der vorliegenden Erfindung überraschenderweise durch die Bereitstellung von chimären Genen, die in der Lage sind, in Baumwollzellen ein Polypeptid zu exprimieren, welches im wesentlichen die Insektentoxizität des *Bt* Kristallproteins (von nun an chimäres *Bt* Toxingen genannt) aufweist, gelöst werden.

Ein weiterer Gegenstand der vorliegenden Erfindung betrifft daher in erster Linie ein Verfahren zur Herstellung eines Toxins in den Zellen von Baumwollpflanzen, das im wesentlichen die Insektentoxizität des kristallinen Proteins von *Bt* aufweist. Besonders bevorzugt ist ein Verfahren zur

a) Rückgewinnung der Zellen sowie von jeglichem embryogenen Kallus aus dem Kallus-Wachstumsmedium;

b) Resuspendierung der Zellen und des embryogenen Kallus in einem Kallus-Wachstumsmedium, das einen *Agrobacterium*-Vektor enthält, der ein Gen besitzt, welches Baumwollzellen eine Resistenz gegenüber dem Antibiotikum Hygromycin verleiht, wobei die Bedingungen für ein Wachstum in der Suspension für einen Zeitraum aufrechterhalten werden, der für die Transformation der suspendierten Zellen ausreicht;

c) Rückgewinnung der suspendierten Zellen aus dem *Agrobacterium*-haltigen Kallus-Wachstumsmedium;

d) Behandlung der transformierten Zellen und des embryogenen Kallus mit einem Antibiotikum in einer Konzentration, die ausreicht, die Agrobakterien abzutöten;

e) in Kontakt bringen der Zellen und des embryogenen Kallus mit dem Antibiotikum Hygromycin zur Selektionierung der transformierten Zellen und des transformierten embryogenen Kallus;

f) Filtrieren der Suspension zur Entfernung von embryogenem Kallus, der eine Grösse von ca. 600 Micron (μm) überschreitet.

Ein weiterer Gegenstand der vorliegenden Erfindung betrifft ein Verfahren zur Abtötung oder zur Kontrolle von Insektenlarven, die sich von Baumwollpflanzenzellen ernähren, die chimäre Gene enthalten, welche eine insektizide Menge eines Toxins exprimieren, das im wesentlichen die Insektentoxizität des kristallinen Proteins von *Bt* aufweist. Unter insektiziden Baumwollpflanzenzellen sind erfindungsgemäss solche von ganzen Pflanzen sowie von Teilen von Pflanzen zu verstehen sowie einzelne Baumwollzellen in Kultur.

Ein weiterer Gegenstand dieser Erfindung betrifft ein Verfahren zum Schutz von Baumwollpflanzen vor Insektenfrass, das sich dadurch kennzeichnet, dass man innerhalb der die Pflanze aufbauenden Pflanzenzellen ein *Bt* Kristallprotein oder aber ein Protein, das im wesentlichen die Insektentoxizitätseigenschaften des *Bt* Kristallproteins besitzt, in einer Menge exprimiert, die ausreicht, die Insektenlarven abzutöten oder aber zumindest diese unter Kontrolle zu halten.

Besonders bevorzugt ist ein Verfahren zum Schutz von Baumwollpflanzen vor Schäden, die durch Lepidopterenlarven verursacht werden.

Ebenfalls umfasst von der vorliegenden Erfindung ist ein Verfahren, das es erlaubt, das *Bt* Kristallprotein oder aber ein Protein, das im wesentlichen die Insektentoxizitätseigenschaften des *Bt* Kristallproteins besitzt, in einer Menge zu produzieren, die ausreicht, die Pflanze unattraktiv und/oder toxisch zu machen für Insektenlarven, wodurch diese vor Insektenfrass geschützt ist.

Ein weiterer Gegenstand der vorliegenden Erfindung betrifft die Bereitstellung von Genen und anderen DNA-Segmenten sowie von Zellen und Pflanzen, die mit den zuvor näher bezeichneten Verfahren in Zusammenhang stehen.

Weitere Gegenstände der vorliegenden Erfindung umfassen das chimäre *Bt* Toxingen in Vektoren, Bakterien, Pflanzenzellen in Kultur und als Bestandteil lebender Pflanzen sowie Verfahren für die Herstellung eines Toxins, das im wesentlichen die Insektentoxizität des *Bt* Kristallproteins aufweist, in Baumwollzellen sowie Verfahren zum Schutz von Baumwollpflanzen vor Schäden, die durch Insekten verursacht werden, die

4

auf der Herstellung in der pflanzlichen Zelle eines *Bt* Kristallproteins oder eines vergleichbaren Proteins, das im wesentlichen die Insektentoxizitätseigenschaften des *Bt* Kristallproteins aufweist, basiert.

Abbildungen:

Fig. 1: Konstruktion von mp 19/bt, einem Plasmid, welches das 5'-Ende des *Bt* Protoxingens enthält.

Fig. 2: Konstruktion von mp 19/bt ca/del, einem Plasmid, welches den Promotor des CaMV Gens VI in Verknüpfung mit dem 5' Ende der *Bt* Protoxin-kodierenden Region enthält.

Fig. 3: Konstruktion von p7O2/bt, einem Plasmid, welches die 3'-kodierende Region des Protoxins in Verknüpfung mit den Transkriptions/Terminationssignalen von CaMV enthält.

Fig. 4: Konstruktion von pBR322/bt 14, welches die komplette kodierende Region des Protoxins enthält, flankiert von den CaMV Promotor-und Terminatorsequenzen.

Fig. 5: Konstruktion von pRK252/Tn903/BglII.

Fig. 6: Konstruktion von pCIB5.

Fig. 7/8: Konstruktion von pCIB4.

Fig. 9: Konstruktion von pCIB2.

Fig. 10: Konstruktion von pCIB10, einem Plasmid mit breitem Wirtsspektrum, das die T-DNA Grenzsequenzen sowie ein Gen zur Selektion transformierter Pflanzen enthält.

Fig. 11: Konstruktion von pCIB10/19Sbt.

Fig. 12: Konstruktion von pCIB710.

Fig. 13: Konstruktion von pCIB10/710.

Fig. 14: Konstruktion von pCIB10/35Sbt.

Fig. 15: Konstruktion von pCIB10/35Sbt(KpnI).

Fig. 16: Konstruktion von pCIB10/35Sbt(BclI).

Fig. 17: Konstruktion von pCIB10/35Sbt(607).

Fig. 18: nicht existent.

Fig. 19: Konstruktion von pCIB1300, einem Plasmid, das ein chimäres Gen mit dem 35S CaMV Promotor, dem CaMV-Leader, der *Bt*(Bc1)Deletion und dem 35S Terminator enthält.

Fig. 20, 21 und 22: Konstruktion von pCIB1301, einem Plasmid, das ein chimäres Gen mit dem rbc-gX Promotor aus Baumwolle und der *Bt*-Toxin (607 Deletion) kodierenden Sequenz enthält.

Fig. 23: Konstruktion von pCIB1302, einem Plasmid, das ein chimäres Gen mit dem rbc-gY Promotor aus Baumwolle und der *Bt*-Toxin (607 Deletion) kodierenden Sequenz enthält.

Fig. 24: Restriktionskarte der rbc-gX und rbc-gY enthaltenden Baumwoll-Klone

Fig. 25: Nukleotid- und Aminosäuresequenz von rbc-gY. Das erste ATG und Methionin des Transitpeptides sind in der Abbildung eingerahmt.

Fig. 26: Nukleotid- und Aminosäuresequenz von rbc-gX. Das erste ATG und Methionin des Transitpeptides sind in der Abbildung eingerahmt.

Hinterlegung:

Im Zusammenhang mit der vorliegenden Erfindung wurden die im folgenden aufgelisteten Plasmide und/oder Mikroorganismen gemäss den Bestimmungen des Budapester Vertrages bei der als Internationale Hinterlegungsstelle anerkannten "American Type Culture Collection" in Rockville, Maryland hinterlegt.

1) *Escherichia coli* MC1061, pCIB10/35Sbt (Hinterlegungsdatum: 27. Februar 1987)    ATCC 67329

2) *Escherichia coli* HB101, pCIB 10/19Sbt (Hinterlegungsdatum: 27. Februar 1987)    ATCC 67330

3) Plasmid pLV111 (Hinterlegungsdatum: 14. Mai 1986)    ATCC 40235

4) Phage λ/rbc-gY (Hinterlegungsdatum: 25. August 1988)    ATCC 40486

5) Phage λ/rbc-gX (Hinterlegungsdatum: 25. August 1988)    ATCC 40487

Der Hauptgegenstand der vorliegenden Erfindung betrifft die Herstellung einer chimären transformierten Baumwollzelle oder -pflanze, die das *Bt* Toxingen enthält.

Die für die Verwendung im Rahmen dieser Erfindung in Frage kommenden Baumwollpflanzenzellen schliessen die Zellen von jeder beliebigen Baumwollpflanze ein, in welche Fremd-DNA eingeführt und in denen sie repliziert und exprimiert werden kann. Geeignete Baumwollpflanzen umfassen beispielsweise die folgenden Spezies: *Gossypium hirsutum, Gossypium arboreum* und *Gossypium barbadense.*

Diese beispielhafte Aufzählung dient lediglich der Verdeutlichung der vorliegenden Erfindung und soll den Erfindungsgegenstand in keiner Weise limitieren.

Bevorzugt im Rahmen dieser Erfindung ist die Baumwollspezies *Gossypium hirsutum*, die wahlweise dem "Stripper" oder dem "Picker" - Typ angehören kann.

Baumwollsorten vom "Picker" und vom "Stripper"-Typ unterscheiden sich in ihrem Ernteverhalten. Die Kapseln der "Stripper"-Baumwolle sind sehr fest mit der Pflanze verhaftet und werden daher durch die spät in der Vegetationsperiode auftretenden Stürme nicht beeinträchtigt

Bei der Ernte der "Stripper"-Baumwolle wird die Pflanze jedoch zerstört. Die Kapseln der "Picker"-Baumwolle sind dagegen weniger fest mit der Pflanze verhaftet, sodass die Ernte der "Picker"-Baumwolle ohne grössere Beschädigung der Pflanze möglich ist.

Im Folgenden sollen einige Beispiele von kommerziell erhältlichen *G. hirsutum* Varietäten benannt werden, die mit Hilfe des erfindungsgemässen Verfahren regeneriert werden können:
Acala 1515-75, Acala SJ-2, Acala SJ-4, Acala SJ-5, Acala SJC-1, Acala SJC-22, Acala SJC-28, Acala SJC-30, Acala B-1644, Acala B-1810, Acala B-2724, Acala GC-510.
Coker 304, Coker 315, Coker 201, Coker 310, Coker 312,
DP 41, DP 90,
DPL 50, DPL 20, DPL 120, DPL 775,
Lankart 611, Lankart 57,
Paymaster 145, Paymaster HS 26,
Stoneville 506, Stoneville 825,
Funk 519-2, Funk FC 3008, Funk FC 3024, Funk & 1568R, Funk FC 2005, Funk C 0947B, Funk FC 2028, Funk FC 2017, Funk C 1379,
McNair 235, Tomcot SP 21-S, Siokra, Tx-CAB-CS.

Besonders bevorzugt sind im Rahmen dieser Erfindung die *G. hirsutum* Varietäten Acala SJ-2, Acala SJC-1, Acala GC 510, Acala SJC-28, Acala SJC-30, Acala B-1644 und Siokra.

Ganz besonders bevorzugt sind die *G. hirsutum* Varietäten Acala SJ-2, Acala GC 510, Acala B-1644 und Siokra.

Unter dem Ausdruck "Pflanzenzelle" soll im Rahmen dieser Erfindung definitionsgemäss jede von einer Baumwollpflanze abgeleitete Zelle verstanden werden. Einige Beispiele von Zellen, die von der vorliegenden Erfindung umfasst werden, sind differenzierte Zellen, die Teil einer lebenden Pflanze sind, differenzierte und undifferenzierte Zellen in Kultur, die Zellen undifferenzierter Gewebe wie Kallus oder Tumore, sowie die Zellen von Samen, Embryonen, Vermehrungsgut und Pollen.

Das erfindungsgemässe chimäre Gen enthält einen Promotorabschnitt, der in Baumwollpflanzen mit hoher Effizienz funktioniert, sowie einen kodierenden Abschnitt, der das kristalline Protein von *Bt* oder aber ein Polypeptid kodiert, das im wesentlichen die insektiziden Eigenschaften des kristallinen Proteins von *Bt* besitzt. Es ist nicht bekannt, dass die kodierende Sequenz des chimären Gens in natürlich vorkommenden Genen mit dem Promotor verknüpft wäre.

Die 5'- und/oder 3'-nichttranslatierten Abschnitte können natürlicherweise unabhängig voneinander entweder mit dem Promotor oder dem kodierenden Bereich verknüpft sein oder sie sind weder mit dem Promotor noch mit dem kodierenden Bereich assoziiert. In natürlich vorkommenden Genen ist vorzugsweise entweder der 5'- oder der 3'-nichttranslatierte Abschnitt mit dem Promotor assoziiert, insbesondere aber sind in natürlich vorkommenden Genen beide, der 5'- und der 3'-Abschnitt, mit dem Promotor assoziiert.

Ausgehend vom Stand der Technik konnte zum Zeitpunkt, als diese Erfindung gemacht wurde, niemand vorhersagen, dass es möglich wäre, ein chimäres Gen stabil und funktionell in Baumwollzellen einzuführen. Es war noch weniger vorhersagbar, dass solche Zellen ein insektizides Polypeptid in beliebigen Mengen exprimieren würden. Insbesondere war nicht vorhersagbar, dass es in einer Menge produziert würde, die ausreicht, um den Zellen insektizide Eigenschaften zu verleihen. Man konnte vielmehr erwarten, dass es besondere Schwierigkeiten bereiten würde, ein Polypeptid, das so gross und so unlöslich ist wie das Polypeptid, welches die Insektentoxizitätseigenschaften des kristallinen Proteins von *Bt* aufweist, in Pflanzenzellen zu exprimieren.

Um als insektizid angesehen zu werden, müssen die Pflanzenzellen eine insektizid wirksame Menge eines Toxins enthalten, das im wesentlichen die insektizide Aktivität des kristallinen Proteins von *Bt* aufweist. Unter einer insektizid-wirksamen Menge an Toxin soll im Rahmen dieser Erfindung eine in der Pflanzenzelle vorliegende Toxinkonzentration verstanden werden, die ausreicht, die Insektenlarven abzutöten oder aber zumindest ihre Nahrungsaufnahme in substantieller Weise zu reduzieren. Dementsprechend können die erfindungsgemässen Pflanzenzellen Angriffen von Insektenlarven widerstehen, wobei keine oder aber weitaus geringere Mengen des kristallinen Proteins oder anderer Insektizide appliziert werden müssen, verglichen mit Pflanzenzellen, die kein Gen enthalten, das ein insektizides Polypeptid kodiert.

Das chimäre Gen dieser Erfindung enthält Transkriptionskontrollsequenzen wie z.B. Promotor und 5'- und 3'-nichttranslatierte Sequenzen, die in Baumwollpflanzen funktionsfähig sind. Diese Sequenzen können unabhängig voneinander aus irgendeiner beliebigen Quelle stammen, wie beispielsweise aus Virus-, Pflanzen- oder Bakteriengenen.

Die Viruspromotoren sowie die 5′- und 3′-nichttranslatierten Sequenzen, die für die erfindungsgemässe Verwendung geeignet sind, funktionieren in Baumwollpflanzen und können beispielsweise aus Pflanzenviren, wie dem *Cauliflower Mosaik Virus* (CaMV) erhalten werden. Das CaMV-Virus ist von Hohn et al. (1982) 194-220 im Detail charakterisiert und beschrieben worden (Appendix A bis G). Diese Beschreibung ist ein integraler Bestandteil dieser Erfindung.

CaMV ist ein atypisches Pflanzenvirus, weil es doppelsträngige DNA enthält. Wenigstens zwei CaMV-Promotoren sind in Pflanzen funktionsfähig, der 19S-Promotor, der bei der Transkription des CaMV Gens VI beteiligt ist, und der 35S-Promotor. Der 19S-Promotor und der 35S-Promotor sind die bevorzugten Pflanzenviruspromotoren für die Verwendung in der vorliegenden Erfindung.

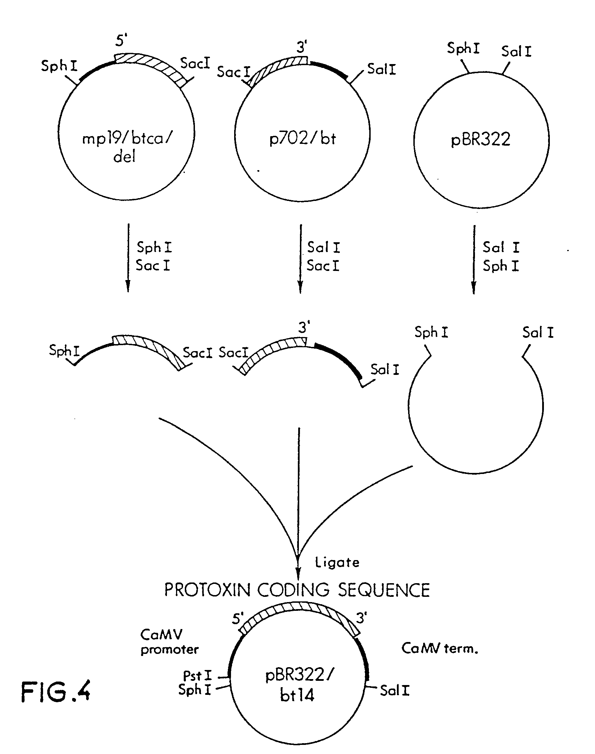

CaMV 19S-Promotoren und 5′-nichttranslatierte Abschnitte können mit Hilfe einer Restriktionskarte, wie sie in Fig. 4 auf Seite 199 des oben erwähnten Hohn et al.-Artikels angegeben ist, oder mit Hilfe der in Anhang C des Hohn et al.-Artikels wiedergegebenen Sequenz, erhalten werden.

Um den CaMV 19S-Promotor und wahlweise den benachbarten 5′-nichttranslatierten Abschnitt zu isolieren, wird ein Restriktionsfragment des CaMV-Genoms mit den gewünschten Sequenzen ausgewählt. Ein geeignetes Restriktionsfragment, das den 19S-Promotor und die 5′-nichttranslatierte Region enthält, ist das Fragment zwischen der PstI-Schnittstelle, beginnend an Position 5386, und der HindIII-Schnittstelle, beginnend an Position 5850 der in Fig. 4 wiedergegebenen Restriktionskarte bzw. der in Anhang C des Hohn et al.-Artikels wiedergegebenen Sequenz.

Wie weiter unten beschrieben, kann durch analoge Verfahren der 35S-Promotor von CaMV erhalten werden.

Unerwünschte Nukleotide in dem Restriktionsfragment können gegebenenfalls mit Hilfe von Standardverfahren entfernt werden. Unerwünschte Nukleotide können beispielsweise durch den Gebrauch von Exonukleasen (Maniatis et al., 1982) oder durch eine Oligonukleotid-vermittelte Mutagenese (Zoller und Smith, 1983) deletiert werden.

Ein ähnliches Verfahren kann benutzt werden, um gegebenenfalls eine wünschenswerte 3′-nichttranslatierte Region zu erhalten. So kann man beispielsweise eine geeignete 3′-nichttranslatierte Sequenz des CaMV 19S-Gens erhalten, indem man die Region zwischen der EcoRV-Schnittstelle an Position 7342 und der BglII-Schnittstelle an Position 7643 des in Fig. 4 bzw. im Anhang & des Hohn et al.-Artikels beschriebenen CaMV-Genoms, isoliert.

Beispiele für Pflanzengen-Promotoren und 5′- sowie 3′-nichttranslatierte Regionen, die für die Verwendung in der vorliegenden Erfindung geeignet sind, schliessen auch die Promotoren der Gene mit ein, welche die kleine Untereinheit der Ribulose-1,5-bisphosphatcarboxylase und das Chlorophyll a/b-Bindungsprotein kodieren. Diese Pflanzengenregionen können auf vergleichbare Art und Weise isoliert werden, wie dies zuvor für die Isolierung der entsprechenden Regionen des CaMV Genoms beschrieben wurde (Morelli et al., 1985).

Ebenso geeignet für die Verwendung im Rahmen der vorliegenden Erfindung sind die Promotoren und 5′- sowie 3′-nichttranslatierten Regionen, die sich in der T-DNA-Region von *Agrobacterium*-Plasmiden befinden. Einige Beispiele geeigneter *Agrobacterium*-Plasmide umfassen das Ti-Plasmid von *A. tumefaciens* und das Ri-Plasmid von *A. rhizogenes*. Besonders bevorzugt für die Verwendung im Rahmen der vorliegenden Erfindung sind *Agrobacterium*-Promotoren sowie 5′- und 3′-nichttranslatierte Regionen, die in den für die Oktopin- und die Nopalin-Synthase kodierenden Genen vorliegen. Diese Sequenzen können durch ähnliche Verfahren erhalten werden, wie sie oben für die Isolierung von CaMV- und Pflanzenpromotoren sowie für die nichttranslatierten Sequenzen beschrieben sind (Bevan et al., 1983).

Die kodierende Region des chimären Gens enthält eine Nukleotidsequenz, die ein Polypeptid kodiert, welches im wesentlichen die Toxizitätseigenschaften eines kristallinen δ-Endotoxin Proteins von *Bt* aufweist. Für den erfindungsgemässen Anwendungszweck weist ein Polypeptid definitionsgemäss dann im wesentlichen die Toxizitätseigenschaften des kristallinen δ-Endotoxin Proteins von *Bt* auf, wenn es gegen ein ähnliches Spektrum von Insekten-Larven insektizid wirkt, wie das kristalline Protein irgendeiner beliebigen Unterart von *Bt*. Einige geeignete Unterarten sind beispielsweise solche, ausgewählt aus der Gruppe *Bt* var. *kurstaki*, *Bt* var. *berliner*, *Bt* var. *alesti*, *Bt* var. *tolworthi*, *Bt* var. *sotto*, *Bt* var. *dendrolimus*, *Bt* var. *tenebrionis*, *Bt* var. *san diego* und *Bt* var. *aizawai*. Die bevorzugte Unterart ist *Bt* var. *kurstaki* und insbesondere *Bt* var. *kurstaki* HD1.

Bei der kodierenden Region kann es sich um eine Region handeln, die natürlicherweise in *Bt* vorkommt. Alternativ dazu kann die kodierende Region gegebenenfalls aber auch eine Sequenz enthalten, die sich von der Sequenz in *Bt* unterscheidet, die ihr aber aufgrund der Degenerierung des genetischen Kodes äquivalent ist.

Die kodierende Region des chimären Gens kann auch ein Polypeptid kodieren, das sich von einem natürlich vorkommenden kristallinen δ-Endotoxin Protein unterscheidet, das aber immer noch im wesentlichen die Insektentoxizitätseigenschaften des kristallinen Proteins aufweist. Dabei wird es sich üblicherweise um eine Variante einer natürlichen kodierenden Region handeln. Unter einer "variante" einer natürlichen DNA-Sequenz ist im Rahmen dieser Erfindung definitionsgemäss eine modifizierte Form einer natürlichen Sequenz zu verstehen, die aber noch dieselbe Funktion erfüllt. Die Variante kann eine Mutante oder eine synthetische DNA-Sequenz sein und ist im wesentlichen der entsprechenden natürlichen Sequenz homolog.

Unter dem Begriff einer "im wesentlichen homologen Sequenz" soll im Rahmen dieser Erfindung definitionsgemäss entweder a) ein DNA Fragment verstanden werden, das eine ausreichend hohe Aehnlichkeit mit einem zweiten DNA Fragment aufweist, sodass es in der Lage ist, auch ein Polypeptid zu exprimieren, das ähnliche Eigenschaften zeigt; oder aber b) ein Polypeptid, das eine Aminosäuresequenz besitzt, die eine ausreichend hohe Aehnlichkeit mit einer zweiten Aminosäuresequenz aufweist, sodass sie in der Folge auch ähnliche Eigenschaften zeigt.

Normalerweise ist im Rahmen dieser Erfindung eine DNA-Sequenz einer zweiten DNA-Sequenz dann im wesentlichen homolog, wenn mindestens 70 % vorzugsweise mindestens 80 % und ganz besonders bevorzugt mindestens 90 % der aktiven Abschnitte der DNA-Sequenz homolog sind. Für die Ermittlung der Homologie werden zwei unterschiedliche Nukleotide in einer DNA-Sequenz einer kodierenden Region dann als homolog angesehen, wenn der Austausch des einen Nukleotids gegen das andere eine stille Mutation darstellt.

Die Erfindung umfasst somit Baumwollzellen und -pflanzen, die irgendein beliebiges chimäres Gen enthalten, das eine Aminosäuresequenz kodiert welche die insektiziden Eigenschaften aufweist, die mit den offenbarten und beanspruchten Erfordernissen in Einklang stehen. Besonders bevorzugt ist eine Nukleotidsequenz, die im wesentlichen wenigstens dem Teil oder den Teilen der natürlichen Sequenz homolog ist, die für die insektizide Aktivität verantwortlich ist (sind).

Das Polypeptid, welches durch das erfindungsgemässe chimäre Gen exprimiert wird, hat in der Regel auch zumindest einige immunologische Eigenschaften mit einem natürlichen *Bt* Kristallprotein gemeinsam, da es wenigstens zum Teil die gleichen antigenen Determinanten besitzt.

Dementsprechend ist das Polypeptid, das von dem chimären Gen der vorliegenden Erfindung kodiert wird, vorzugsweise strukturell mit dem kristallinen δ-Endotoxin von *Bt* verwandt. *Bt* produziert ein kristallines Protein, das einem Protoxin mit einem Mr von etwa 130 000 bis 140 000 entspricht. Dieses Protoxin kann durch Proteasen oder durch Alkali in insektizide Fragmente mit einem Mr von etwa 80 000, vorzugsweise von etwa 70 000, ganz bevorzugt von etwa 60 000 und möglicherweise sogar weniger gespalten werden.

Besagte Fragmente haben ein maximales Molekulargewicht von etwa 120 000, vorzugsweise von etwa 110 000 und ganz besonders bevorzugt von etwa 100 000.

Die Konstruktion chimärer Gene, die erfindungsgemäss solche Protoxinfragmente oder noch kleinere Teile davon kodieren, kann solange fortschreiten, wie die Fragmente oder Teile dieser Fragmente noch die erforderliche insektizide Aktivität aufweisen. Das Protoxin, insektizide Fragmente des Protoxins und insektizide Teile dieser Fragmente können mit anderen Molekülen, wie Polypeptiden und Proteinen, verbunden werden.

Kodierende Regionen, die für eine erfindungsgemässe Verwendung geeignet sind, können von Genen aus *Bt*, die das kristalline Toxingen kodieren, erhalten werden (siehe z.B. PCT Anmeldung WO86/01536 und US Patente 4 448 885 und 4 467 036). Eine bevorzugte Nukleotidsequenz, die ein kristallines Protein kodiert, befindet sich zwischen den Nukleotiden 156 und 3623 der in Formel I wiedergegebenen Sequenz. Ebenfalls bevorzugt ist eine kürzere Nukleotidsequenz, die ein insektizides Fragment eines solchen kristallinen Proteins kodiert. Diese Sequenz ist bei Geiser et al. (1986) publiziert und integraler Bestandteil dieser Erfindung.

Formel I

```
          10         20         30         40         50         60
GTTAACACCC TGGGTCAAAA ATTGATATTT AGTAAAATTA GTTGCACTTT GTGCATTTTT

          70         80         90        100        110        120
TCATAAGATG AGTCATATGT TTTAAATTGT AGTAATGAAA AACAGTATTA TATCATAATG

         130        140        150        160        170        180
AATTGGTATC TTAATAAAAG AGATGGAGGT AACTTATGGA TAACAATCCG AACATCAATG

         190        200        210        220        230        240
AATGCATTCC TTATAATTGT TTAAGTAACC CTGAAGTAGA AGTATTAGGT GGAGAAAGAA

         250        260        270        280        290        300
TAGAAACTGG TTACACCCCA ATCGATATTT CCTTGTCGCT AACGCAATTT CTTTTGAGTG

         310        320        330        340        350        360
AATTTGTTCC CGGTGCTGGA TTTGTGTTAG GACTAGTTGA TATAATATGG GGAATTTTTG .

         370        380        390        400        410        420
GTCCCTCTCA ATGGGACGCA TTTCTTGTAC AAATTGAACA GTTAATTAAC CAAAGAATAG

         430        440        450        460        470        480
AAGAATTCGC TAGGAACCAA GCCATTTCTA GATTAGAAGG ACTAAGCAAT CTTTATCAAA

         490        500        510        520        530        540
TTTACGCAGA ATCTTTTAGA GAGTGGGAAG CAGATCCTAC TAATCCAGCA TTAAGAGAAG

         550        560        570        580        590        600
AGATGCGTAT TCAATTCAAT GACATGAACA GTGCCCTTAC AACCGCTATT CCTCTTTTTG

         610        620        630        640        650        660
CAGTTCAAAA TTATCAAGTT CCTCTTTTAT CAGTATATGT TCAAGCTGCA AATTTACATT

         670        680        690        700        710        720
TATCAGTTTT GAGAGATGTT TCAGTGTTTG GACAAAGGTG GGGATTTGAT GCCGCGACTA

         730        740        750        760        770        780
TCAATAGTCG TTATAATGAT TTAACTAGGC TTATTGGCAA CTATACAGAT CATGCTGTAC

         790        800        810        820        830        840
GCTGGTACAA TACGGGATTA GAGCGTGTAT GGGGACCGGA TTCTAGAGAT TGGATAAGAT

         850        860        870        880        890        900
ATAATCAATT TAGAAGAGAA TTAACACTAA CTGTATTAGA TATCGTTTCT CTATTTCCGA

         910        920        930        940        950        960
ACTATGATAG TAGAACGTAT CCAATTCGAA CAGTTTCCCA ATTAACAAGA GAAATTTATA

         970        980        990       1000       1010       1020
CAAACCCAGT ATTAGAAAAT TTTGATGGTA GTTTTCGAGG CTCGGCTCAG GGCATAGAAG

        1030       1040       1050       1060       1070       1080
GAAGTATTAG GAGTCCACAT TTGATGGATA TACTTAACAG TATAACCATC TATACGGATG
```

```
          1090        1100        1110        1120        1130        1140
     CTCATAGAGG AGAATATTAT TGGTCAGGGC ATCAAATAAT GGCTTCTCCT GTAGGGTTTT

          1150        1160        1170        1180        1190        1200
     CGGGGCCAGA ATTCACTTTT CCGCTATATG GAACTATGGG AAATGCAGCT CCACAACAAC

          1210        1220        1230        1240        1250        1260
     GTATTGTTGC TCAACTAGGT CAGGGCGTGT ATAGAACATT ATCGTCCACT TTATATAGAA

          1270        1280        1290        1300        1310        1320
     GACCTTTTAA TATAGGGATA AATAATCAAC AACTATCTGT TCTTGACGGG ACAGAATTTG

          1330        1340        1350        1360        1370        1380
     CTTATGGAAC CTCCTCAAAT TTGCCATCCG CTGTATACAG AAAAAGCGGA ACGGTAGATT

          1390        1400        1410        1420        1430        1440
     CGCTGGATGA AATACCGCCA CAGAATAACA ACGTGCCACC TAGGCAAGGA TTTAGTCATC

          1450        1460        1470        1480        1490        1500
     GATTAAGCCA TGTTTCAATG TTTCGTTCAG GCTTTAGTAA TAGTAGTGTA AGTATAATAA

          1510        1520        1530        1540        1550        1560
     GAGCTCCTAT GTTCTCTTGG ATACATCGTA GTGCTGAATT TAATAATATA ATTCCTTCAT

          1570        1580        1590        1600        1610        1620
     CACAAATTAC ACAAATACCT TTAACAAAAT CTACTAATCT TGGCTCTGGA ACTTCTGTCG

          1630        1640        1650        1660        1670        1680
     TTAAAGGACC AGGATTTACA GGAGGAGATA TTCTTCGAAG AACTTCACCT GGCCAGATTT

          1690        1700        1710        1720        1730        1740
     CAACCTTAAG AGTAAATATT ACTGCACCAT TATCACAAAG ATATCGGGTA AGAATTCGCT

          1750        1760        1770        1780        1790        1800
     ACGCTTCTAC CACAAATTTA CAATTCCATA CATCAATTGA CGGAAGACCT ATTAATCAGG

          1810        1820        1830        1840        1850        1860
     GGAATTTTTC AGCAACTATG AGTAGTGGGA GTAATTTACA GTCCGGAAGC TTTAGGACTG

          1870        1880        1890        1900        1910        1920
     TAGGTTTTAC TACTCCGTTT AACTTTTCAA ATGGATCAAG TGTATTTACG TTAAGTGCTC

          1930        1940        1950        1960        1970        1980
     ATGTCTTCAA TTCAGGCAAT GAAGTTTATA TAGATCGAAT TGAATTTGTT CCGGCAGAAG

          1990        2000        2010        2020        2030        2040
     TAACCTTTGA GGCAGAATAT GATTTAGAAA GAGCACAAAA GGCGGTGAAT GAGCTGTTTA

          2050        2060        2070        2080        2090        2100
     CTTCTTCCAA TCAAATCGGG TTAAAAACAG ATGTGACGGA TTATCATATT GATCAAGTAT

          2110        2120        2130        2140        2150        2160
     CCAATTTAGT TGAGTGTTTA TCTGATGAAT TTGTCTGGA TGAAAAAAAA GAATTGTCCG
```

```
      2170        2180        2190        2200        2210        2220
AGAAAGTCAA  ACATGCGAAG  CGACTTAGTG  ATGAGCGGAA  TTTACTTCAA  GATCCAAACT

      2230        2240        2250        2260        2270        2280
TTAGAGGGAT  CAATAGACAA  CTAGACCGTG  GCTGGAGAGG  AAGTACGGAT  ATTACCATCC

      2290        2300        2310        2320        2330        2340
AAGGAGGCGA  TGACGTATTC  AAAGAGAATT  ACGTTACGCT  ATTGGGTACC  TTTGATGAGT

      2350        2360        2370        2380        2390        2400
GCTATCCAAC  GTATTTATAT  CAAAAAATAG  ATGAGTCGAA  ATTAAAAGCC  TATACCCGTT

      2410        2420        2430        2440        2450        2460
ACCAATTAAG  AGGGTATATC  GAAGATAGTC  AAGACTTAGA  AATCTATTTA  ATTCGCTACA

      2470        2480        2490        2500        2510        2520
ATGCCAAACA  CGAAACAGTA  AATGTGCCAG  GTACGGGTTC  CTTATGGCCG  CTTTCAGCCC

      2530        2540        2550        2560        2570        2580
CAAGTCCAAT  CGGAAAATGT  GCCCATCATT  CCCATCATTT  CTCCTTGGAC  ATTGATGTTG .

      2590        2600        2610        2620        2630        2640
GATGTACAGA  CTTAAATGAG  GACTTAGGTG  TATGGGTGAT  ATTCAAGATT  AAGACGCAAG

      2650        2660        2670        2680        2690        2700
ATGGCCATGC  AAGACTAGGA  AATCTAGAAT  TTCTCGAAGA  GAAACCATTA  GTAGGAGAAG

      2710        2720        2730        2740        2750        2760
CACTAGCTCG  TGTGAAAAGA  GCGGAGAAAA  AATGGAGAGA  CAAACGTGAA  AAATTGGAAT

      2770        2780        2790        2800        2810        2820
GGGAAACAAA  TATTGTTTAT  AAAGAGGCAA  AAGAATCTGT  AGATGCTTTA  TTTGTAAACT

      2830        2840        2850        2860        2870        2880
CTCAATATGA  TAGATTACAA  GCGGATACCA  ACATCGCGAT  GATTCATGCG  GCAGATAAAC

      2890        2900        2910        2920        2930        2940
GCGTTCATAG  CATTCGAGAA  GCTTATCTGC  CTGAGCTGTC  TGTGATTCCG  GGTGTCAATG

      2950        2960        2970        2980        2990        3000
CGGCTATTTT  TGAAGAATTA  GAAGGGCGTA  TTTTCACTGC  ATTCTCCCTA  TATGATGCGA

      3010        3020        3030        3040        3050        3060
GAAATGTCAT  TAAAAATGGT  GATTTTAATA  ATGGCTTATC  CTGCTGGAAC  GTGAAAGGGC

      3070        3080        3090        3100        3110        3120
ATGTAGATGT  AGAAGAACAA  AACAACCACC  GTTCGGTCCT  TGTTGTTCCG  GAATGGGAAG

      3130        3140        3150        3160        3170        3180
CAGAAGTGTC  ACAAGAAGTT  CGTGTCTGTC  CGGGTCGTGG  CTATATCCTT  CGTGTCACAG

      3190        3200        3210        3220        3230        3240
CGTACAAGGA  GGGATATGGA  GAAGGTTGCG  TAACCATTCA  TGAGATCGAG  AACAATACAG

      3250        3260        3270        3280        3290        3300
ACGAACTGAA  GTTTAGCAAC  TGTGTAGAAG  AGGAAGTATA  TCCAAACAAC  ACGGTAACGT
```

11

```
        3310       3320       3330       3340       3350       3360
GTAATGATTA TACTGCGACT CAAGAAGAAT ATGAGGGTAC GTACACTTCT CGTAATCGAG

        3370       3380       3390       3400       3410       3420
GATATGACGG AGCCTATGAA AGCAATTCTT CTGTACCAGC TGATTATGCA TCAGCCTATG

        3430       3440       3450       3460       3470       3480
AAGAAAAAGC ATATACAGAT GGACGAAGAG ACAATCCTTG TGAATCTAAC AGAGGATATG

        3490       3500       3510       3520       3530       3540
GGGATTACAC ACCACTACCA GCTGGCTATG TGACAAAAGA ATTAGAGTAC TTCCCAGAAA

        3550       3560       3570       3580       3590       3600
CCGATAAGGT ATGGATTGAG ATCGGAGAAA CGGAAGGAAC ATTCATCGTG GACAGCGTGG

        3610       3620       3630       3640       3650       3660
AATTACTTCT TATGGAGGAA TAATATATGC TTTATAATGT AAGGTGTGCA AATAAAGAAT

        3670       3680       3690       3700       3710       3720
GATTACTGAC TTGTATTGAC AGATAAATAA GGAAATTTTT ATATGAATAA AAAACGGGCA

        3730       3740       3750       3760       3770       3780
TCACTCTTAA AAGAATGATG TCCGTTTTTT GTATGATTTA ACGAGTGATA TTTAAATGTT

        3790       3800       3810       3820       3830       3840
TTTTTTGCGA AGGCTTTACT TAACGGGGTA CCGCCACATG CCCATCAACT TAAGAATTTG

        3850       3860       3870       3880       3890       3900
CACTACCCCC AAGTGTCAAA AAACGTTATT CTTTCTAAAA AGCTAGCTAG AAAGGATGAC

        3910       3920       3930       3940       3950       3960
ATTTTTTATG AATCTTTCAA TTCAAGATGA ATTACAACTA TTTTCTGAAG AGCTGTATCG

        3970       3980       3990       4000       4010       4020
TCATTAACC CCTTCTCTTT TGGAAGAACT CGCTAAAGAA TTAGGTTTTG TAAAAAGAAA

        4030       4040       4050       4060       4070       4080
ACGAAAGTTT TCAGGAAATG AATTAGCTAC CATATGTATC TGGGGCAGTC AACGTACAGC

        4090       4100       4110       4120        4130        4140
GAGTGATTCT CTCGTTCGAC TATGCAGTCA ATTACACGCC GCCACAGCAC TCTTATGAGT

        4150       4160       4170       4180       4190       4200
CCAGAAGGAC TCAATAAACG CTTTGATAAA AAAGCGGTTG AATTTTTGAA ATATATTTTT

        4210       4220       4230       4240       4250       4260
TCTGCATTAT GGAAAAGTAA ACTTTGTAAA ACATCAGCCA TTTCAAGTGC AGCACTCACG

        4270       4280       4290       4300       4310       4320
TATTTTCAAC GAATCCGTAT TTTAGATGCG ACGATTTCC AAGTACCGAA ACATTTAGCA

        4330       4340       4350       4360
CATGTATATC CTGGGTCAGG TGGTTGTGCA CAAACTGCAG
```

Die durch die Nukleotide 156 bis 3623 der in Formel I wiedergegebenen Sequenz definierte kodierende Region kodiert ein Polypeptid der Formel II.

Formel II

Met Asp Asn Asn Pro Asn Ile Asn Glu Cys 10

Ile Pro Tyr Asn Cys Leu Ser Asn Pro Glu 20

Val Glu Val Leu Gly Gly Glu Arg Ile Glu 30

Thr Gly Tyr Thr Pro Ile Asp Ile Ser Leu 40

Ser Leu Thr Gln Phe Leu Leu Ser Glu Phe 50

Val Pro Gly Ala Gly Phe Val Leu Gly Leu 60

Val Asp Ile Ile Trp Gly Ile Phe Gly Pro 70

Ser Gln Trp Asp Ala Phe Leu Val Gln Ile 80

Glu Gln Leu Ile Asn Gln Arg Ile Glu Glu 90

Phe Ala Arg Asn Gln Ala Ile Ser Arg Leu 100

Glu Gly Leu Ser Asn Leu Tyr Gln Ile Tyr 110

Ala Glu Ser Phe Arg Glu Trp Glu Ala Asp 120

Pro Thr Asn Pro Ala Leu Arg Glu Glu Met 130

Arg Ile Gln Phe Asn Asp Met Asn Ser Ala 140

Leu Thr Thr Ala Ile Pro Leu Phe Ala Val 150

Gln Asn Tyr Gln Val Pro Leu Leu Ser Val 160

Tyr Val Gln Ala Ala Asn Leu His Leu Ser 170

Val Leu Arg Asp Val Ser Val Phe Gly Gln 180

Arg Trp Gly Phe Asp Ala Ala Thr Ile Asn 190

Ser Arg Tyr Asn Asp Leu Thr Arg Leu Ile 200

Gly Asn Tyr Thr Asp His Ala Val Arg Trp 210

Tyr Asn Thr Gly Leu Glu Arg Val Trp Gly 220

Pro Asp Ser Arg Asp Trp Ile Arg Tyr Asn 230

Gln Phe Arg Arg Glu Leu Thr Leu Thr Val 240

Leu Asp Ile Val Ser Leu Phe Pro Asn Tyr 250

Asp Ser Arg Thr Tyr Pro Ile Arg Thr Val 260

Ser Gln Leu Thr Arg Glu Ile Tyr Thr Asn 270

Pro Val Leu Glu Asn Phe Asp Gly Ser Phe 280

Arg Gly Ser Ala Gln Gly Ile Glu Gly Ser 290

Ile Arg Ser Pro His Leu Met Asp Ile Leu 300

Asn Ser Ile Thr Ile Tyr Thr Asp Ala His 310

Arg Gly Glu Tyr Tyr Trp Ser Gly His Gln          320

Ile Met Ala Ser Pro Val Gly Phe Ser Gly          330

Pro Glu Phe Thr Phe Pro Leu Tyr Gly Thr          340

Met Gly Asn Ala Ala Pro Gln Gln Arg Ile          350

Val Ala Gln Leu Gly Gln Gly Val Tyr Arg          360

Thr Leu Ser Ser Thr Leu Tyr Arg Arg Pro          370

Phe Asn Ile Gly Ile Asn Asn Gln Gln Leu          380

Ser Val Leu Asp Gly Thr Glu Phe Ala Tyr          390

Gly Thr Ser Ser Asn Leu Pro Ser Ala Val          400

Tyr Arg Lys Ser Gly Thr Val Asp Ser Leu          410

Asp Glu Ile Pro Pro Gln Asn Asn Asn Val          420

Pro Pro Arg Gln Gly Phe Ser His Arg Leu          430

Ser His Val Ser Met Phe Arg Ser Gly Phe          440

Ser Asn Ser Ser Val Ser Ile Ile Arg Ala          450

Pro Met Phe Ser Trp Ile His Arg Ser Ala          460

Glu Phe Asn Asn Ile Ile Pro Ser Ser Gln          470

Ile Thr Gln Ile Pro Leu Thr Lys Ser Thr          480

Asn Leu Gly Ser Gly Thr Ser Val Val Lys          490

Gly Pro Gly Phe Thr Gly Gly Asp Ile Leu          500

Arg Arg Thr Ser Pro Gly Gln Ile Ser Thr          510

Leu Arg Val Asn Ile Thr Ala Pro Leu Ser          520

Gln Arg Tyr Arg Val Arg Ile Arg Tyr Ala          530

Ser Thr Thr Asn Leu Gln Phe His Thr Ser          540

Ile Asp Gly Arg Pro Ile Asn Gln Gly Asn          550

Phe Ser Ala Thr Met Ser Ser Gly Ser Asn          560

Leu Gln Ser Gly Ser Phe Arg Thr Val Gly          570

Phe Thr Thr Pro Phe Asn Phe Ser Asn Gly          580

Ser Ser Val Phe Thr Leu Ser Ala His Val          590

Phe Asn Ser Gly Asn Glu Val Tyr Ile Asp          600

Arg Ile Glu Phe Val Pro Ala Glu Val Thr          610

Phe Glu Ala Glu Tyr Asp Leu Glu Arg Ala          620

Gln Lys Ala Val Asn Glu Leu Phe Thr Ser          630

Ser Asn Gln Ile Gly Leu Lys Thr Asp Val          640

Thr Asp Tyr His Ile Asp Gln Val Ser Asn          650

Leu Val Glu Cys Leu Ser Asp Glu Phe Cys          660

Leu Asp Glu Lys Lys Glu Leu Ser Glu Lys          670

Val Lys His Ala Lys Arg Leu Ser Asp Glu          680

15

Arg Asn Leu Leu Gln Asp Pro Asn Phe Arg          690

Gly Ile Asn Arg Gln Leu Asp Arg Gly Trp          700

Arg Gly Ser Thr Asp Ile Thr Ile Gln Gly          710

Gly Asp Asp Val Phe Lys Glu Asn Tyr Val          720

Thr Leu Leu Gly Thr Phe Asp Glu Cys Tyr          730

Pro Thr Tyr Leu Tyr Gln Lys Ile Asp Glu          740

Ser Lys Leu Lys Ala Tyr Thr Arg Tyr Gln          750

Leu Arg Gly Tyr Ile Glu Asp Ser Gln Asp          760

Leu Glu Ile Tyr Leu Ile Arg Tyr Asn Ala          770

Lys His Glu Thr Val Asn Val Pro Gly Thr          780

Gly Ser Leu Trp Pro Leu Ser Ala Pro Ser          790

Pro Ile Gly Lys Cys Ala His His Ser His          800

His Phe Ser Leu Asp Ile Asp Val Gly Cys          810

Thr Asp Leu Asn Glu Asp Leu Gly Val Trp          820

Val Ile Phe Lys Ile Lys Thr Gln Asp Gly          830

His Ala Arg Leu Gly Asn Leu Glu Phe Leu          840

Glu Glu Lys Pro Leu Val Gly Glu Ala Leu          850

Ala Arg Val Lys Arg Ala Glu Lys Lys Trp          860

Arg Asp Lys Arg Glu Lys Leu Glu Trp Glu          870

Thr Asn Ile Val Tyr Lys Glu Ala Lys Glu          880

Ser Val Asp Ala Leu Phe Val Asn Ser Gln          890

Tyr Asp Arg Leu Gln Ala Asp Thr Asn Ile          900

Ala Met Ile His Ala Ala Asp Lys Arg Val          910

His Ser Ile Arg Glu Ala Tyr Leu Pro Glu          920

Leu Ser Val Ile Pro Gly Val Asn Ala Ala          930

Ile Phe Glu Glu Leu Glu Gly Arg Ile Phe          940

Thr Ala Phe Ser Leu Tyr Asp Ala Arg Asn          950

Val Ile Lys Asn Gly Asp Phe Asn Asn Gly          960

Leu Ser Cys Trp Asn Val Lys Gly His Val          970

Asp Val Glu Glu Gln Asn Asn His Arg Ser          980

Val Leu Val Val Pro Glu Trp Glu Ala Glu          990

Val Ser Gln Glu Val Arg Val Cys Pro Gly          1000

Arg Gly Tyr Ile Leu Arg Val Thr Ala Tyr          1010

Lys Glu Gly Tyr Gly Glu Gly Cys Val Thr          1020

Ile His Glu Ile Glu Asn Asn Thr Asp Glu          1030

Leu Lys Phe Ser Asn Cys Val Glu Glu Glu          1040

Val Tyr Pro Asn Asn Thr Val Thr Cys Asn          1050

16

```
Asp Tyr Thr Ala Thr Gln Glu Glu Tyr Glu            1060

Gly Thr Tyr Thr Ser Arg Asn Arg Gly Tyr            1070

Asp Gly Ala Tyr Glu Ser Asn Ser Ser Val            1080

Pro Ala Asp Tyr Ala Ser Ala Tyr Glu Glu            1090

Lys Ala Tyr Thr Asp Gly Arg Arg Asp Asn            1100

Pro Cys Glu Ser Asn Arg Gly Tyr Gly Asp            1110

Tyr Thr Pro Leu Pro Ala Gly Tyr Val Thr            1120

Lys Glu Leu Glu Tyr Phe Pro Glu Thr Asp            1130

Lys Val Trp Ile Glu Ile Gly Glu Thr Glu            1140

Gly Thr Phe Ile Val Asp Ser Val Glu Leu            1150

Leu Leu Met Glu Glu End                            1156
```

Die vorliegende Erfindung umfasst somit auch Polypeptide mit der Aminosäuresequenz der Formel (II) oder insektizid wirksame Teile davon.

Darüberhinaus hat es sich gezeigt, dass die Toxine einiger *Bt*-Stämme gegen andere Insekten als Lepidopteren toxisch wirken. So ist beispielsweise das Toxin von *Bt* var. *tenebrionis* spezifisch gegenüber Coleopteren toxisch.

Die Toxizität des *Bt*-Stammes *san diego* gegenüber Coleopteren und die Sequenz des entsprechenden Toxingens sind in den Europäischen Patentanmeldungen EP-0,202,739 und EP-0,213,818 offenbart.

Um das erfindungsgemässe chimäre Gen in Pflanzenzellen einzuführen, wird das Gen zuerst in einen Vektor eingebaut. Wenn das entsprechende Gen nicht in einer Menge zur Verfügung steht, die für eine Einschleusung in Pflanzenzellen ausreicht, besteht die Möglichkeit, den Vektor zunächst durch Replikation in einer Wirtszelle zu amplifizieren. Am besten geeignet für die Amplifikation von Genen sind Bakterien- oder Hefezellen. Wenn eine ausreichende Menge des chimären Gens zur Verfügung steht, wird es in Baumwollzellen oder -gewebe eingeschleust. Die Einführung des Gens in Baumwollzellen oder -gewebe kann mit Hilfe des gleichen Vektors, der zur Replikation genutzt wurde, oder aber mit einem anderen Vektor durchgeführt werden.

Einige Beispiele für bakterielle Wirtszellen, die für eine Replikation des chimären Gens geeignet sind, umfassen Bakterien, ausgewählt aus der Gruppe bestehend aus den Gattungen *Escherichia*, wie *E. coli*, und *Agrobacterium*, wie *A. tumefaciens* oder *A. rhizogenes*. Verfahren der Klonierung heterologer Gene in Bakterien sind in den US Patenten 4,237,224 und 4,468,464 beschrieben.

Die Replikation in *E. coli* von Genen, die das kristalline Protein von *Bt* kodieren, wird von Wong et al. (1983) beschrieben.

Die im Rahmen dieser Erfindung bevorzugte bakterielle Wirtszelle für die Amplifikation der chimären *Bt* Gene ist *Agrobacterium*. Der Vorteil einer Amplifikation von Genen in *Agrobacterium* besteht darin, dass das Agrobakterium anschliessend direkt für die Einschleusung der amplifizierten Gene in die pflanzliche Zelle verwendet werden kann, ohne dass weitere genetische Manipulationen notwendig werden.

Hefewirtszellen, die sich zur Replikation der erfindungsgemässen Gene eignen, schliessen beispielsweise solche aus der Gattung Saccharomyces ein.

Für die Amplifikation der erfindungsgemässen Gene kann jeder beliebige Vektor verwendet werden, in den das chimäre Gen eingebaut werden kann und der sich in einer geeigneten Wirtszelle, wie z.B. in Bakterien oder Hefe, repliziert. Der Vektor kann beispielsweise von einem Phagen oder einem Plasmid abgeleitet sein. Beispiele für Vektoren, die von Phagen abgeleitet sind und im Rahmen dieser Erfindung verwendet werden können, umfassen Vektoren, die sich von M13- und von λ-Phagen ableiten. Als Beispiele für Vektoren, die von M13-Phagen abgeleitet sind, seien hier M13mp18 und M13mp19 genannt. Einige geeignete, von λ-Phagen abgeleitete Vektoren schliessen λgt11, λgt7 und λCharon4 ein.

Vektoren, die von Plasmiden abgeleitet und ganz besonders für die Replikation in Bakterien geeignet sind, umfassen beispielsweise pBR322 (Bolivar et al., 1977), pUC18 und pUC19 (Norrander et al., 1983) sowie die Ti-Plasmide (Bevan et al., 1983). Die bevorzugten Vektoren zur Amplifikation von Genen in Bakterien sind pBR322, pUC18 und pUC19.

Um ein für die Replikation in Bakterien geeignetes chimäres Gen zu konstruieren, werden eine Promotorsequenz, eine 5'-nichttranslatierte Sequenz, eine kodierende Sequenz und eine 3'-nichttranslatierte Sequenz zusammen in einen Vektor eingespleisst oder aber in der richtigen Reihenfolge in einem der zuvor beschriebenen Vektoren zusammengebaut. Geeignete Vektoren sind erfindungsgemäss solche, die in der Lage sind, sich in der Wirtszelle zu replizieren.

17

Der Promotor, die 5'-nichttranslatierte Region, die kodierende Region und die 3'-nichttranslatierte Region, die zusammen das erfindungsgemässe chimäre Gen bilden, können zuerst ausserhalb des Vektors als eine Einheit zusammengefasst und dann als Ganzes in den Vektor eingespleisst werden. Alternativ dazu können aber auch Teile des chimären Gens einzeln in den Vektor eingebaut werden.

Der Vektor enthält darüberhinaus vorzugsweise auch ein Gen, welches der Wirtszelle eine Eigenschaft verleiht, die eine Selektionierung derjenigen Zellen erlaubt, die den Vektor enthalten. Die bevorzugte Eigenschaft ist eine Antibiotikaresistenz. Als Beispiele für Antibiotika, die in diesem Zusammenhang geeignet sind, seien Ampicillin, Tetracyclin, Hygromycin, G418, Chloramphenicol, Kanamycin und Neomycin genannt.

Der Einbau des Gens in den Vektor bzw. der Zusammenbau des Gens im Vektor wird mit Hilfe von Standardverfahren durchgeführt, beispielsweise unter Verwendung von rekombinanter DNA (Maniatis et al., 1982) und Anwendung der homologen Rekombination (Hinnen et al., 1978).

Die Verfahren der rekombinanten DNA-Technologie beruhen darauf, dass der Vektor zunächst geschnitten und die gewünschte DNA-Sequenz zwischen die geschnittenen Stücke des Vektors eingefügt wird; die Enden der gewünschten DNA-Sequenz werden anschliessend mit den entsprechenden Enden des Vektors verknüpft.

Der Vektor wird vorzugsweise mit geeigneten Restriktionsendonukleasen geschnitten. Geeignete Restriktionsendonukleasen sind beispielsweise solche, die glatte Enden bilden, wie SmaI, HpaI und EcoRV, sowie solche, die kohäsive Enden bilden, wie EcoRI, SacI und BamHI.

Die gewünschte DNA-Sequenz existiert normalerweise als Teil eines grösseren DNA-Moleküls, wie eines Chromosoms, eines Plasmids, eines Transposons oder eines Phagen. Die gewünschte DNA-Sequenz wird in diesen Fällen aus ihrer ursprünglichen Quelle herausgeschnitten und gegebenenfalls so modifiziert, dass ihre Enden mit denen des geschnittenen Vektors verbunden werden können. Wenn die Enden der gewünschten DNA-Sequenz und des geschnittenen Vektors glatte Enden sind, werden sie mit für glatte Enden spezifischen Ligasen, wie z.B. der T4 DNA-Ligase, miteinander verknüpft.

Die Verknüpfung der Enden einer gewünschten DNA-Sequenz mit den Enden des geschnittenen Vektors kann auch in Form von kohäsiven Enden erfolgen, wobei eine für kohäsive Enden spezifische Ligase benutzt wird; auch in diesem Fall kann es sich dabei um eine T4 DNA-Ligase handeln. Eine andere geeignete, für kohäsive Enden spezifische Ligase ist beispielsweise die E. coli DNA-Ligase.

Kohäsive Enden werden zweckmässigerweise dadurch gebildet, dass man die gewünschte DNA-Sequenz und den Vektor mit der gleichen Restriktionsendonuklease schneidet. In diesem Fall haben die gewünschte DNA-Sequenz und der geschnittene Vektor kohäsive Enden, die einander komplementär sind.

Die kohäsiven Enden können aber auch artifiziell konstruiert werden, indem man mit Hilfe der terminalen Desoxynukleotidyl-Transferase komplementäre, homopolymere Schwänze an die Enden der gewünschten DNA-Sequenz und des geschnittenen Vektors anhängt. Alternativ dazu können kohäsive Enden aber auch dadurch hergestellt werden, dass man eine synthetische Oligonukleotid-Sequenz, die von einer bestimmten Restriktionsendonuklease erkannt wird und die unter der Bezeichnung Linker bekannt ist, anhängt und anschliessend die Sequenz mit der Endonuklease spaltet (siehe beispielsweise Maniatis et al., 1982).

Die erfindungsgemässen Bt Toxingene können direkt in die pflanzliche Zelle eingeschleust werden, unter Verwendung bestimmter, in Agrobacterium vorkommender Plasmide. Diese Plasmide enthalten Regionen, die im Zuge einer Agrobacterium-Infektion natürlicherweise in das Genom pflanzlicher Zellen eingebaut werden. Die eingebauten Abschnitte werden als T-DNA ("transferierte DNA") bezeichnet.

Besagte Plasmide, zu denen beispielsweise das Ti-Plasmid (Tumorinduzierendes Plasmid) von A. tumefaciens gehört sowie das Ri-Plasmid [Wurzel ("root") induzierendes Plasmid] von A. rhizogenes, besitzen sogenannte T-DNA Grenzsequenzen, die beide oder möglicherweise zumindest eine für den Transfer der T-DNA Region vom Plasmid auf das Genom der infizierten pflanzlichen Zelle notwendig sind.

Die natürlicherweise vorkommende Ti- und Ri-Plasmide enthalten darüberhinaus noch sogenannte Virulenz-Regionen. Dabei handelt es sich um DNA-Abschnitte, von denen angenommen wird, dass sie ausserhalb der T-DNA Region lokalisiert sind. Diese Virulenz-Regionen werden für den Transfer der T-DNA in die pflanzliche Zelle benötigt.

In artifiziell modifizierten Systemen können diese Virulenzregionen auch auf anderen Plasmiden vorliegen als auf dem T-DNA enthaltenden Plasmid. Solche, eine Virulenz-Region enthaltende Plasmide, bezeichnet man als Helfer-Plasmide.

Die natürlicherweise vorkommenden T-DNA Regionen sind oncogen und führen zur Ausbildung von Tumoren. Diese oncogenen Abschnitte der T-DNA Regionen können entweder vor oder aber gleichzeitig mit dem Einbau einer gewünschten DNA-Sequenz ganz oder teilweise entfernt werden. Plasmide, die eine auf die zuvor beschriebene Weise modifizierte T-DNA Region enthalten, werden als entschärfte (disarmed) Plasmide bezeichnet.

Die Gene, die für den erfindungsgemässen Verwendungszweck geeignet sind, werden mit Hilfe an sich bekannter Methoden (Barton und Chilton, 1983; Chilton, 1985) in einem T-DNA Vektorsystem zusammengebaut oder aber als eine Einheit in ein solches Vektorsystem eingespleisst.

Der T-DNA Vektor kann dabei oncogen (Hernalsteens et al, 1980), teilweise entschärft ("partially disarmed") (Barton und Chilton, 1983) oder aber vollständig entschärft ("fully disarmed") (Zambryski et al, 1983) sein. Der T-DNA Vektor kann sich darüberhinaus aber auch von artifiziellen T-DNA Vektoren ableiten, die synthetische, T-DNA Grenzsequenzen-ähnliche Strukturen besitzen (Wang et al, 1984).

Stellvertretend für geeignete entschärfte Vektoren, die eine T-DNA Grenzsequenz enthalten, seien hier pGA436, pGA437 und pGA438 genannt, die bei An et al, 1985 beschrieben sind, desweiteren pMON120 (siehe Fraley et al, 1983) sowie pCIB10 (Rothstein et al, 1987).

Der Transfer der T-DNA wird gewöhnlich durch Inkubation von *Agrobacterium* mit pflanzlichen Zellprotoplasten oder verwundetem pflanzlichem Gewebe erreicht (siehe Caplan et al, 1983).

Zusätzlich zu dem chimären Gen, das ein *Bt* Toxin oder aber ein Toxin, das im wesentlichen die Toxizitätseigenschaften des *Bt* Toxins aufweist, kodiert, enthalten die Vektoren vorzugsweise weiterhin eine DNA-Sequenz, die die Selektionierung oder das Screening von Vektor-haltigen Baumwollpflanzenzellen in Anwesenheit von Zellen ohne Vektor erlaubt. Solche selektier- oder screenbare Marker können entweder bereits natürlicherweise in dem Vektor, der das erfindungsgemässe chimäre Gen eingebaut enthält, vorhanden sein oder sie können entweder vor oder nach dem Einbau des chimären Gens in den Vektor eingefügt werden. Alternativ dazu kann das selektier- oder screenbare Markergen oder ein Teil davon zuerst mit dem gewünschten chimären Gen oder irgendeinem Teil davon verknüpft werden und die auf diese Weise rekombinierten Gene oder Gensegmente können dann als eine Einheit in den Vektor eingespleisst werden. Der selektier- oder screenbare Marker kann selbst chimären Charakter haben.

Der bevorzugte selektierbare Marker ist ein Gen, das Antibiotikaresistenz kodiert. Das Gen muss in den zu transformierenden Zellen zur Expression fähig sein. Die Zellen können dann in einem Antibiotikumhaltigen Medium kultiviert werden, wobei diejenigen Zellen, die den Vektor enthalten und die damit eine verbesserte Ueberlebensfähigkeit in dem Medium aufweisen, selektioniert werden. Alle Gene, die Baumwollpflanzen eine Resistenz gegen Toxine wie Hygromycin, Chloramphenicol, Kanamycin, G418 oder im Prinzip gegenüber jedem beliebigen anderen Antibiotikum verleihen, können als selektierbare Marker verwendet werden.

Als Beispiele für Gene, die Antibiotikaresistenz verleihen, seien das Neomycin-Phosphotransferase-Gen (Kanamycin- und G418-Resistenz, Velten et al., 1984), das Hygromycin-Phosphotransferase-Gen (Hygromycinresistenz, van den Elzen et al., 1985) sowie das Chloramphenicol-Acetyltransferase-Gen genannt.

Als Beispiel für ein Gen, das in erster Linie als screenbarer Marker in Gewebekulturen für die Identifizierung von pflanzlichen Zellen geeignet ist, die einen genetisch modifizierten Vektor enthalten, sei ein Gen genannt, das ein Enzym mit einem chromogenen Substrat kodiert. Handelt es sich dabei beispielsweise um ein Gen, welches das Enzym β-Galaktosidase kodiert, so werden die transformierten pflanzlichen Zellen auf einem Gewebekultur-Medium ausplattiert, welches das chromogene Substrat Xgal (5-Chlor-4-brom-3-indolyl-β-D-galaktosid) enthält. Pflanzenzellen, die Kopien des β-Galaktosidase Gens enthalten, werden unter geeigneten Bedingungen durch das aufgrund der enzymatischen Spaltung von Xgal freigesetzte Indigo blau gefärbt.

Die Einschleusung chimärer Gene in die Pflanzen kann erfindungsgemäss unter Verwendung eines beliebigen T-DNA Vektorsystems durchgeführt werden, sofern besagtes Vektorsystem in der Lage ist, Gene von *Agrobacterium* auf Baumwollpflanzenzellen zu übertragen.

Bei dem oben erwähnten Vektorsystem kann es sich beispielsweise um ein co-integriertes System handeln (Comai et al, 1983; Zambryski et al, 1983), wie das "split-end" Vektorsystem (Fraley et al, 1985), das bei Chilton (1985) beschrieben ist.

Als Vektorsystem kann auf der anderen Seite auch ein binäres System verwendet werden (deFramond et al, 1983; Hoekema et al, (1983) oder ein Ti-Plasmid, welches das Gen in die T-DNA eingebaut (Matzke und Chilton, 1981) enthält.

Eine weitere Möglichkeit bildet ein System, worin die T-DNA auf einem Plasmid vorliegt, während sich die Virulenzgene auf der chromosomalen DNA befinden.

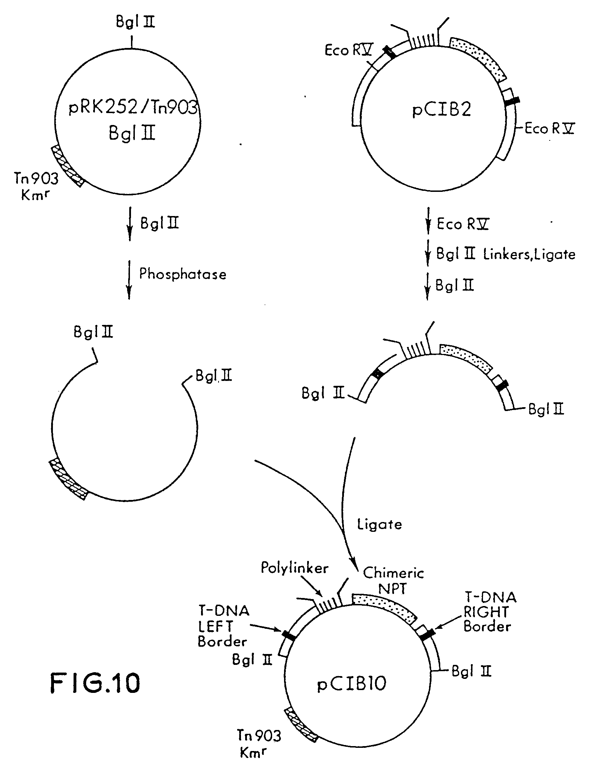

Bevorzugt im Rahmen dieser Erfindung ist ein binäres Vektorsystem und zwar insbesondere ein System, welches das Plasmid pCIB10 (Rothstein et al, 1987) beinhaltet (siehe Fig. 10).

Der Einbau heterologer Gene in ein binäres Vektorsystem mit Hilfe der rekombinanten DNA Technologie ist bei Klee et al (1985) beschrieben. Das Einspleissen der Gene in einen T-DNA Vektor kann durch homologe Rekombination unter Verwendung einer doppelten Rekombinationsstrategie (Matzke und Chilton, 1981), einer einfachen Rekombinationsstrategie (Comai et al, 1983; Zambryski et al, 1983), oder aber einer einfachen Rekombinationsstrategie ohne Wiederholungen in der T-DNA (Fraley et al, 1985) erfolgen. Letzteres ist bei Chilton et al (1985) beschrieben.

Werden die das chimäre Gen enthaltenden Vektoren ausserhalb der *Agrobacterium*-Zelle zusammengebaut, so können diese mit Hilfe an sich bekannter Methoden, wie beispielsweise einer Transformation oder einer Konjugation, in *Agrobacterium* eingeschleust werden.

Bei der Transformation werden die Bakterien mit nackter DNA inkubiert. Die Aufnahmefähigkeit der *Agrobacterium*-Zellen für nackte DNA kann durch Einfrieren und anschliessendes Auftauen verbessert werden. Die Transformation von *Agrobacterium* ist bei Holsters et al (1978) beschrieben.

Bei der Konjugation kommt es zu einer Paarung einer den gewünschten Vektor enthaltenden Zelle, gewöhnlich einer *E. coli* Zelle, mit *Agrobacterium*. Diese Methode ist bei Comai et al (1983), sowie bei Chilton et al (1976) beschrieben.

Für den erfindungsgemässen Verwendungszweck kann jede beliebige *Agrobacterium*-Spezies (*Agrobacterium* spp.) verwendet werden, die in der Lage ist, Gene auf Baumwollpflanzenzellen zu übertragen. Geeignete *Agrobacterium*-Spezies sind beispielsweise *A. tumefaciens*, *A. rhizogenes* und *A. radiobacter*.

Transformierte Baumwollpflanzenzellen, die das erfindungsgemässe chimäre Gen enthalten, können entweder in Kultur gehalten oder aber zu ganzen lebenden Pflanzen regeneriert werden. Die Expression erfolgt vorzugsweise mit einer Effizienz, die ausreicht, den pflanzlichen Zellen eine insektizide Wirksamkeit zu verleihen.

Das für die erfindungsgemässe Verwendung in Frage kommende Medium muss in der Lage sein, eine bestimmte pflanzliche Zelle in Kultur am Leben zu erhalten. Seine Zusammensetzung ist abhängig von der jeweils verwendeten Baumwoll-Varietät.

Einige der für den erfindungsgemässen Verwendungszweck geeignete Medien enthalten beispielsweise etwa 10 mg/Liter 2,4-Dichlorphenoxyessigsäure sowie entweder die anorganischen Salze des Murashige und Skoog Mediums (Murashige und Skoog, 1962) oder des Gamborg B-5 Mediums (Gamborg et al., 1968).

Zur Keimung und zur Regenerierung von Pflanzen befähigte Baumwoll (*Gossypium* spp.)-Embryonen lassen sich in grosser Zahl durch somatische Embryogenese herstellen, indem man von proembryonalen Zellmassen ausgeht und daraus in einem Zellsuspensionskultursystem Embryonen heranzieht.

Mit diesem Verfahren kann man beispielsweise in einem Standard-250 ml-Delong-Gefäss etwa 10 000 globuläre Embryonen erzeugen, aus denen etwa 1000 reife Embryonen und daraus wiederum etwa 50 Pflanzen entstehen. Dabei kann es sich um Zuchtformen oder Wildformen von Baumwollpflanzen handeln. Zuchtformen sind bevorzugt.

Schritt a: Embryogener Baumwollkallus

Der erste Schritt besteht in der Induktion der Baumwollkallusbildung aus verpflanztem Baumwollgewebe. Einige Beispiele von geeignetem verpflanztem Baumwollgewebe sind somatische Embryonen, reife und unreife zygotische Embryonen, Keimblätter oder Hypokotyle eines Sämlings und junges Gewebe einer reifen Pflanze. Somatische Embryonen und Sämlingskeimblätter oder -hypokotyle sind dabei bevorzugt.

Beispielsweise können zygotische Embryonen durch Herausschneiden aus Samenanlagen erhalten werden. Die Samenanlagen werden vorzugsweise etwa 7 bis 30 Tage nach der Bestäubung herausgeschnitten, vorzugsweise etwa 10 bis 21 Tage nach der Bestäubung und insbesondere etwa 12 bis 16 Tage nach der Bestäubung.

Keimblätter und Hypokotyle kann man jungen Sämlingen entnehmen. Die Sämlinge sind normalerweise etwa 3 bis 21 Tage alt, bevorzugt etwa 4 bis 9 Tage, insbesondere etwa 7 Tage alt. Hypokotyle werden längs aufgeschnitten und in passende Abschnitte geteilt, beispielsweise in Abschnitten von 1 bis 20 mm, bevorzugt von etwa 2 mm Länge. Das Keimblattgewebe wird in Stücke mit einer Fläche von etwa 1 bis 400 mm² geschnitten, bevorzugt von etwa 5 bis 100 mm², insbesondere von etwa 10 mm².

Somatische Embryonen, die sich von dieser Vorgehensweise ableiten, sind eine ganz besonders bevorzugte Quelle, um embryogenen Kallus gemäss dem vorliegenden Verfahren zu erhalten.

Somatische Embryonen können beispielsweise erhalten werden, indem das Verfahren angewendet wird, das oben für Hypokotyl- und Keimblattgewebe als Quelle des verpflanzten Gewebes beschrieben ist. Jeder somatische Embryo, der vor der Entfaltung des Primärblattes genommen wird, ist geeignet. Die Grösse des somatischen Embryos ist nicht kritisch. Bevorzugt sind jedoch somatische Embryonen von weniger als 5 mm Länge.

Junggewebe von ausgewachsenen Baumwollpflanzen wird normalerweise dadurch erhalten, dass man die apikalen 10 cm, vorzugsweise etwa 5 cm der Sprossspitze entnimmt. Stengel und Petiolengewebe werden längs aufge schnitten und in Abschnitte derselben Grösse wie bei den Hypokotylen (siehe oben) geteilt. Blattgewebe wird in gleich grosse Stücke geschnitten wie das Keimblattgewebe (siehe oben).

Das Baumwollpflanzengewebe wird auf ein zur Kallusinduktion geeignetes Medium, bei etwa 20° bis 40°C, vorzugsweise 23° bis 35°C, insbesondere etwa 31°C gelegt. Jedes Medium, das aus einem Gewebe einen Kallus induzieren kann, kann in diesem Regenerationsverfahren eingesetzt werden. Das Medium kann flüssig oder fest sein, obwohl ein festes Medium bevorzugt ist, weil es bequemer zu handhaben ist.

Ein gemäss vorliegender Erfindung gebräuchliches, die Kallusbildung induzierendes Medium enthält im allgemeinen anorganische Salze sowie Vitamine, eine Kohlenstoffquelle, ein Auxin und ein Cytokinin. Dieses Medium wird auf einen pH zwischen 3,5 und 7,5, bevorzugt zwischen 4,5 und 6,5, insbesondere auf etwa 5,7 eingestellt.

Es sind an sich alle anorganischen Salze sowie Vitamine geeignet, die die Kallusinduktion fördern. Einige Beispiele von geeigneten anorganischen Salzen und von Vitaminen werden von Murashige und Skoog (1962) (MS) sowie Gamborg et al. (1968) (B-5) beschrieben. Ein weiteres Beispiel dafür ist die Modifikation des MS- oder Gamborgs B-5-Mediums, das Cheng et al. (1980) beschreiben. Die bevorzugten anorganischen Salze sind MS anorganische Salze. Die bevorzugten Vitamine sind Gamborgs B-5 Vitamine.

Als geeignete Kohlenstoffquelle kann jede Kohlenstoffquelle eingesetzt werden, auf der Kallus wachsen kann. Bevorzugte Kohlenstoffquellen sind Zucker und Derivate von Zuckern. Bevorzugte Zucker sind Glucose und Saccharose. Es ist besonders wünschenswert, Kallus in einem Kallusinduktionsmedium zu initiieren, das Glucose enthält, damit das Braunwerden des Gewebes verringert wird, und dann den Kallus in ein Kallusinduktionsmedium, das Saccharose enthält, zu übertragen.

Die Konzentration der Kohlenstoffquelle ist 5 bis 60 g/Liter, bevorzugt etwa 30 g/Liter.

Das im Kallusinduktionsmedium vorhandene Auxin kann jedes Auxin sein, das einen Kallus induzieren kann. Einige geeignete Auxine umfassen α-Naphthalinessigsäure, Picloram, 2-4,5-Trichlorphenoxyessigsäure, 2,4-Dichlorphenoxyessigsäure, Indol-3-buttersäure, Indol-3-milchsäure, Indol-3-bernsteinsäure, Indol-3-essigsäure und p-Chlorphenoxyessigsäure, Indol-3-essigsäure und p-Chlorphenoxyessigsäure. Ein bevorzugtes Auxin ist α-Naphthalinessigsäure

Jede Auxinkonzentration, die die Kallusbildung induzieren kann, kann im erfindungsgemässen Verfahren verwendet werden. Besonders geeignete Konzentrationen liegen bei 0,1 bis 10 mg/Liter, insbesonders wenn α-Naphthalinessigsäure als Auxin eingesetzt wird.

Das im Kallusinduktionsmedium vorhandene Cytokinin kann jedes Cytokinin sein, das einen Kallus induzieren kann. Einige geeignete Cytokinine umfassen Kinetin, 6-Benzyladenin, 2-Isopentenyladenin und Zeatin. Ein bevorzugtes Cytokinin ist Kinetin.

Jede Cytokininkonzentration, die die Kallusbildung induzieren kann, kann im Verfahren der Erfindung eingesetzt werden. Geeignete Konzentrationen liegen bei 0,1 bis 10 mg/Liter. Eine bevorzugte Konzentration ist 1 mg/Liter, insbesonders wenn das Cytokinin Kinetin ist.

Wenn das Medium fest ist, enthält es als Verfestigungsmittel beispielsweise etwa 0,8 % Agar, wie Agar Noble (Difco), oder etwa 0,8 % Agarose. (Alle Prozentangaben in dieser Beschreibung beziehen sich auf das Gewicht.)

Das Gewebe wird einige Zeit auf dem Kallusinduktionsmedium kultiviert, und zwar so lange, bis sich der Kallus bildet. Man kann beispielsweise Gewebe auf einem Kallusinduktionsmedium kultivieren, das Glucose als Kohlenstoffquelle enthält. Eine fünfwöchige Induktionsdauer ist typisch. Ueberimpfungen in frisches Medium und weitere Kultivierung werden durchgeführt, weil sie nötig sind, um Braunwerden zu verhindern. Wöchentliche Ueberimpfungen sind bevorzugt.

Der Kallus, der sich bildet, kann unorganisiert sein, oder er kann proembryonale Zellmassen, embryogenen Kallus oder auch Embryonen enthalten. Normalerweise, wenn Hypokotyle oder Keimblätter als Quelle für das verpflanzte Gewebe verwendet werden, scheint sich bevorzugt unorganisierter Kallus zu bilden. Wenn somatische Embryonen als Quelle für das verpflanzte Gewebe verwendet werden, scheint wenigstens ein Teil des Kallus aus einem embryogenen Kallus zu bestehen, welcher durch eine leicht gelbe Farbe und Knotenbildung charakterisiert ist.

Der resultierende Kallus kann anschliessend für eine Zeitspanne von bis zu 5 Monaten vorteilhafterweise auf ein Kallusweiterkulturmedium übertragen werden, das dem Kallusinduktionsmedium ähnlich . ist, aber Saccharose als Kohlenstoffquelle enthält. Man kultiviert den Kallus vorzugsweise für einen Zeitraum von einem Monat auf einem Kallusinduktionsmedium, das Saccharose enthält, oder für einen Zeitraum von zwei Monaten, sollte dann jedoch nach einem Monat auf frisches Medium überimpfen.

Der Kallus kann im Dunkeln induziert werden, wird aber vorzugsweise im Licht induziert. Das Licht kann eine Intensität von beispielsweise 0,5 bis 150 μE m$^{-2}$s$^{-1}$ (= 41,75 bis 12525 1x) haben.

## Schritt (b): Klumpige Zusammenballungen von proembryonalen Zellmassen

Der Kallus von Schritt (a) wird in einem Flüssigmedium suspendiert, das die Entwicklung von proembryonalen oder sich vermehrenden embryonalen Zellmassen fördert. Es ist wichtig, dass die Zelldichte niedrig ist. Deshalb wird nicht mehr als 40 mg Kallus/ml Kulturmedium, bevorzugt nicht mehr als 15 mg Kallus/ml Kulturmedium und insbesondere nicht mehr als 5 mg Kallus/ml Kulturmedium suspendiert.

Das Medium, brauchbar in Schritt (b), kann jedes Medium sein, das proembryonale Zellmassen induzieren kann. Das Medium enthält im allgemeinen anorganische Salze sowie Vitamine, eine Kohlenstoffquelle und ein Auxin. Das Medium kann auch organische Stickstoffquellen, Cytokinine, Aminosäuren und andere Zusätze enthalten, wie Kaseinhydrolysat oder Kokosmilch.

Die anorganischen Salze und die Vitamine können die gleichen sein wie unter Schritt (a), weiter oben. MS anorganische Salze und B-5-Vitamine sind bevorzugt.

Die Kohlenstoffquelle kann die gleiche sein wie die in Schritt a beschriebene. Saccharose ist bevorzugt. Die Konzentration der Kohlenstoffquelle ist 0,1 bis 100 g/Liter. Etwa 20 g/Liter ist bevorzugt, besonders wenn die Kohlenstoffquelle Saccharose ist.

Das Auxin kann aus den Auxinen, die in Schritt (a) verwendet werden, ausgewählt sein. Die bevorzugten Auxine sind 2,4-Dichlorphenoxyessigsäure und Picloram. Picloram ist besonders bevorzugt.

Die Konzentration des Auxins in Schritt (b) ist relativ niedrig. Die genaue Konzentration hängt von dem spezifischen, verwendeten Auxin ab. Die relativ niedrige Auxinkonzentration ist im allgemeinen ähnlich der, die gewöhnlicherweise in Suspensionskulturmedien verwendet wird, und ist bedeutend niedriger als die entsprechende Auxinkonzentration, die in Schritt (c) verwendet wird. Wenn Picloram das in Schritt (b) verwendete Auxin ist, ist die Konzentration 0,01 bis 5 mg/Liter, bevorzugt 0,1 bis 1 mg/Liter und insbesondere etwa 0,5 mg/Liter. Wenn 2,4-Dichlorphenoxyessigsäure das in Schritt (b) verwendete Auxin ist, ist die Konzentration 0,01 bis 0,5 mg/Liter, bevorzugt 0,05 bis 0,25 mg/Liter und insbesondere etwa 0,1 mg/Liter.

Die Induktion proembryonaler Zellmassen wird vozugsweise in einem belüfteten Medium bei einer Temperatur zwischen 20° und 35°C durchgeführt. Bevorzugt sind Temperaturen zwischen 22° und 33°C und insbesondere zwischen 25° und 31°C. Das Medium kann auf jede im Stand der Technik bekannte Weise belüftet werden, beispielsweise durch Schütteln. Schritt (b) kann im Dunkeln durchgeführt werden oder im Licht von bis zu 75 μE m$^{-2}$s$^{-1}$ (= 6262,5 1x), bevorzugt zwischen 5 und 10 μE m$^{-2}$s$^{-1}$ (= 417,5 und 835 1x).

Der Kallus wird vorzugsweise ohne Ueberimpfen in dem Medium gehalten, bis sich klumpige Zusammenballungen von proembryonalen Zellmassen bilden und beginnen, sich rasch zu teilen. Der Ausbruch rascher Teilung erfolgt gewöhnlich nach 3 bis 8 Wochen, typischerweise nach 5 bis 7 Wochen. Während der Induktionsperiode kann das Medium an sich durch frisches Medium ersetzt werden, obwohl es vorzuziehen ist, das Medium während dieser Periode nicht zu ersetzen.

Der Wechsel von Kallus zu klumpigen Zusammenballungen proembryonaler Zellmassen ist für den Fachmann der Pflanzengewebekultur leicht erkennbar. Er ist durch die leicht gelbe Farbe und die klumpige Natur der proembryonalen Zellmassen gekennzeichnet.

Wenn die klumpigen Zusammenballungen der proembryonalen Zellmassen erst einmal begonnen haben, sich rasch zu teilen, können sie direkt in das in Schritt (c) beschriebene Medium eingebracht werden oder in frisches Medium überimpft werden, um das Braunwerden zu verhindern. Ein Ueberimpfen im Zeitraum von 3 bis 7 Tagen, bevorzugt alle 5 bis 7 Tage ist günstig. Ohne Ueberimpfen überleben die Zellmassen etwa vierzehn Tage.

Schritt c: Fein dispergierte proembryonale Zellmassen

Die klumpigen Zusammenballungen proembryonaler Zellmassen von Schritt (b) werden in ein Flüssigmedium übertragen, das dazu beiträgt, dass die klumpigen Zusammenballungen der proembryonalen Zellmassen fein dispergiert sind. Das Medium kann dem in Schritt (b) beschriebenen ähnlich sein, sollte aber eine relativ hohe Auxinkonzentration aufweisen. Als Auxin kann jedes in Schritt (a) verwendete Auxin eingesetzt werden. Bevorzugte Auxine sind 2,4,5-Trichlorphenoxyessigsäure und 2,4-Dichlorphenoxyessigsäure. Insbesonders bevorzugt ist 2,4-Dichlorphenoxyessigsäure.

Die Auxinkonzentration hängt von dem einzelnen Auxin ab. Die Auxinkonzentration im Medium von Schritt (c) ist generell höher oder wenigstens am oberen Ende des Konzentrationsbereichs, der gewöhnlicherweise in Suspensionskulturmedien verwendet wird; und ist auf jeden Fall bedeutend höher als die entsprechende Auxinkonzentration in Schritt (b).

Wenn beispielsweise 2,4-Dichlorphenoxyessigsäure als Auxin in Schritt (c) eingesetzt wird, kann die Konzentration bei etwa 0,5 bis 100 mg/Liter liegen, bevorzugt bei 1 bis 10 mg/Liter und insbesondere bei etwa 2,5 bis 7,5 mg/Liter.

Bis auf die Konzentration und möglicherweise das gewählte Auxin können Medium, Temperatur und Lichtmenge in Schritt (c) gleich sein wie in Schritt (b).

Die Bedingungen von Schritt (c) werden aufrecht erhalten, bis die klumpigen Zusammenballungen der proembryonalen Zellmassen zu kleineren, feiner dispergierten proembryonalen Zellmassen werden. Das Erscheinen der kleineren, feiner dispergierten proembryonalen Zellmassen ist für den Fachmann leicht erkennbar. Diese Zellmassen sind durch ihre gelbe Farbe, glatte Oberfläche, mittlere Dichte und geringe Grösse charakterisiert. Der Uebergang zu den kleineren, feiner dispergierten Zellmassen geschieht gewöhnlich innerhalb von 6 Wochen, typischer noch innerhalb von 2 Wochen.

Die Kultur der kleinen, fein dispergierten proembryonalen Zellmassen kann unbegrenzt aufrecht erhalten werden, und kann überimpft werden, um aktives Wachstum zu erhalten. Es ist günstig, beispielsweise alle 3 bis 28 Tage, bevorzugt alle 5 bis 10 Tage zu überimpfen.

Schritt d: Reife Embryonen

Die kleineren, feiner dispergierten proembryonalen Zellmassen werden zu einem Medium, das die Entwicklung reifer Embryonen induziert, hinzugefügt. Das Medium ist vorzugsweise flüssig.

Embryonen durchlaufen eine Anzahl von Entwicklungsstadien, bevor sie reif und fähig sind, zu keimen. Diese Stadien umfassen globuläre, herz- und torpedoförmige sowie reife Stadien. Die Bezeichnungen der Stadien basieren auf dem ungefähren Aussehen der Embryonen.

Das in Schritt (d) brauchbare Medium kann jedes Medium sein, dass die Entwicklung reifer Embryonen zu induzieren vermag. Ein brauchbares Medium enthält im allgemeinen anorganische Salze sowie Vitamine, eine Kohlenstoffquelle und eine organische Verbindung, die reduzierten Stickstoff enthält.

Die Salze und Vitamine sowie deren Konzentrationen können die gleichen sein wie bei Schritt (a). Die Kohlenstoffquelle kann auch wie in Schritt (a) gewählt sein. Die Konzentration der Kohlenstoffquelle ist etwa 1 bis 10 g/Liter, bevorzugt etwa 2 bis 6 g/Liter. Eine bevorzugte Kohlenstoffquelle ist Saccharose.

Die organische Stickstoffquelle kann jede solche Verbindung sein, die, wenn sie dem Medium von Schritt d hinzugefügt wurde, die Entwicklung reifer Embryonen induziert. Die bevorzugten Verbindungen sind Aminosäuren. Eine bevorzugte Aminosäure ist Glutamin.

Die Konzentration der organischen Stickstoffquelle hängt von der eingesetzten Verbindung ab. Eine wirksame Konzentration von Glutamin als organischer Stickstoffquelle liegt bei 2 bis 260 mM, bevorzugt 5 bis 100 mM, insbesondere 10 bis 50 mM.

Das Medium von Schritt (d) kann ein Auxin enthalten. Auxine sind erstrebenswert während der frühen Stadien der Embryonalentwicklung, aber nicht während der späteren Stadien. Deshalb sind sie, falls überhaupt anwesend, vorzugsweise nur bis zum herzförmigen Stadium der Entwicklung einzusetzen. Danach werden die Embryonen in ein Medium, das kein Auxin enthält, übertragen.

Falls vorhanden, kann die Auxinkonzentration bei 0,01 bis 0,1 mg/Liter liegen.

Das Auxin kann eines der in Schritt (a) brauchbaren Auxine sein. Die bevorzugten Auxine sind Picloram und 2,4-Dichlorphenoxyessigsäure.

Die Embryonen können sich im Medium von Schritt (d) bei Temperaturen von 20° bis 35° C im Dunkeln oder im Licht entwickeln. Die Lichtintensität kann beispielsweise 5 bis 75 $\mu$E m$^{-2}$s$^{-1}$ ( = 6262,5 1x) betragen.

Die Embryonen werden im Medium von Schritt (d) gehalten, bis die Embryonen zu torpedoförmigen oder reifen Stadien herangewachsen sind. Für den mit Pflanzengewebekulturen umgehenden Fachmann sind die globulären, herz-und torpedoförmigen sowie reifen Embryonen klar und einfach zu erkennen. Die Embryonen reifen typischerweise in etwa 2 bis 5 Wochen, gewöhnlicherweise in etwa 3 bis 4 Wochen. Es ist gewöhnlich unnötig, die Embryonen ein weiteres Mal zu kultivieren bzw. die Embryonen in frisches Medium zu übertragen.

Nur im herzförmigen Stadium könnte es vorteilhaft sein, wenn man von einem Auxin-enthaltenden Medium zu einem-Medium, das kein Auxin enthält, überwechselt.

### Schritt e: Keimung

Die reifen Embryonen werden auf ein festes Medium aufgebracht, das Keimung induziert. Das Medium enthält anorganische Salze sowie Vitamine und eine Kohlenstoffquelle. Das Medium ist mit einem gebräuchlichen Verfestigungsmittel, wie Gelrite (Kelko, San Diego, Kalifornien), Agarose oder Agar, verfestigt.

Die anorganischen Salze können diejenigen aus Schritt (a) sein, wobei das Nitrat in hohen Konzentrationen vorliegt, während Ammonium fehlt oder in sehr niedrigen Konzentrationen vorliegen kann. Die Nitratkonzentration beträgt normalerweise 20 bis 60 mM, bevorzugt 30 bis 60 mM, insbesondere 35 bis 45 mM. Die Konzentration der Ammoniumionen sollte 5 mM nicht übersteigen.

Als Kohlenstoffquelle kommt vorzugsweise ein Zucker in Frage. Ein bevorzugter Zucker ist Saccharose. Die Konzentration der Kohlenstoffquelle hängt von der Art der verwendeten Kohlenstoffquelle ab. Wenn beispielsweise Saccharose als Kohlenstoffquelle eingesetzt wird, beträgt deren Konzentration 0,1 bis 6 % (Gewichtsprozente), bevorzugt 0,5 bis 4 %, insbesondere 1 bis 3 %.

Im Medium von Schritt (e) ist fakultativ eine organische Verbindung, die reduzierten Stickstoff enthält, vorhanden. Als bevorzugte organische Verbindungen kommen Aminosäuren oder deren Gemische in Frage, die befähigt sind, die Keimung zu unterstützen. Zu den bevorzugten Aminosäuren oder Mischungen davon gehören Glutamin und Kaseinhydrolysat.

Die Konzentration der organischen Stickstoffquelle hängt im allgemeinen von der Art der speziell verwendeten Verbindung ab. Wenn die Verbindung beispielsweise Glutamin ist, kann deren Konzentration 2 bis 50 mM sein, liegt jedoch bevorzugt bei 5 bis 30 mM, insbesondere bei 10 bis 20 mM. Wenn die Verbindung Kaseinhydrolysat oder modifiziertes Kaseinhydrolysat ist, liegt die Konzentration bei 100 bis 3000 mg/Liter, bevorzugt 1000 bis 2800 mg/Liter, insbesondere bei 1500 bis 2500 mg/Liter.

Die Keimung wird bis zur Sprossbildung auf einem Medium durchgeführt, das eine organische Stickstoffquelle enthält. Zur Förderung des Längenwachstums der Wurzeln werden die Embryonen dann auf ein weiteres Medium überführt, das aber keine organische Stickstoffquelle enthält.

Die Dichte der Embryonen in diesem Medium wird so begrenzt, dass sie geringer ist als die Dichte, die dafür sorgt, dass die Entwicklung selbsthemmend ist. Geeignete Dichten sind beispielsweise 1 bis 100 Embryonen in einer 9 cm Petrischale, die etwa 10 bis 75 ml, bevorzugt 25 bis 50 ml, insbesonders etwa 35 ml Medium enthält.

Das Medium bzw. die Medien von Schritt (e) werden bei 20° bis 30°C gehalten. Die Temperatur beträgt vorzugsweise etwa 25°C (Raumtemperatur).

In Schritt (e) wird im allgemeinen etwas Licht benötigt. Geeignete Lichtintensitäten liegen zwischen 5 und 150 $\mu$E m$^{-2}$s$^{-1}$ ($= 417,5$ bis 12525 1x), vorzugsweise zwischen 10 und 75 $\mu$E m$^{-2}$s$^{-1}$ ($= 835$ bis 6262,5 1x).

Bis zur Keimung werden die Embryonen auf dem Medium bzw. den Medien von Schritt (e) gehalten; typischerweise 1 bis 20 Tage, üblicherweise 2 bis 4 Tage. Für den Fachmann ist ein gekeimter Embryo leicht erkennbar.

### Schritt f: Pflanzen

Nach der Keimung werden die Pflänzchen in Erde eingebracht und bis zur Reife weiterkultiviert. Die ausgepflanzten Pflänzchen werden anfangs mit Glas abgedeckt, um eine hohe Feuchtigkeit aufrechtzuerhalten. Nach etwa einer Woche unter Glas ist keine besondere Behandlung der Pflänzchen oder Pflanzen mehr nötig.

### Brauchbarkeit - Vermehrung

Reife Embryonen können für Massenvermehrung und Klonierung verwendet werden. Dazu bedarf es der Keimung der Embryonen und des Auspflanzens der Pflänzchen in Erde, in andere Wachstumssubstrate oder andere Wachstumsumgebungen. Reife Embryonen können auch von einem künstlichen Samenmantel umschlossen und als "somatische Samen" ausgesät werden. Massenvermehrung und Klonierung ist nutzbringend, wenn Hybrideltern oder ein Hybrid selbst in Massen produziert werden müssen.

Zellen, Proembryonen, Embryonen, Pflänzchen und Pflanzen können zu jeder Zeit während der oben beschriebenen Stadien analysiert werden, um zu bestimmen, ob irgendeine neue Eigenschaft als Folge genetischer Veränderungen vorhanden ist. Die Eigenschaft kann eine nützliche in vitro oder in planta Eigenschaft sein. Einige Beispiele von nützlichen Eigenschaften sind Phytotoxintoleranz, Trockentoleranz, Kältetoleranz, Krankheitstoleranz, etc.

Die aus den Schritten (a), (b) und (c) resultierenden Pflanzenzellen können auch in Gewebekulturverfahren eingesetzt werden, bei denen Pflanzen mit wünschenswerten Eigenschaften, wie beispielsweise Herbizidtoleranz, erzeugt werden sollen. Einige Beispiele solcher Verfahren sind beispielsweise in Chaleff und Ray (1984) beschrieben.

Die vorliegende Erfindung umfasst somit weiterhin lebende Baumwollpflanzen, deren Zellen das erfindungsgemässe chimäre Gen enthalten, welches ein Polypeptid kodiert und exprimiert, das im wesentlichen die Insektentoxizitätseigenschaften des Bt Kristallproteins besitzt.

Die erfindungsgemässen Pflanzenzellen enthalten das chimäre Gen und können zur Herstellung eines Polypeptids verwendet werden, das im wesentlichen die Insektentoxizität von Bt aufweist. Die Pflanzenzellen können per se das Insektizid darstellen. Bei den direkt als Insektizid genutzten Pflanzenzellen kann es sich um

23

kultivierte Pflanzenzellen oder um Bestandteile einer lebenden Pflanze handeln.

Das Toxin kann auch mit Hilfe bekannter Verfahren, wie beispielsweise durch Extraktion oder Chromatographie, direkt aus Pflanzenzellen isoliert werden. Der Extrakt kann der gesamte Pflanzenzellextrakt, ein teilweise gereinigter Extrakt oder eine reine Zubereitung des Polypeptids sein. Jeder auf diese Weise gewonnene Extrakt oder jedes chromatographische Isolat kann auf die gleiche Weise wie das kristalline Protein von *Bt* verwendet werden (siehe beispielsweise Deacon, 1983; Miller et al., 1983).

Die erfindungsgemässen insektiziden Zellen sind toxisch für Insekten, die Baumwollzellen und -pflanzen angreifen.

Somit stellt die vorliegenden Erfindung ein Verfahren zur Herstellung eines Polypeptids, das im wesentlichen die Toxizitätseigenschaften eines *Bt* Kristallproteins aufweist, in Baumwollpflanzen bereit, das die folgenden Verfahrensmassnahmen umfasst:

a) Einschleusung eines Gens in Baumwollzellen, das ein Polypeptid kodiert welches im wesentlichen die Insektentoxizitätseigenschaften eines *Bt* Kristallproteins aufweist, worin der Promotor, die 5'-nichttranslatierte Region und gegebenenfalls die 3'-nichttranslatierte Region des Gens von einem Pflanzengen oder einem Pflanzenvirusgen stammen und

b) Expression des Polypeptids.

Die vorliegende Erfindung umfasst weiterhin eine Methode zur Kontrolle von Insektenlarven, die sich dadurch kennzeichnet, dass die Larven mit einer insektizid wirksamen Menge an transgenen Baumwollzellen gefüttert werden, die ein Gen enthalten, das ein kristallines *Bt* Toxin kodiert oder ein Polypeptid, welches im wesentlichen die Insektentoxizitätseigenschaften eines *Bt* Kristallproteins aufweist.

Ebenso ein Bestandteil der vorliegenden Erfindung ist ein Verfahren zur Abtötung oder Kontrolle von Insektenlarven, das sich dadurch kennzeichnet, dass die Larven mit einer insektizid wirksamen Menge an transgenen Baumwollzellen gefüttert werden, die das erfindungsgemässe chimäre Gen enthalten.

Darüberhinaus umfasst die vorliegende Erfindung ein Verfahren zur Abtötung oder Kontrolle von Coleopteren-Larven, das sich dadurch kennzeichnet, dass die Larven mit einer insektizid wirksamen Menge der erfindungsgemässen, transgenen, insektiziden Baumwollzellen gefüttert werden, wobei die erfindungsgemässen, insektiziden Bauwollzellen das chimäre Gen, das das kristalline Toxin von *Bt* var. *tenebrionis* oder mindestens einen insektizid wirksamen Teil davon kodiert, exprimieren.

Die Pflanzenzellen können kultivierte Pflanzenzellen oder Bestandteile lebender Pflanzen sein.

Die vorliegende Erfindung umfasst weiterhin Baumwoll-Samen von erfindungsgemäss genetisch veränderten Pflanzen, sofern die Samen das eingefügte Gen und die daraus resultierende Eigenschaft enthalten. Die Nachkommen von Pflanzen, die mit Hilfe des erfindungsgemässen Verfahrens hergestellt werden, einschliesslich sexuell und vegetativ erzeugter Nachkommen, sind weitere Gegenstände dieser Erfindung. Sexuell erzeugte Nachkommenschaft kann aus Selbst- oder Fremdbestäubung resultieren.

## Nichtlimitierende Ausführungsbeispiele

## Allgemeine Verfahren der rekombinanten DNA-Technologie

Da viele der im Rahmen dieser Erfindung verwendeten Techniken für den Fachmann auf dem Gebiet der rekombinanten DNA-Technologie Routine sind, soll im Folgenden eine kurze zusammenfassende Beschreibung der gebräuchlichsten Verfahren gegeben werden, sodass diese in den nachfolgenden konkreten Ausführungsbeispielen nicht jedesmal wieder neu angegeben werden müssen.

Alle diese Routineverfahren sind bei Maniatis et al (1982) beschrieben, es sei denn, es wird gesondert darauf hingewiesen.

## A. Schneiden mit Restriktionsendonukleasen

Typischerweise sind in dem Reaktionsansatz etwa 50 bis 500 µg/ml DNA in der vom Hersteller, New England Biolabs, Beverly, MA., empfohlenen Pufferlösung enthalten. 2 bis 5 Einheiten an Restriktionsendonukleasen werden für jedes µg DNA hinzugefügt und der Reaktionsansatz wird bei der vom Hersteller empfohlenen Temperatur für eine bis drei Stunden inkubiert. Die Reaktion wird durch 10 minütiges Erhitzen auf 65°C oder durch Extraktion mit Phenol beendet; es folgt eine Präzipitation der DNA mit Ethanol. Diese Technik wird auch auf den Seiten 104 bis 106 der Maniatis et al.-Referenz beschrieben.

## B. Behandlung der DNA mit Polymerase zur Herstellung glatter Enden

DNA-Fragmente werden in dem vom Hersteller, New England Biolabs, empfohlenen Puffer in einer Konzentration von 50 bis 500 µg/ml zu einem Reaktionsansatz hinzugefügt. Der Reaktionsansatz enthält alle vier Desoxynukleotidtriphosphate in einer Konzentration von 0.2 mM. Die Reaktion erfolgt während 30 Minuten bei 15°C und wird anschliessend durch 10 minütiges Erhitzen auf 65°C beendet. Für Fragmente, die durch Schneiden mit Restriktionsendonukleasen erhalten werden, welche 5'-überstehende Enden erzeugen, wie EcoRI und BamHI, wird das grosse Fragment, oder Klenow-Fragment, der DNA-Polymerase verwendet. Für Fragmente, die durch Endonukleasen erhalten werden, welche 3'-überstehende Enden erzeugen, wie PstI und SacI, wird die T4-DNA-Polymerase verwendet. Die Verwendung dieser beiden Enzyme wird auf den Seiten 113 bis 121 der Maniatis et al.-Referenz beschrieben.

## C. Agarose-Gelelektrophorese und Reinigung von DNA-Fragmenten aus den Gelen

Die Agarose-Gelelektrophorese wird in einem horizontalen Apparat durchgeführt, wie auf den Seiten 150 bis 163 der Maniatis et al-Referenz beschrieben. Als Puffer wird der dort beschriebene Tris-Boratpuffer verwendet. Die DNA-Fragmente werden durch 0.5 µg/ml Ethidiumbromid gefärbt, das entweder im Gel- oder Tankpuffer bereits während der Elektrophorese vorhanden ist oder aber erst nach der Elektrophorese zugegeben wird. Die DNA wird durch Beleuchtung mit kurz- oder langwelligem Ultraviolettlicht sichtbar gemacht. Wenn die Fragmente nicht vom Gel abgetrennt werden sollen, wird eine Agarose verwendet, die bei niedriger Temperatur geliert und von Sigma Chemical, St. Louis, - Missouri, bezogen werden kann. Nach der Elektrophorese wird das gewünschte Fragment ausgeschnitten, in ein Plastikröhrchen gegeben, etwa 15 Minuten auf 65°C erhitzt, dreimal mit Phenol extrahiert und zweimal mit Ethanol gefällt. Dieses Verfahren ist gegenüber dem von Maniatis et al. auf Seite 170 beschriebenen leicht verändert.

## D. Anknüpfen synthetischer Linkerfragmente an DNA-Enden

Wenn eine neue Endonukleaseschnittstelle an das Ende eines DNA-Moleküls angefügt werden soll, wird das Molekül gegebenenfalls zuerst mit DNA-Polymerase behandelt, um glatte Enden zu erzeugen, wie in Abschnitt B beschrieben. Etwa 0.1 bis 1.0 µg dieses Fragments wird zu etwa 10 ng phosphorylierter Linker-DNA (New England Biolabs) zugegeben, die in einem Volumen von 20 µl bis 30 µl eines vom Hersteller empfohlenen Puffers vorliegt, zusammen mit 2 µl T4 DNA-Ligase (New England Biolabs) und 1 mM ATP. Nach einer Inkubation über Nacht bei 15°C wird die Reaktion durch 10 minütiges Erhitzen auf 65°C beendet. Der Reaktionsansatz wird in einem für die Restriktionsendonuklease, welche die synthetische Linkersequenz schneidet, geeigneten Puffer auf etwa 100 µl verdünnt. Ungefähr 50 bis 200 Einheiten dieser Endonuklease werden zu diesem Ansatz hinzugefügt. Die Mischung wird 2 bis 6 Stunden bei der angemessenen Temperatur inkubiert, dann wird das Fragment einer Agarose-Gelelektrophorese unterworfen und wie in Abschnitt C beschrieben gereinigt. Das resultierende Fragment sollte nun Endigungen aufweisen, die normalerweise durch Schneiden mit der jeweiligen Restriktionsendonuklease erzeugt werden. Diese Enden sind gewöhnlich kohäsiv, so dass das resultierende Fragment nun leicht mit anderen Fragmenten, die die gleichen kohäsiven Enden aufweisen, verknüpft werden kann.

## E. Entfernen von 5′-terminalen Phosphaten von DNA-Fragmenten

Die Rezirkularisation eines Vektors während der Plasmidklonierung kann durch die Behandlung des Vektorplasmids mit Phosphatase vermindert werden (diskutiert auf Seite 13 der Maniatis et al-Referenz). Nach der Verdauung der DNA mit der richtigen Restriktionsendonuklease wird eine Einheit alkalische Phosphatase aus dem Darm von Kälbern zugegeben, die von Boehringer-Mannheim, Indianapolis, IN, bezogen werden kann. Die DNA wird eine Stunde bei 37°C inkubiert und anschliessend zweimal mit Phenol extrahiert und mit Ethanol gefällt.

## F. Verknüpfen der DNA-Fragmente

Wenn Fragmente mit komplementären kohäsiven Enden miteinander verknüpft werden sollen, werden etwa 100 ng von jedem Fragment in einem Reaktionsgemisch von 20 µl bis 40 µl mit etwa 0.2 Einheiten T4 DNA-Ligase (New England Biolabs) in dem vom Hersteller empfohlenen Puffer inkubiert. Die Inkubationszeit beträgt zwischen 1 bis 20 Stunden bei einer Temperatur von 15°C. Sollen DNA-Fragmente mit glatten Enden verknüpft werden, so werden diese in der zuvor angegebenen Weise inkubiert, wobei die Menge an T4 DNA-Ligase in diesem Fall auf 2 bis 4 Einheiten erhöht wird.

## G. Transformation von DNA in *E. coli*

Der *E. coli* Stamm HB101 wird für die meisten Experimente verwendet. Die DNA wird dabei mit dem Kalziumchloridverfahren, das von Maniatis et al, auf den Seiten 250 bis 251, beschrieben wird, in *E. coli* eingeführt.

Transformierte Bakterien sind zu einem selektiven Wachstum auf Medien befähigt, die ein geeignetes Antibiotikum enthalten. Diese Fähigkeit zum selektiven Wachstum macht es möglich, die gewünschten Bakterien von denjenigen Wirtsbakterien zu unterscheiden, die keine transformierende DNA erhalten. Die Bestimmung geeigneter Antibiotika für die Selektion von Wirtsbakterien ist Routine und basiert auf der Kenntnis der Resistenzgene, die sich auf der eingeschleusten DNA befinden, sowie der Sensitivität der Wirtsbakterien gegenüber bestimmten Wirkstoffen. Falls z.B. bekannt ist, dass ein bestimmtes Wirtsbakterium gegenüber dem Antibiotikum Ampicillin sensitiv und auf der eingeschleusten transformierenden DNA ein entsprechendes Resistenzgen gegen Ampicillin vorliegt, dann ist Ampicillin ein geeignetes Antibiotikum für die Selektion der Transformanten.

## H. Screening von *E. coli* auf Plasmide

Nach der Transformation werden die resultierenden Kolonien von *E. coli* mit Hilfe eines schnellen Plasmidisolationsverfahrens auf das Vorhandensein des gewünschten Plasmids geprüft. Zwei gebräuchliche Verfahren werden auf den Seiten 366 bis 369 der Maniatis et al-Referenz beschrieben.

I. Isolierung von Plasmid-DNA in grossem Massstab

Verfahren zur Isolierung von Plasmiden aus *E. coli* in grossem Massstab werden auf den Seiten 88 bis 94 der Maniatis et al-Referenz beschrieben.

J. Klonierung in M13 Phagenvektoren

Für die folgende Beschreibung gilt selbstverständlich, dass für Routineverfahren, wie Schneiden mit Restriktionsendonuklease, Verknüpfen etc., die doppelsträngige replikative Form der Phage M13-Abkömmlinge benutzt wird.

Beispiel 1: Konstruktion des chimären Gens im Plasmid pBR322

Um den CaMV GenVI-Promotor und die Protoxin-kodierende Sequenz miteinander zu verknüpfen, wird ein Abkömmling des Phagenvektors mp19 (Yanish-Perron et al., 1985) konstruiert.

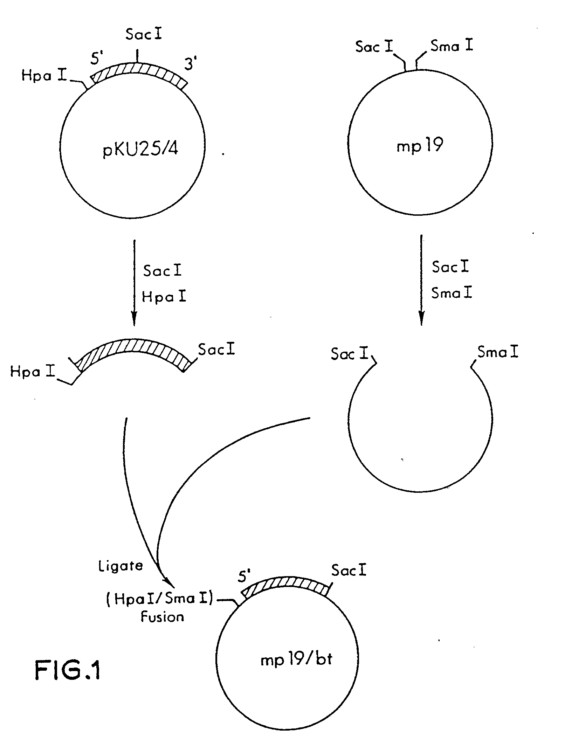

Zuerst werden ein DNA-Fragment mit etwa 155 Nukleotiden 5' zur Protoxin-kodierenden Region und die angrenzenden etwa 1346 Nukleotide der kodierenden Sequenz in den Phagen mp19 eingespleisst. Die mp19 ds rf (doppelsträngige, replikative Form) Phagen-DNA wird mit den Restriktionsendonukleasen Sacl und Smal verdaut und das etwa 7.2 kBp (Kilobasenpaare) umfassende Vektorfragment wird nach der Elektrophorese über eine bei niedrigen Temperaturen gelierende Agarose mit Hilfe von Standardverfahren gereinigt. Das Plasmid pKU25/4, das etwa l0 kBp *Bt*-DNA einschliesslich des Protoxin-Gens enthält, wurde von Dr. J. Nüesch, Ciba-Geigy AG, Basel, Schweiz, erhalten. Die im Plasmid pKU25/4 vorhandene Nukleotidsequenz des Protoxin-Gens ist in Formel I abgebildet. Die pKU25/4 Plasmid-DNA wird mit den Endonukleasen Hpal und Sacl verdaut, und ein 1503 Bp Fragment (mit den Nukleotiden 2 bis 1505 der Formel I) wird wie zuvor beschrieben gereinigt (Dieses Fragment enthält etwa 155 Bp der Bakterien-Promotorsequenzen und etwa 1346 Bp vom Start der Protoxin-kodierenden Sequenz). Etwa 100 ng von jedem Fragment werden unter Zugabe von T4 DNA-Ligase vermischt, und über Nacht bei 15°C inkubiert. Mit dem resultierenden Gemisch wird der *E. coli* Stamm HB101 transformiert, mit den Indikatorbakterien *E. coli* JM101 vermischt und, wie bei Messing (1983) beschrieben, ausplattiert. Ein als mp19/bt bezeichneter Phage wird für die weitere Konstruktion verwendet. (vgl. Fig. 1)

Als nächstes wird ein DNA-Fragment mit dem CaMV GenVI-Promotor und einem Teil der das CaMV-GenVI kodierenden Sequenzen in den Phagen mp19/bt eingefügt. Die mp19/bt ds rf Phagen-DNA wird mit BamHI verdaut, zur Herstellung glatter Enden mit dem grossen Fragment der DNA-Polymerase behandelt, und anschliessend erneut mit der Endonuklease Pstl geschnitten. Das grössere Vektorfragment wird wie oben beschrieben durch Elektrophorese gereinigt. Die Plasmid pABD1-DNA (Paszkowski et al., 1984) wird mit Pstl und HindIII verdaut. Das etwa 465 Bp lange Fragment, das den CaMV GenVI-Promotor und etwa 75 Bp der GenVI kodierenden Sequenz enthält, wird gereinigt. Die zwei Fragmente werden verknüpft und, wie zuvor beschrieben ausplattiert. Einer der resultierenden rekombinanten Phagen mit der Bezeichnung mp19/btca wird im folgenden Experiment verwendet.

Der Phage mp19/btca enthält CaMV GenVI-Promotorsequenzen, einen Teil der kodierenden Sequenz von GenVI, etwa 155 Bp der *Bt*-DNA, stromaufwärts der Protoxin kodierenden Sequenz und etwa 1346 Bp der Protoxin-kodierenden Sequenz. Um die CaMV Promotorsequenzen präzise mit den kodierenden Sequenzen für das Protoxin zu verbinden, wird die dazwischenliegende DNA durch Oligonukleotid-vermittelte Mutagenese der mp19/btca-DNA deletiert. Ein DNA Oligonukleotid mit der Sequenz
(5') TTCGGATTGTTATCCATGGTTGGAGGTCTGA (3') wird mit Hilfe von Routine verfahren unter Verwendung einer DNA-Synthese Apparatur ('Applied Biosystems DNA Synthesizer') synthetisiert. Dieses Oligonukleotid ist den Sequenzen der mp19/btcaPhagen-DNA am 3'-Ende des CaMV Promotors (Nukleotide 5762 bis 5778, Hohn et al., 1982) und dem Anfang der kodierenden Sequenz (Nukleotide 156 bis 172 in Formel I) komplementär.

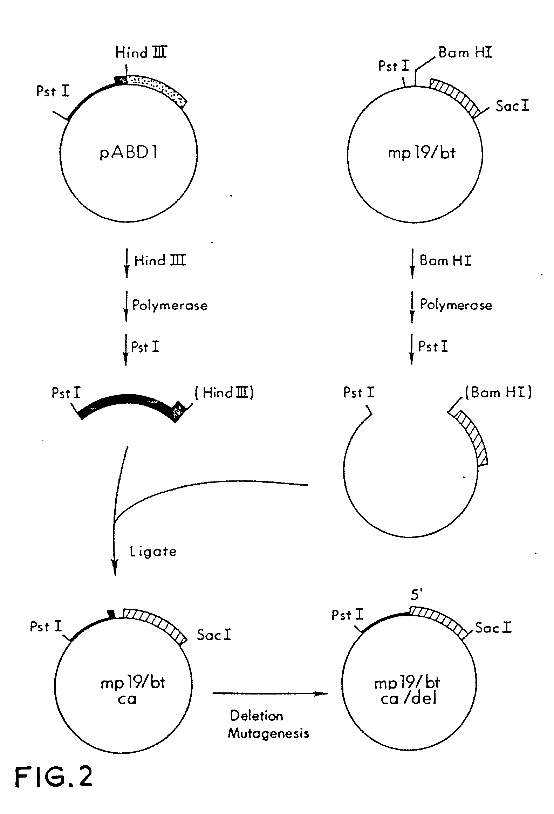

Das allgemeine Verfahren für die Mutagenese wird von Zoller und Smith (1983) beschrieben. Etwa 5 µg einzelsträngige mp19/btcaPhagen-DNA wird mit 0.3 µg des phosphorylierten Oligonukleotids in einem Volumen von 40 µl vermischt. Das Gemisch wird 5 Minuten auf 65°C erhitzt, zunächst auf 50°C und anschliessend langsam weiter auf 4°C weiter abgekühlt. Als nächstes werden Puffer, Nukleotidtriphosphate, ATP, T4 DNA-Ligase und das grosse Fragment der DNA-Polymerase hinzugefügt und über Nacht bei 15°C, wie bei Zoller und Smith (1983) beschrieben, inkubiert. Nach der Gelelektrophorese in Agarose wird zirkuläre doppelsträngige DNA gereinigt und mittels Transfektion in den *E coli* Stamm JM101 überführt. Die resultierenden Plaques werden auf das Vorhandensein von Sequenzen gescreent, die mit dem $^{32}$P-markierten Oligonukleotid hybridisieren, und die Phagen werden mit Hilfe einer DNA-Restriktionsendonukleaseanalyse untersucht. Unter den resultierenden Phagenklonen befinden sich solche, bei denen die unerwünschten Sequenzen zwischen dem CaMV GenVI-Promotor und der kodierenden Sequenz für das Protoxin korrekt deletiert sind. Dieser Phage wird als mp19/btca/del bezeichnet (Fig. 2).

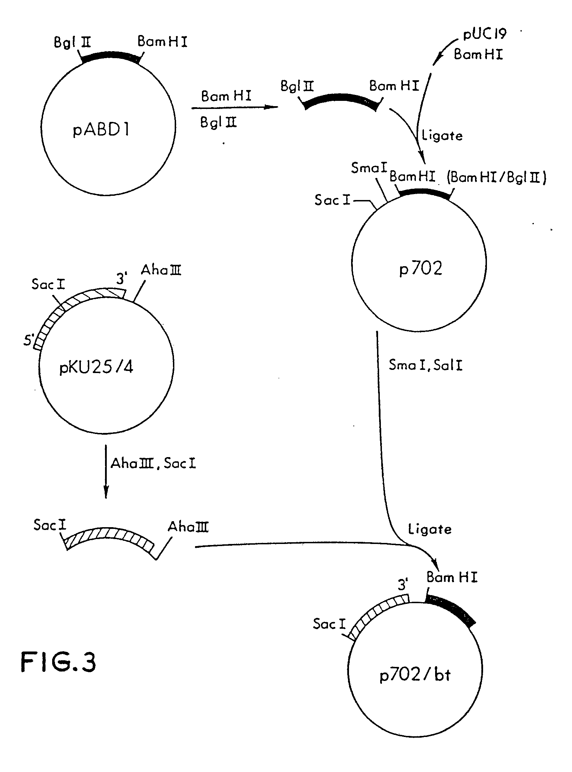

Als nächstes wird ein Plasmid konstruiert, in welchem die 3'-kodierende Region des Protoxin-Gens mit CaMV Transkriptionsterminationssignalen verbunden ist. Die im folgenden einzeln aufgeführten Verfahrensschritte sind in Fig. 3 wiedergegeben.

Zuerst wird die pABD1 Plasmid-DNA mit den Endonukleasen BamHI und BglII verdaut. Ein 0.5 kBp umfassendes Fragment mit den CaMV Transkriptionsterminationssequenzen wird isoliert. Anschliessend wird das Plasmid pUC19 (Yanish-Perron et al., 1985) mit BamHI verdaut, mit dem 0.5 kBp Fragment vermischt und mit T4 DNA-Ligase inkubiert. Nach der Transformation der DNA in den *E coli* Stamm HB101 besitzt einer der resultierenden Klone, mit der Bezeichnung p702 die in Fig. 3 gezeigte Struktur.

Als nächstes wird die p702 Plasmid-DNA mit den Endonukleasen Sacl und Smal geschnitten und das grössere, etwa 3.2 kBp umfassende Fragment durch Gelelektrophorese isoliert. Die pKU25/4 Plasmid-DNA wird mit den Endonukleasen Ahalll und Sacl verdaut und das 2.3 kBp Fragment (Nukleotide 1502 bis 3773 in Formel I) mit dem 3'-Teil der kodierenden Sequenz für das Protoxin (Nukleotide 1504 bis 3773 der in Formel I gezeigten Sequenz) nach Gelelektrophorese isoliert. Diese zwei DNA-Fragmente werden gemischt, mit T4 DNA-Ligase inkubiert und in den *E. coli* Stamm HB101 transformiert. Das resultierende Plasmid ist p702/bt (Fig. 3).

Zur Herstellung eines Plasmids, das die komplette kodierende Sequenz des Protoxins, flankiert von CaMV Promotor- und Terminatorsequenzen, enthält, werden schliesslich Teile der mp19/btca/del ds rf Phagen-DNA und des Plasmids p702/bt miteinander verknüpft.

Die mp19/btca/del Phagen-DNA wird mit den Endonukleasen Sacl und Sphl verdaut und ein Fragment von etwa 1.75 kBp wird nach Durchführung einer Agarose-Gelelektrophorese gereinigt. Auf ähnliche Weise wird die p702/bt Plasmid-DNA mit den Endonukleasen Sacl und Sall verdaut und ein Fragment von etwa 2.5 kBp isoliert. Schliesslich wird die pBR322 Plasmid-DNA (Bolivar et al., 1977) mit Sall und Sphl verdaut und das grössere 4,2 kBp Fragment isoliert. Alle drei DNA-Fragmente werden gemischt, mit T4 DNA-Ligase inkubiert und damit der *E coli* Stamm HB101 transformiert. Das resultierende Plasmid mit der Bezeichnung pBR322/btl4 ist ein Abkömmling von pBR322, das die CaMV GenVI-Promotor- und Translationsstartsignale enthält, verbunden mit der kodierenden Sequenz für das kristalline Protein von *Bt*, gefolgt von CaMV Transkriptionsterminationssignalen (Fig. 4).

Beispiel 2: Konstruktion eines vom Ti-Plasmid abgeleiteten Vektors

Beim Vektor pCIB10 (Rothstein et al, 1987) handelt es sich um einen vom Ti-Plasmid abgeleiteten Vektor, der für den Transfer chimärer Gene auf Pflanzen via *Agrobacterium tumefaciens* verwendet werden kann.

Der Vektor leitet sich von dem Plasmid pKR252 ab, das einen weiten Wirtsbereich aufweist und das von Dr. W. Barnes, Washington University, St. Louis, Mo. bezogen werden kann. Der Vektor enthält weiterhin ein Gen, welches eine Kanamycinresistenz in *Agrobacterium* vermittelt und aus dem Transposon Tn903 (Oka et al, 1981) stammt, sowie linke und rechte T-DNA Grenzsequenzen aus dem Ti-Plasmid pTiT37. Zwischen den Grenzsequenzen befinden sich eine Polylinkerregion aus dem Plasmid pUC18 und ein chimäres Gen, welches eine Kanamycinresistenz in Pflanzen hervorruft.

In einem ersten Verfahrensschritt wird das Plasmid pKR252 in der Weise modifiziert, dass das Tetracyclinresistenzgen gegen das Kanamycinresistenzgen aus dem Transposon Tn903 ausgetauscht wird.

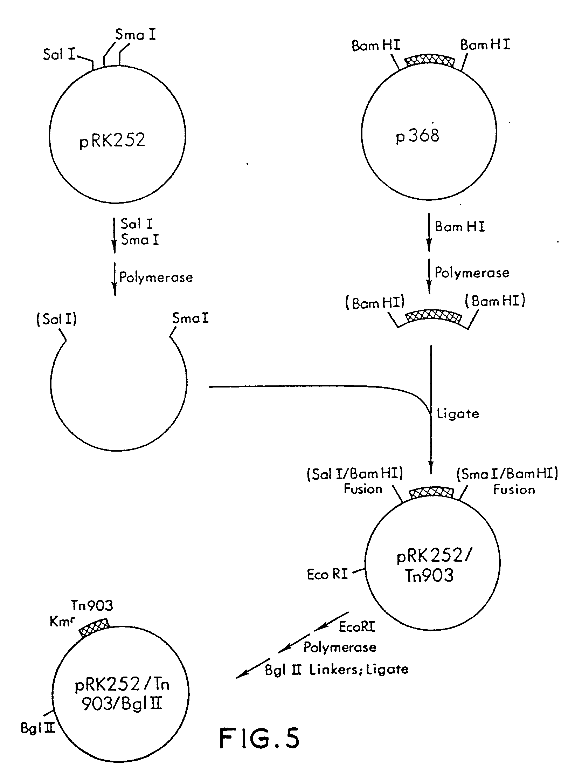

Eine weitere Modifikation betrifft den Austausch der einzigen EcoRI Schnittstelle in pKR252 gegen eine BglII Schnittstelle (siehe Fig. 5, die einen zusammenfassenden Ueberblick über die oben angegebenen Modifikationen gibt).

Das Plasmid pKR252 wird zunächst mit den Endonucleasen Sall und Smal verdaut und anschliessend mit der grossen Untereinheit der DNA Polymerase I behandelt, zur Herstellung glatter Enden. Das grosse Vektorfragment wird über eine Agarose-Gelelektrophorese gereinigt.

Als nächstes wird das Plasmid p368, das auf einem ca. 1050 Bp umfassenden BamHI Fragment Tn903 enthält, mit der Endonuklease BamHI verdaut und mit dem grossen Fragment der DNA Polymerase behandelt. Das etwa 1050 Bp umfassende Fragment wird dann nach Durchführung einer Agarose-Gelelektrophorese isoliert. Dieses Fragment enthält das Gen aus dem Transposon Tn903, das eine Resistenz gegenüber dem Antibiotikum Kanamycin vermittelt (Oka et al, 1981). Zur Erzeugung glatter Enden werden beide Fragmente mit der grossen Untereinheit der DNA-Polymerase behandelt. Anschliessend werden beide Fragmente vermischt und über Nacht bei einer Temperatur von 15°C mit T4 DNA-Ligase inkubiert. Nach der Transformation in den *E. coli*-stamm HB101 und einer Selektion Kanamycin-resistenter Kolonien erhält man das Plasmid pKR252/Tn903 (Fig. 5).

Das so erhaltene Plasmid pKR252/Tn903 wird an seiner einzigen EcoRI Schnittstelle geschnitten und anschliessend zur Herstellung glatter Enden mit der grossen Untereinheit der *E. coli* DNA-Polymerase behandelt. Dieses Fragment wird mit synthetischen Linkern, die BglII Restriktionsschnittstellen enthalten, vermischt und über Nacht mit T4 DNA-Ligase inkubiert. Die aus dieser Behandlung resultierende DNA wird mit einem Ueberschuss an BglII Restriktionsendonuklease verdaut und das grössere Vektorfragment mit Hilfe einer Agarose-Gelelektrophorese gereinigt. Das resultierende Fragment wird erneut mit T4 DNA Ligase inkubiert, um das Fragment über seine neu hinzugefügten kohäsiven BglII Enden zu rezirkularisieren.

Nach Transformation in den *E. coli*-Stamm HB101 erhält man das Plasmid pKR252/Tn903/BglII (Fig. 5).

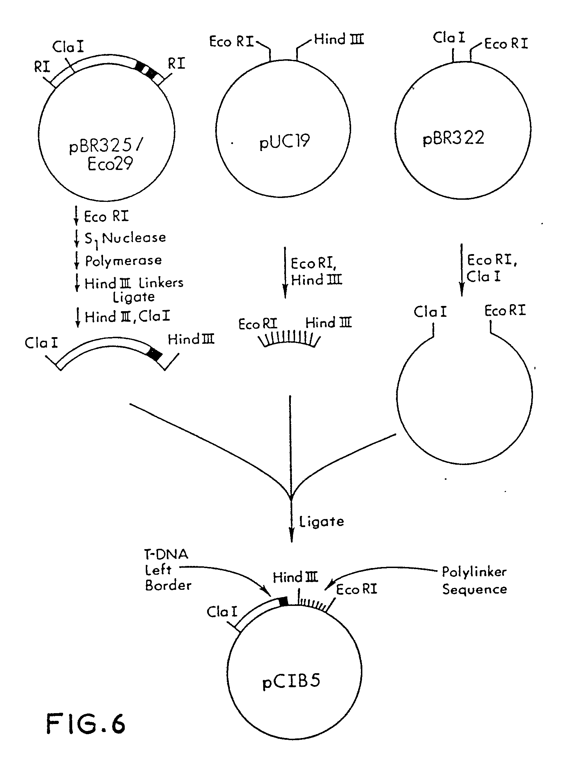

In einem weiteren Verfahrensschritt wird ein Derivat des Plasmids pBR322 konstruiert, das neben den T-DNA Grenzsequenzen aus dem Ti-Plasmid und der Polylinkerregion aus dem Plasmid pUC19 ein selektierbares Gen für Kanamycinresistenz in Pflanzen enthält (Fig. 6).

Das Plasmid pBR325/Eco29 enthält das 1.5 kBp umfassende EcoRI Fragment aus dem Nopalin Ti-Plasmid pTiT37. Dieses Fragment enthält die linken T-DNA Grenzsequenzen (Yadav et al, 1982). Für den Austausch der EcoRI Enden dieses Fragments mit HindIII Enden wird das Plasmid pBR325/Eco29 mit EcoRI verdaut und anschliessend mit Nuclease S1 inkubiert. Es folgt eine Inkubation mit dem grossen Fragment der DNA-Polymerase zur Herstellung glatter Enden. Dieser Ansatz wird dann mit synthetischen HindIII-Linkern vermischt und mit T4 DNA-Ligase inkubiert.

Die resultierende DNA wird mit den Endonukleasen Clal und einem Ueberschuss an HindIII verdaut; das

27

resultierende 1.1 kBp umfassende Fragment, das die linke T-DNA Grenzsequenz enthält, wird mit Hilfe einer Gelelektrophorese gereinigt. Als nächstes wird die Polylinkerregion des Plasmids pUC19 isoliert, indem man die Plasmid DNA mit den Endonukleasen EcoRI und HindIII verdaut und das kleinere Fragment (annähernd 53 Bp) über eine Agarose-Gelelektrophorese isoliert. Anschliessend wird das Plasmid pBR322 mit den Endonukleasen EcoRI und ClaI verdaut, mit den beiden anderen, zuvor isolierten Fragmenten vermischt, mit T4 DNA-Ligase inkubiert und damit der E. coli Stamm HB101 transformiert. Das resultierende Plasmid pCIB5, enthält die Polylinkerregion und die linke T-DNA Grenzsequenz integriert in einen Abkömmling des Plasmids pBR322 (Fig. 6).

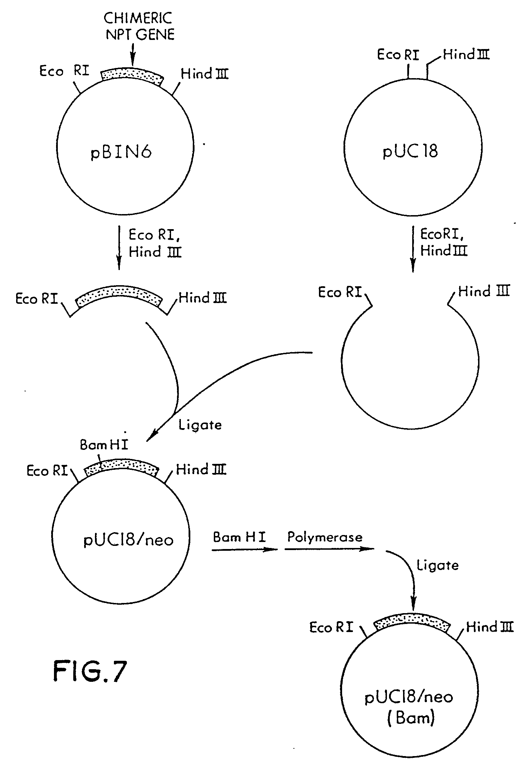

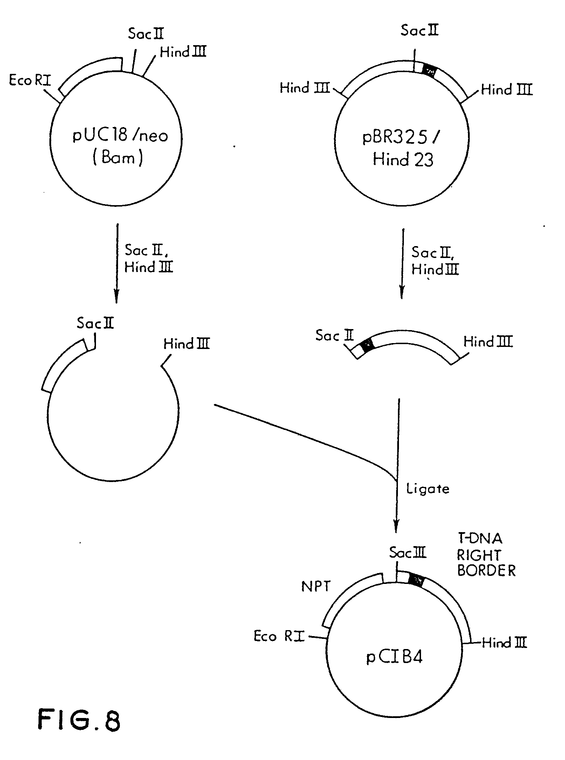

In einem weiteren Verfahrensschritt wird ein Plasmid konstruiert, das ein Gen enthält, welches die Expression einer Kanamycinresistenz in Pflanzen vermittelt (Fig. 7 und 8). Das Plasmid pBIN6 ist erhältlich bei Dr. M. Bevan, Plant Breeding Institute, Cambridge, UK. Dieses Plasmid wird ausserdem bei Bevan (1984) beschrieben.

Das Plasmid pBIN6 wird mit EcoRI und HindIII verdaut. Das etwa 1.5 kBp umfassende Fragment, welches das chimäre Neomycinphosphotransferase (NPT) Gen enthält, wird isoliert und anschliessend über eine Agarosegelelektrophorese gereinigt. Dieses Fragment wird dann mit pUC18 Plasmid DNA vermischt, die zuvor mit den Endonukleasen EcoRI und HindIII geschnitten wurde.

Nach Inkubation mit T4 DNA-Ligase wird mit der resultierenden DNA der E. coli Stamm HB101 transformiert. Das entstehende Plasmid wird als pUC18/neo bezeichnet. Dieses Plasmid enthält eine unerwünschte BamHI Schnittstelle zwischen dem Neomycinphosphotransferase Gen und der Terminator Sequenz des Nopalinsynthase Gens (siehe Bevan, 1984). Um diese Erkennungssequenz zu entfernen, wird das Plasmid pUC18/neo mit der Endonuklease BamHI verdaut, gefolgt von einer Behandlung mit der grossen Untereinheit der DNA-Polymerase zur Erzeugung glatter Enden. Um das Fragment zu rezirkularisieren, wird es anschliessend mit T4 DNA-Ligase inkubiert und damit der E. coli Stamm HB101 transformiert. Das resultierende Plasmid, pUC18/neo(Bam) besitzt keine BamHI Erkennungssequenz mehr.

In einem weiteren Verfahrensschritt wird die rechte T-DNA Grenzsequenz unmittelbar neben das chimäre NPT Gen inseriert (Fig. 8). Das Plasmid pBR325/Hind23 enthält das 3.4 kBp HindIII Fragment des Plasmids pTiT37. Dieses Fragment besitzt die rechte T-DNA Grenzsequenz (Bevan et al., 1983).

Das Plasmid pBR325/Hind23 wird mit den Endonukleasen SacII und HindIII geschnitten und ein 1.0 kBp umfassendes Fragment, welches die rechte Grenzsequenz enthält, im Anschluss an eine Agarosegelelektrophorese in gereinigter Form isoliert.

Das Plasmid pUC18/neo(Bam) wird mit den Endonukleasen SacII und HindIII verdaut und das 4.0 kBp umfassende Fragment mit Hilfe einer Agarosegelelektorphorese isoliert. Die beiden Fragmente werden miteinander vermischt, mit T4 DNA Ligase inkubiert und damit der E. coli Stamm HB101 transformiert. Das resultierende Plasmid, pCIB4 (Fig. 8) enthält die rechte T-DNA Grenzsequenz sowie einen in Pflanzen selektierbaren Marker für Kanamycinresistenz in einem Abkömmling des Plasmids pUC18.

In einem letzten Verfahrensschritt wird ein Plasmid konstruiert, das sowohl die linke als auch die rechte T-DNA Grenzsequenz und zwischen diesen Grenzsequenzen das in Pflanzen selektierbare Kanamycinresistenzgen und den Polylinker des Plasmids pUC18 enthält.

Zunächst wird das Plasmid pCIB4 mit der Endonuklease HindIII verdaut, gefolgt von einer Behandlung mit der grossen Untereinheit der DNA Polymerase zur Herstellung glatter Enden sowie einer Verdauung mit der Endonuklease EcoRI. Das 2.6 kBp umfassende Fragment, welches das chimäre Kanamycinresistenzgen und die rechte T-DNA Grenzsequenz enthält, wird mit Hilfe einer Agarosegelelektrophorese isoliert.

Anschliessend wird das Plasmid pCIB5 mit der Endonuklease AatII verdaut, zur Erzeugung glatter Enden mit T4 DNA-Polymerase behandelt und dann mit der Endonuklease EcoRI geschnitten. Das grössere Vektorfragment wird mit Hilfe einer Agarosegelelektrophorese gereinigt, mit dem pCIB4-Fragment vermischt, mit T4 DNA-Ligase inkubiert und damit der E. coli Stamm HB101 transformiert.

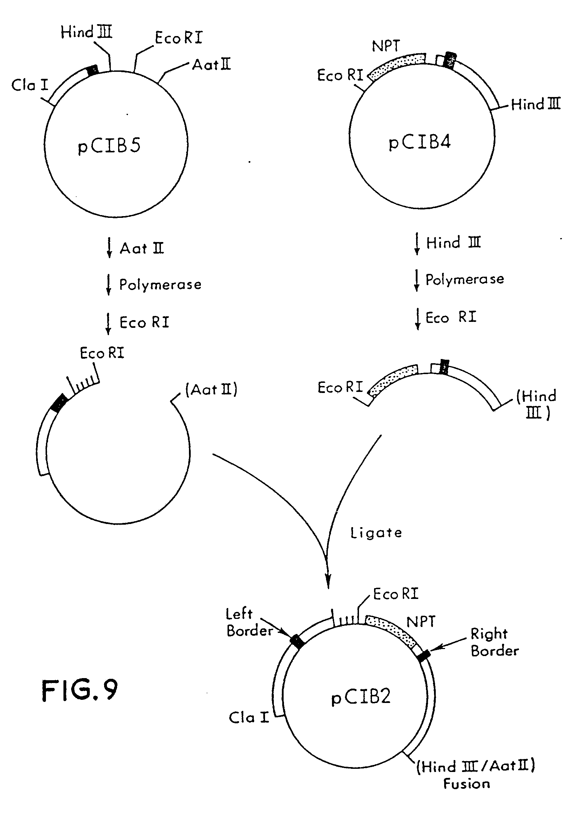

Das resultierende Plasmid pCIB2 (Fig. 9) ist ein Derivat des Plasmids pBR322, das die gewünschten Sequenzen zwischen den beiden T-DNA Grenzsequenzen enthält.

Die folgenden Schritte komplettieren die Konstruktion des Vektors pCIB10. Sie sind in Fig. 10 dargestellt. Das Plasmid pCIB2 wird mit der Endonuklease EcoRV verdaut und, wie zuvor beschrieben, mit synthetischen Linkern, die eine BglII Erkennungsstelle besitzen, versehen. Nach einer Verdauung mit einem Ueberschuss an BglII, wird das annähernd 2.6 kBp umfassende Fragment mit Hilfe einer Agarosegelelektrophorese isoliert. Das zuvor bereits beschriebene Plasmid pK252/Tn903/BglII (Fig. 5), wird mit der Endonuklease BglII verdaut und anschliessend mit Phosphatase behandelt, um eine Rezirkularisierung zu verhindern. Diese beiden DNA Fragmente werden dann miteinander vermischt, mit T4 DNA-Ligase inkubiert und anschliessend in den E. coli Stamm HB101 transformiert. Das resultierende Plasmid ist der vervollständigte Vektor pCIB10.

Beispiel 3: Einbau des chimären Protoxingens in den Vektor pCIB10

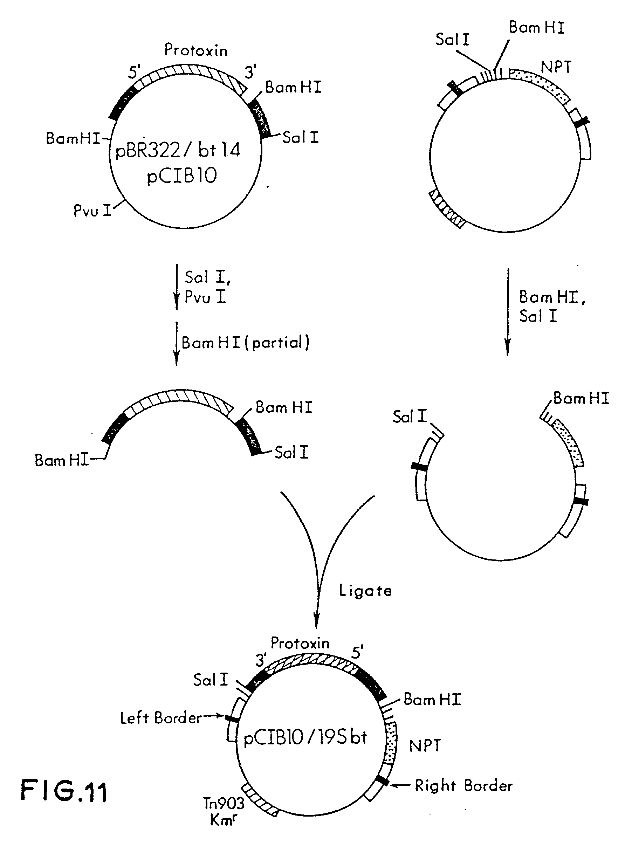

Die im Folgenden beschriebenen Verfahrensschritte sind in Fig. 11 wiedergegeben.

Das Plasmid pBR322/btI4 wird mit den Endonukleasen PvuI und SalI geschnitten und anschliessend mit der Endonuklease BamHI partiell verdaut.

Ein ca. 4.2 kBp umfassendes BamHI-SalI Fragment, welches das chimäre Gen enthält, wird mit Hilfe der Agarosegelelektrophorese isoliert und mit dem durch die Endonukleasen BamHI und SalI verdauten Plasmid pCIB10 vermischt. Nach einer Inkubation mit T4 DNA-Ligase und Transformation des E. coli Stamms HB101, erhält man das Plasmid pCIB10/19Sbt (Fig. 11). Dieses Plasmid enthält das chimäre Protoxingen im

28

Plasmid-Vektor pCIB10.

Für den Transfer des Plasmids pCIB10/19Sbt von *E. coli* HB101 auf *Agrobacterium* wird ein intermediärer *E. coli*-Wirt verwendet und zwar der *E. coli* Stamm S17-1 (Simon et al, 1983). Dieser *E. coli* Stamm, der von Agrigenetics Research Co., Boulder, Co., bezogen werden kann, enthält Mobilisierungsfunktionen, die einen direkten Transfer des Plasmids pCIB10/19Sbt auf *Agrobacterium* via einer Konjugation erlauben. Damit kann die Notwendigkeit eines direkten Transfers nackter Plasmid DNA in *Agrobacterium* umgangen werden.

Zunächst wird pCIB10/19Sbt Plasmid DNA in Calciumchlorid behandelte S17-1 Zellen eingeschleust. Danach werden Kulturen transformierter S17-1 Zellen mit *Agrobacterium tumefaciens* LBA4404 (Ooms et al, 1982) vermischt und auf N Agar (Difco) Platten über Nacht bei Raumtemperatur gepaart.

Von den resultierenden Bakterien wird eine Probe entnommen und auf AB Minimalmedium, das 50 µg/ml Kanamycin enthält, überimpft (Chilton et al, 1974) und ausplattiert. Die Inkubation erfolgt bei 28°C. Die gewachsenen Kolonien werden auf demselben Medium ein zweites Mal ausgestrichen und dann auf N Agarplatten überimpft und ausplattiert. Langsam wachsende Kolonien werden herausgegriffen und auf einem AB Minimalmedium mit Kanamycin ausgestrichen und einzelne Kolonien isoliert. Nach diesem Verfahren werden Agrobakterien isoliert, die das Plasmid pCIB10/Sbt enthalten.

### Beispiel 4: Transfer des chimären Gens auf Tabak-Zellen

Protoplasten von *Nicotiana tabacum* cv. "Çoker 176" werden wie folgt hergestellt:
Vier bis fünf Wochen alte Sprosskulturen werden unter aseptischen Bedingungen in einem MS Medium (Murashige und Skoog, 1962) ohne Hormone bei einer Temperatur von 26°C und einer Photoperiode von 16 Stunden Licht/ 8 Stunden Dunkelheit, herangezogen. Ca. 1.5 g Blattgewebe werden von der Pflanze entnommen und gleichmässig auf 8 bis 10 Petrischalen (100 x 25 mm, Lab-Tek), die jeweils 10 ml einer Enzymlösung enthalten, verteilt. Die Enzymlösung enthält 1 % Cellulase R-10, (Yakult Pharmaceuticals Co.), 0,25 Macerase (Calbiochem Ca.), 1 % Pectolyase Y-23 (Seishin Pharmaceuticals Co.), 0,45 M Mannit und 0,1xK3 Salze (Nagy und Maliga, 1976).

Die Tabakblätter werden mit Hilfe eines Skalpells in dünne Streifen geschnitten. Die Petrischalen werden anschliessend verschlossen und auf einer Rundschüttelmaschine bei einer Umdrehungsgeschwindigkeit von 35 Upm und Raumtemperatur für einen Zeitraum von 4 bis 5 Stunden mit den Enzymen inkubiert.

Anschliessend wird der Inhalt der Petrischalen durch einen mit einem feinmaschigen Gewebe (Mull) ausgelegten Trichter filtriert und in einem Auffanggefäss gesammelt. Das Filtrat wird dann in "Babcook" Flaschen pipettiert, die jeweils 35 ml einer Waschlösung enthalten [Die Waschlösung enthält: 0,45 M Saccharose, MES (2-[N-Morpholino]ethansulfonsäure) sowie 0.1 x K3 Salze]. Die Flaschen werden 10 Minuten bei 80 g zentrifugiert, wodurch die Protoplasten an die Oberfläche der Flaschen flottieren. Die Protoplasten werden mit Hilfe einer 1 ml Pipette entnommen, in einer Flasche gesammelt und zwei weitere Male gewaschen. Die resultierenden Protoplasten werden in K3 Medium in einem 15 ml Einmalzentrifugenröhrchen suspendiert.

Die Konzentration der Protoplasten wird durch Auszählen in einem Fuchs-Rosenthal Hämocytometer bestimmt. Die Protoplasten werden dann in Petrischalen (100 x 20 mm, Corning), die 6 ml eines flüssigen K3 Mediums enthalten, in einer Dichte von 100 000/ml ausplattiert. Die Petrischalen mit den Protoplasten werden anschliessend für zwei Tage bei einer Temperatur von 26°C im Dunklen inkubiert. Während dieser Zeit erfolgt die Regeneration der Zellwand.

Nach Beendigung der zweitägigen Inkubation werden 5 µl einer stationären *A. tumefaciens* Kultur, die das Plasmid pCIB10/19Sbt enthalten, zu den Protoplasten hinzugegeben (Die Agrobakterien werden in einem YEP-Medium, das 50 µg/ml Kanamycin als Zusatz enthält, bei einer Temperatur von 28°C kultiviert bis die stationäre Phase erreicht ist).

Nach einer Inkubationszeit von drei weiteren Tagen bei 26°C, wird Cefotaxim (Calbiochem Co.) hinzugegeben, bis eine Endkonzentration von 500 µg/ml erreicht ist, um die Agrobakterien abzutöten.

Am darauffolgenden Tag werden die Zellen mit 3 ml frischem K3 Medium pro Petrischale verdünnt. Anschliessend wird erneut Cefotaxim bis zu einer Endkonzentration von 500 µg/ml zugegeben. Die Zellen werden dann bei einer Temperatur von 26°C für 3 bis 4 Wochen kultiviert und anschliessend auf Selektivmedien gescreent, wie bei DeBlock et al (1984) beschrieben.

### Beispiel 5: Konstruktion eines chimären Gens für das *Bt* Protoxin mit dem CaMV 35S Promotor

### 5.1 Konstruktion einer CaMV 35S Promotor-Kassette

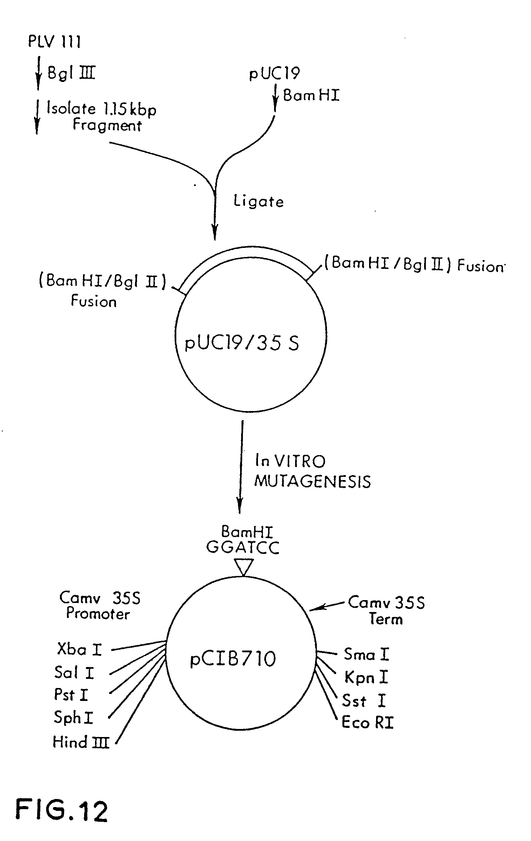

Das Plasmid pCIB710 wird wie in Fig. 12 gezeigt konstruiert. Dieses Plasmid enthält CaMV Promotor- und Transkriptionsterminationssequenzen für das 35S RNA-Transkript (Covey et al., 1981). Ein 1149 Bp BglII-Restriktionsfragment der CaMV-DNA (Bp 6494 bis 7643; Hohn et al., 1982) wird von dem Plasmid pLV111 (erhalten von Dr. S. Howell, Univ. Calif., San Diego) isoliert; alternativ dazu kann das Fragment durch präparative Agarose-Gelelektrophorese, wie zuvor beschrieben, direkt isoliert, mit BamHI-geschnittener pUC19 Plasmid-DNA vermischt, mit T4 DNA-Ligase behandelt und damit *E. coli* transformiert werden (Beachte, dass die BamHI-Restriktionsschnittstelle im resultierenden Plasmid durch Verknüpfen der BglII kohäsiven Enden mit den BamHI kohäsiven Enden zerstört wird). Das resultierende Plasmid, genannt pUC19/35S, wird dann in einer Oligonukleotid-vermittelten in vitro Mutagenese dazu verwendet, um die BamHI-Erkennungssequenz GGATCC direkt im Anschluss an das CaMV Nukleotid 7483 der Hohn et al.-Referenz einzufügen. Das resultierende Plasmid pCIB710 enthält die CaMV 35S Promotor- und Transkriptionsterminationsregion, getrennt durch eine BamHI-Restriktionsschnittstelle. In diese BamHI-Schnittstelle eingefügte DNA-Sequenzen werden in Pflanzen durch diese CaMV Transkriptions-Regulationssequenzen exprimiert (Beachte auch, dass pCIB710 keine ATG Translationsinitiationskodons zwischen dem Start der Transkription und der BamHI-Schnittstelle enthält).

### 5.2. Einfügen der CaMV 35S Promotor/Terminatorkassette in pCIB10

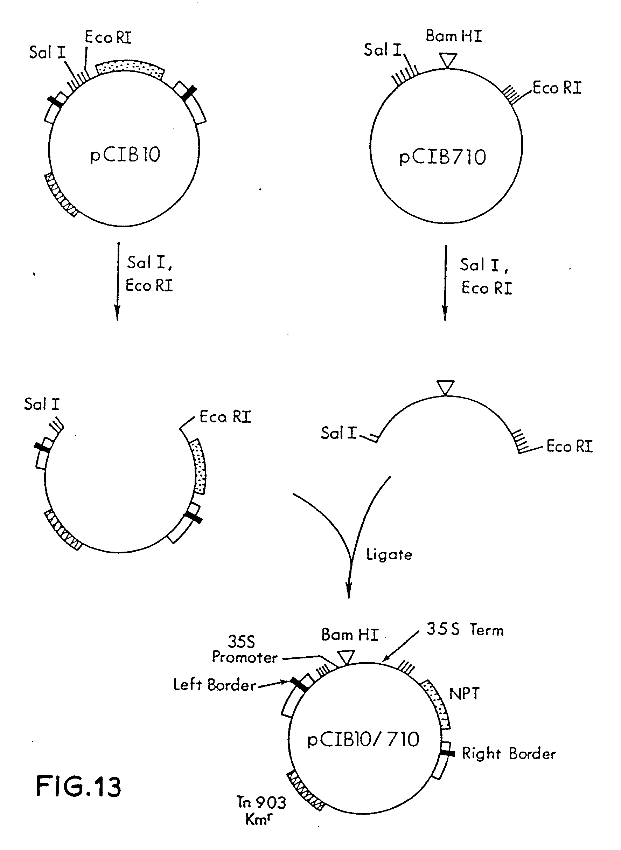

Die folgenden Schritte sind in Fig. 13 dargestellt. Die pCIB10- und pCIB710 Plasmid-DNA wird mit EcoRI und SalI geschnitten, vermischt und verknüpft. Das resultierende Plasmid pCIB10/710 enthält die CaMV 35S Promotor-/Terminatorkassette, eingefügt in den Pflanzentransformationsvektor pCIB10. Die CaMV 35S-Sequenzen befinden sich zwischen den T-DNA-Grenzsequenzen in pCIB10 und werden so bei Pflanzentransformationsexperimenten in das pflanzliche Genom eingefügt.

### 5.3. Einfügen des *Bt* Protoxin Gens in pCIB10/710

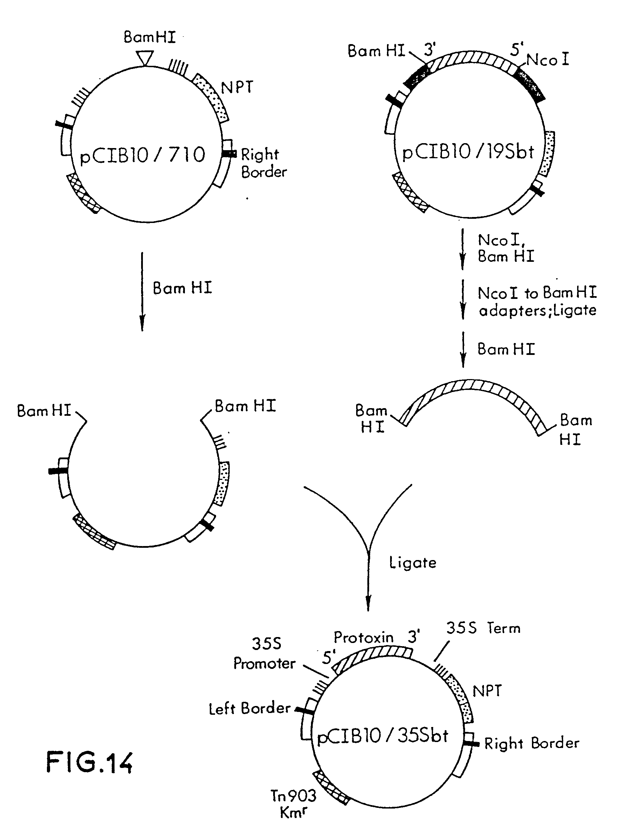

Die folgenden Schritte sind in Fig. 14 dargestellt. Als eine Quelle für das Protoxin-Gen wird das Plasmid pCIB10/19Sbt mit BamHI und Ncol geschnitten und das 3.6 kBp Fragment mit dem Protoxin-Gen durch präparative Gelelektrophorese isoliert. Das Fragment wird dann mit einem synthetischem Ncol-BamHI-Adaptor, der die Sequenz 5'-CATGGCCGGATCCGGC-3' aufweist, vermischt und mit BamHI geschnitten. Dieser Schritt erzeugt BamHI kohäsive Enden an beiden Seiten des Protoxinfragments. Dieses Fragment wird dann in zuvor mit BamHI geschnittenes pCIB10/710 eingefügt. Das in Fig. 14 gezeigte resultierende Plasmid pCIB10/35Sbt enthält das Protoxin-Gen zwischen den CaMV 35S Promotor- und Transkriptionsterminationssequenzen.

### 5.4. Transfer des Plasmids pCIB10/35SBt in *Agrobacterium tumefaciens* für die Pflanzentransformation

Das Plasmid pCIB10/35Sbt wird in den *A. tumefaciens* Stamm LBA4404 transferiert, wie zuvor in Beispiel 4 beschrieben.

### Beispiel 6: Konstruktion des Plasmids pTOX, das ein chimäres Gen enthält, welches das insektizide Toxinprotein von *Bt* var. *tenebrionis* kodiert.

Ein Gen, welches das insektizide Kristallprotein von *Bt* var. *tenebrionis* kodiert, wurde von Sekar et al. (1987) charakterisiert und sequenziert.

Diese kodierende Sequenz wird in Form eines gewöhnlichen Restriktionsfragments isoliert, wie z.B. einem HindIII Fragment von annähernd 3 kBp, und in einem geeigneten Pflanzen-Expressions-Vektor eingespleisst, wie etwa das Plasmid pCIB770 (Rothstein et al., 1987).

Das Plasmid pCIB770 enthält ein chimäres Kanamycingen, das in Pflanzen exprimiert wird, sowie den Promotor und Terminator des 35S RNA Transkripts von CaMV, die durch eine nur einmal im Plasmid vorliegende BamHI Restriktionsschnittstelle voneinander getrennt sind.

Das Restriktionsfragment, das die Toxin-kodierende Sequenz enthält, wird mit dieser einzigen BamHI Restriktionsschnittstelle des Plasmids pCIB770 mit Hilfe eines geeigneten molekularen Adapters kompatibel gemacht. Anschliessend werden die beiden Fragmente miteinander verküpft.

### Beispiel 7: Konstruktion des Plasmids pSAN, das ein chimäres Gen enthält, welches das insektizide Toxinprotein von *Bt* var. *san diego* kodiert

Ein Gen, welches das insektizide Protein von *Bt* var. *san diego* kodiert, wurde von Herrnstadt et al. (EP-202,739 und EP-213,818) charakterisiert und sequenziert.

Diese kodierende Sequenz wird auf einem gewöhnlichen Restriktionsfragment isoliert und in einen geeigneten Pflanzen-Expressions-Vektor, wie das Plasmid pCIB770, eingespleisst.

Das Plasmid pCIB770 enthält ein chimäres Kanamycingen, das in Pflanzen exprimiert wird, sowie den Promotor und Terminator des 35S RNA Transkripts von CaMV, die durch eine nur einmal im Plasmid vorliegende BamHI Restriktionsschnittstelle voneinander getrennt sind.

Das Restriktionsfragment, das die Toxin-kodierende Sequenz enthält, wird mit dieser einzigen BamHI Restriktionsschnittstelle des Plasmids pCIB770 mit Hilfe eines geeigneten molekularen Adapters kompatibel gemacht. Anschliessend werden die beiden Fragmente miteinander verküpft.

### Beispiel 8: Konstruktion eines deletierten *Bt* Protoxin Gens, das ein Polypeptid mit etwa 725 Aminosäuren kodiert, sowie Konstruktion eines chimären Gens, welches das deletierte Gen unter der Kontrolle des CaMV 35S Promotors enthält

Für die Herstellung eines deletierten Protoxin Gens, das ein Polypeptid mit ca. 725 Aminosäuren kodiert, wird der Teil des Gens, der den COOH-terminalen Anteil des Toxins kodiert, durch Schneiden an der Kpnl Restriktionsschnittstelle an Position 2325 der in Formel I wiedergegebenen Sequenz entfernt.

Das Plasmid pCIB10/35Sbt (Fig. 14) wird mit BamHI und Kpnl verdaut, und das etwa 2.2 kBp umfassende BamHI/Kpnl Fragment, das das deletierte Protoxin Gen enthält, wird mit Hilfe einer präparativen Agarosegelelektrophorese isoliert. Um die Kpnl Schnittstelle am 3' Ende in eine BamHI Schnittstelle umzuwandeln, wird das Fragment mit einem Kpnl/BamHI Oligonukleotid-Adapter vermischt und verknüpft. Dieses Fragment wird dann mit dem BamHI-geschnittenen Plasmid pCIB10/710 (Fig. 13) vermischt.

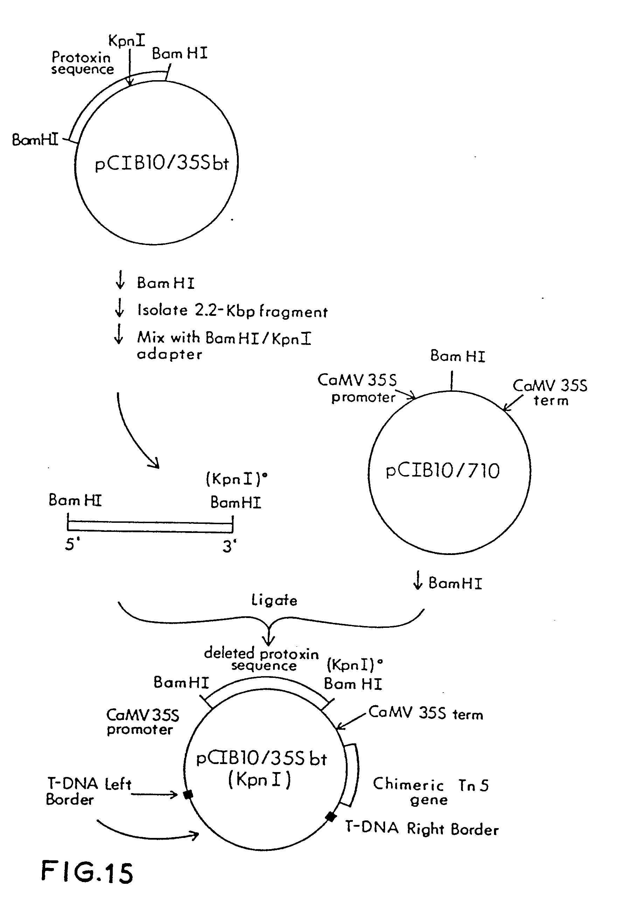

Die resultierenden Transformanten, die mit pCIB10/35Sbt (Kpnl) bezeichnet werden und in Fig. 15 wiedergegeben sind, enthalten das deletierte Protoxin Gen, das ein Polypeptid mit ca. 725 Aminosäuren kodiert. Diese Transformanten werden auf Kanamycin selektioniert.

Beispiel 9: Konstruktion eines deletierten *Bt* Protoxin Gens, das ein Polypeptid mit etwa 645 Aminosäuren kodiert, sowie Konstruktion eines chimären Gens, welches das deletierte Gen unter der Kontrolle des CaMV 35S Promotors enthält

Für die Herstellung eines deletierten Protoxin Gens, das ein Polypeptid mit ca. 645 Aminosäuren kodiert, wird der Teil des Gens, der den COOH-terminalen Anteil des Toxins kodiert, durch Schneiden an der BclI Restriktionsschnittstelle an Position 2090 der in Formel I wiedergegebenen Sequenz entfernt.

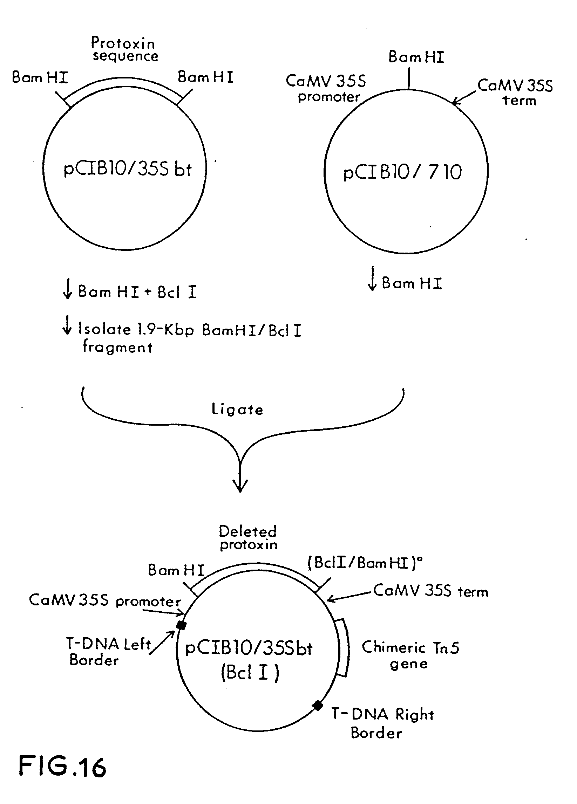

Das Plasmid pCIB10/35Sbt (Fig. 14) wird mit BamHI und BclI verdaut und das ca. 1.9 kBp umfassende Fragment, welche das deletierte Toxingen enthält, wird mit Hilfe einer präparativen Agarosegelelektrophorese isoliert. Da BclI ein kohäsives Ende entstehen lässt, das mit BamHI kompatibel ist, müssen vor dem Einspleissen dieses Fragmentes in das BamHI-geschnittene Plasmid pCIB10/710 (Fig. 13) keine weiteren Manipulationen vorgenommen werden. Das resultierende Plasmid, das die Struktur des in Fig. 16 wiedergegebenen Plasmids pCIB10/35Sbt (BclI) aufweist, wird auf Kanamycin selektioniert.

Beispiel 10: Konstruktion eines deletierten *Bt* Protoxin Gens, das ein Polypeptid mit etwa 607 Aminosäuren kodiert, sowie Konstruktion eines chimären Gens, welches das deletierte Gen unter der Kontrolle des CaMV 35S Promotors enthält

Für die Herstellung eines deletierten Protoxin Gens wird eine BamHI Schnittstelle (GGATCC) unmittelbar im Anschluss an das Nukleotid 1976 der in Formel I wiedergegebenen Sequenz eingefügt.

Dabei wird zunächst das BamHI Fragment, welches die Protoxinsequenz enthält, von pCIB10/35Sbt in mp18 kloniert, unter Verwendung der zuvor im Rahmen der Oligonukleotid Mutagenese beschriebenen Standardverfahren

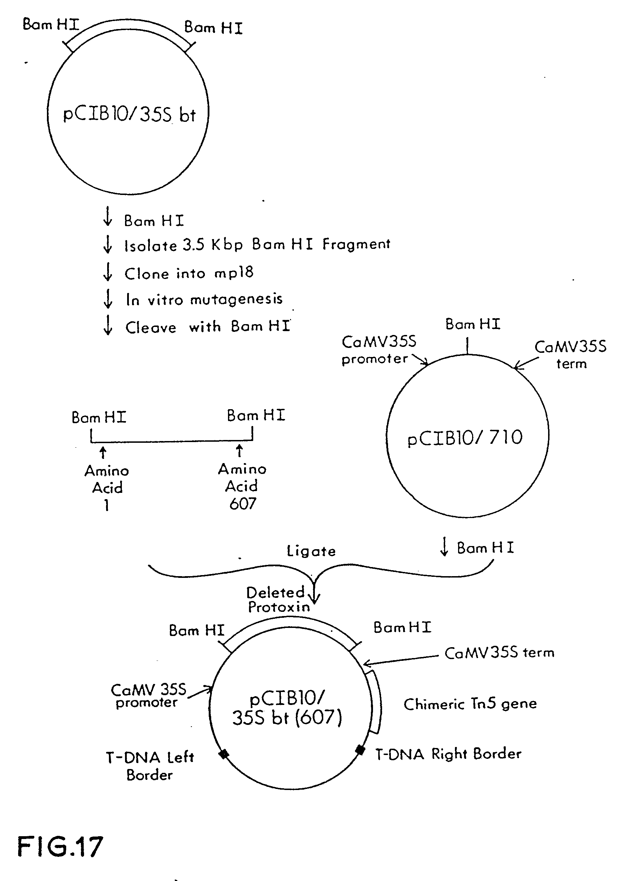

Nach Durchführung der Mutagenese wird doppelsträngige replikative DNA von dem M13 Klon gewonnen, die anschliessend mit BamHI verdaut wird. Das ca. 1.9 kBp umfassende Fragment, welches das deletierte Protoxin Gen enthält, wird dann in das BamHI-geschnittene Plasmid pCIB10/710 eingespleisst. Das resultierende Plasmid, das die Struktur des in Fig. 17 wiedergegebenen Plasmids pCIB10/35Sbt(607) aufweist, wird auf Kanamycin selektioniert.

Die weiteren Beispiele beschreiben spezifische Rezepte für die Transformation von Baumwollzellen sowie die Regeneration von Baumwollpflanzen aus Bauwollzellen und -kallus.

Es versteht sich von selbst, dass jeder Fachmann auf diesem Gebiet in der Lage ist, die nachstehend beschriebenen Rezepte in ihren Details zu verändern, ohne aber dadurch den erfindungsgemässen Umfang zu verlassen. So existieren beispielsweise eine ganze Reihe von bekannten Kulturmedien für pflanzliche Gewebe, von denen einige im Folgenden im Detail beschrieben werden. Ein Durchschnittsfachmann auf dem Gebiet pflanzlicher Gewebekulturen weiss, in welcher Weise er diese Kulturlösungen variieren kann, um die gleichen oder aber ähnlichen Ergebnisse zu erhalten. So offenbart beispielsweise Ausführungsbeispiel 12 ein modifiziertes White's Basis-Medium für die Keimung von Samen und die Kallus-Entwicklung; Beispiel 13 beschreibt ein Murashige und Skoog's Basismedium für Wachstum und Erhaltung von Kallus; Beispiel 14 beschreibt ein Beasley und Ting Basismedium als Pflanzenkeimungsmedium. Der Durchschnittsfachmann auf dem Gebiet der pflanzlichen Gewebekulturen weiss, wie er diese Basismedien variieren muss, um ähnliche Ergebnisse zu erhalten wie die, welche in den Beispielen beschrieben sind. So kann beispielsweise der Zucker im Kallus-Wachstumsmedium Glucose sein, der die phenolischen Sekretionen auf ein Miniumum reduziert, oder aber Saccharose, das die Ausbildung eines embryogenen Kallus fördert.

Die für die Transformation verwendeten Explanate können aus jeder beliebigen geeigneten Quelle stammen, wie z.B. aus Sämlingen, vorzugsweise aus einem Hypocotyl oder einem Keimblatt, oder aber aus einem Embryo einer sich entwickelnden Frucht.

Für die Abtötung der nach der Transformation noch vorhandenen Agrobakterien kann jedes Antibiotikum verwendet werden, das toxisch ist für *Agrobacterium*. Besonders bevorzugt ist Cefatoxim.

Beispiel 11: Regeneration von Baumwollpflanzen

11.1. Medien

Alle Medien enthalten die bei Murashige und Skoog genannten anorganischen Salze und die bei Gamborg genannten B-5-Vitamine; sie sind auf einen pH von 5,7 eingestellt und haben die folgende Zusammensetzung (mg/Liter):

31

|   Makronährstoffe   |        |
| --- | --- |
| MgSO$_4$•7H$_2$O | 370 |
| KH$_2$PO$_4$ | 170 |
| KNO$_3$ | 1900 |
| NH$_4$NO$_3$ | 1650 |
| CaCl$_2$•2H$_2$O | 440 |
|   Mikronährstoffe   |        |
| H$_3$BO$_3$ | 6.2 |
| MnSO$_4$•H$_2$O | 15.6 |
| ZnSO$_4$•7H$_2$O | 8.6 |
| NaMoO$_4$•2H$_2$O | 0.25 |
| CuSO$_4$•5H$_2$O | 0.025 |
| CaCl$_2$•6H$_2$O | 0.025 |
| KI | 0.83 |
| FeSO$_4$•7H$_2$O | 27.8 |
| Na$_2$EDTA | 37.3 |
|   Vitamine   |        |
| Thiamin•HCl | 10 |
| Pyridoxin•HCl | 1 |
| Nicotinsäure | 1 |
| Myo-Inosit | 100 |

Zusätzlich enthalten die verschiedenen Medien die folgenden Komponenten:

| Medium | Zusätzliche Komponenten |
| --- | --- |
| 1 | 20 g/Liter Saccharose, 0,6 % Agar Noble (Difco) |
| 2 | 30 g/Liter Glucose, 2 mg/l α-Naphthalinessigsäure, 1 mg/Liter Kinetin, 0,8 % Agar Noble |
| 3 | 30 g/Liter Saccharose, 2 mg/l α-Naphthalinessigsäure, 1 mg/Liter Kinetin, 0,8 % Agar Noble |
| 4 | 20 g/Liter Saccharose, 0,5 mg/l Picloram |
| 5 | 20 g/Liter Saccharose, 5 mg/l 2,4-Dichlorphenoxyessigsäure |
| 6 | 20 g/Liter Saccharose, 15 mM Glutamin |

Bei Medien, die bei 25°, 28° und 31°C eingesetzt werden, wird zusätzlich zur Temperatur auf eine Photoperiode von 16 Stunden Licht/8 Stunden Dunkelheit, bei einer Lichtintensität von 20 µE m$^{-2}$s$^{-1}$ (= 1670 1x), Bezug genommen.

11 .2: Samensterilisation und Auspflanzen

Samen von Baumwolle (*Gossypium hirsutum* var. Coker 310) werden entfasert, indem der Samen 2 Minuten in konzentrierte H$_2$SO$_4$ gelegt wird. Die Samen werden dann viermal mit sterilem, destilliertem Wasser gewaschen, in 95 % Ethanol getaucht, abgeflammt und auf Medium 1 bei 31°C gepflanzt.

### 11.3: Kallusinduktion

Sieben Tage nach dem Auspflanzen werden die Sämlinghypokotyle herausgeschnitten, längs aufgeschnitten, in 2 mm Abschnitte geschnitten und bei 31°C auf Medium 2 gelegt. Die Hypokotylabschnitte (2 mm) werden wöchentlich auf frisches Medium 2 übertragen, und diese Kulturen werden ebenfalls bei 31°C gehalten. Nach vier wöchentlichen Uebertragungen auf Medium 2 wird Kallusgewebe, das an den Hypokotylabschnitten proliferiert, von dem ursprünglich verpflanzten Gewebe entfernt und bei einer Temperatur von 31°C kultiviert. Der Kallus wird nach einem Monat auf frisches Medium 3 übertragen und für weitere ein bis zwei Monate unterhalten.

### 11.4: Initiierung der Suspensionskultur

Zur Initiierung von Suspensionskulturen werden 100 mg Kallusgewebe in 35 ml Medium 4 in einem 125 ml DeLong Gefäss gegeben. Die Suspensionen werden 6 Wochen mit 140 upm (umdrehungen pro Minute) und 28°C geschüttelt. Während dieser Zeit beginnen sie, rasch zu proliferieren.

### 11.5: Embryoentwicklung und Pflanzenregenerierung

Die sich im Medium 4 entwickelnden Embryonen vermehren sich noch schneller, wenn Medium 4 durch Medium 5 ersetzt wird. Die embryogene Suspension wird geteilt und alle 3 bis 7 Tage in frischem Medium 5 weitere Male kultiviert. Zur Entwicklung der sich in Medium 5 vermehrenden Embryonen werden diese zunächst mit Medium 6 gewaschen und anschliessend in das gleiche Medium übertragen. Drei bis vier Wochen nach dem Transfer in Medium 6 werden die reifen Embryonen auf ein festes Medium bei 25°C gegeben. Das feste Medium besteht aus einem modifizierten MS-Medium, das die MS Salze, 40 mM $KNO_3$ an Stelle von $KNO_3$ und $NH_4NO_3$, B-5-Vitamine, 2 % Saccharose sowie 15 mM Glutamin enthält und das mit 0,2 % Gelrite verfestigt ist (pH 5,7). Die Embryonen werden in Petrischalen bei 25°C gegeben. Die Sprossentwicklung geschieht auf diesem Medium vereinzelt, und das Wurzellängenwachstum wird durch den Transfer der Embryonen auf das obige modifizierte MS-Medium ohne Glutamin verstärkt. Keimende Embryonen werden dann in Blähton in Tontöpfe gepflanzt und mit einem Becher bedeckt (25°C). Nachdem sich Pflänzchen im Blähton entwickelt haben, wird der Becher entfernt. Nach einer Woche bei 28°C werden die Pflänzchen in das Treibhaus in Erde gebracht, damit sie sich weiter zu ausgewachsenen Pflanzen entwickeln.

### Beispiel 12: Medien für die Samenkeimung und Kallusentwicklung

[Zusammensetzung der modifizierten Stammlösung nach White (1961)]

| Bestandteil | Konzentration pro 1000 ml. | Anmerkungen |
|---|---|---|
| $MgSO_4 \bullet 7\ H_2O$<br>$Na_2SO_4$<br>$NaH_2PO_4 \bullet H_2O$ | 3,6 g<br>2,0 g<br>1,65 g | Lösen der Bestandteile und Auffüllen auf ein Endvolumen von 1000 ml. Bezeichnung: <u>Stammlösung White's A.</u> Verwendung von 100 ml/Liter des fertigen Mediums. |
| $Ca(NO_3)_2 \bullet 4\ H_2O$<br>$KNO_3$<br>KCl | 2,6 g<br>800 mg<br>650 mg | Lösen der Bestandteile und Auffüllen auf ein Endvolumen von 1000 ml. Bezeichnung: <u>Stammlösung White's B.</u> Verwendung von 100 ml/Liter des fertigen Mediums. |
| $Na_2MoO_4 \bullet 2H_2O$<br>$CoCl_2 \bullet 6H_2O$<br>$MnSO_4 \bullet H_2O$<br>$ZnSO_4 \bullet 7\ H_2O$<br>$CuSO_4 \bullet 5\ H_2O$<br>$H_3BO_3$ | 2,5 mg<br>2,5 mg<br>300 mg<br>50 mg<br>2,5 mg<br>50 mg | Lösen der Bestandteile und Auffüllen auf ein Endvolumen von 1000 ml. Bezeichnung: <u>Stammlösung White's C.</u> Verwendung von 100 ml/Liter des fertigen Mediums. |
| Fe-EDTA | | Verwendung von 10 ml/Liter von MSFe/EDTA (siehe unten) |
| Organische Bestandteile | | Verwendung von 10 ml/Liter von MS organisch (siehe unten) |

### Beispiel 13: Medien für das Wachstum und die Aufrechterhaltung von Kallus

[Zusammensetzung der Murashige und Skoog (1962) (MS) Stammlösung]

Beispiel 13: Medien für das Wachstum und die Aufrechterhaltung von Kallus

[Zusammensetzung der Murashige und Skoog (1962) (MS) Stammlösung]

| Bestandteil | Konzentration pro 1000 ml. | | Anmerkungen |
|---|---|---|---|
| $NH_4NO_3$ | 41,26 | g | Lösen der Bestandteile und Auffüllen auf ein Endvolumen von 1000 ml. Bezeichnung: MS-Major. Verwendung von 40 ml/Liter des fertigen Mediums. |
| $KNO_3$ | 47,50 | g | |
| $CaCl_2 \cdot 2\ H_2O$ | 11,00 | g | |
| $MgSO_4 \cdot 7\ H_2O$ | 9,25 | g | |
| $KH_2PO_4$ | 4,25 | g | |
| KI | 83 | mg | Lösen der Bestandteile und Auffüllen auf ein Endvolumen von 1000 ml. Bezeichnung: MS-Minor. Verwendung von 100 ml/Liter des fertigen Mediums. |
| $H_3BO_3$ | 620 | mg | |
| $MnSO_4 \cdot H_2O$ | 1690 | mg | |
| $ZnSO_4 \cdot 7\ H_2O$ | 860 | mg | |
| $Na_2MoO_4 \cdot 2\ H_2O$ | 25 | mg | |
| $CuSO_4 \cdot H_2O$ | 2,5 | mg | |
| $CoCl_2 \cdot 6\ H_2O$ | 2,5 | mg | |
| Nicotinsäure | 50 | mg | Lösen der Bestandteile und Auffüllen auf ein Endvolumen von 1000 ml. Bezeichnung: MS-organisch. Einfrieren von 10 ml Aliquots. Verwendung von 10 ml/Liter des fertigen Mediums. |
| Pyridoxin HCl | 50 | mg | |
| Thiamin HCl | 10 | mg | |

| Bestandteil | Konzentration pro 1000 ml. | Anmerkungen |
|---|---|---|
| Fe EDTA<br>Fe SO$_4$.7H$_2$O<br>Na$_2$ EDTA.2 H$_2$O | 2,78 g<br>3,73 g | Lösen von 2.78 g FeSO$_4$•7 H$_2$O in ca. 200 ml destilliertem Wasser. Lösen von 3,73 g Na$_2$-EDTA•2H$_2$O (Dinatriumsalz des Ethylendiaminotetraessigsäuredihydrats) in 200 ml destilliertem Wasser in einem separaten Gefäss. Erhitzen der Na$_2$-EDTA Lösung auf einer Wärmplatte für ca. 10 Minuten. Unter ständigem Rühren FeSO$_4$ Lösung zur Na-EDTA Lösung zugeben. Abkühlen der Lösung auf Raumtemperatur und Auffüllen auf ein Endvolumen von 1000 ml. <u>Bezeichnung</u>: <u>MSFe-EDTA</u>. Verschliessen des Aufbewahrungsgefässes mit Folie und Aufbewahren im Kühlschrank. Verwendung von 10 ml/Liter des fertigen Mediums. |
| Thiamin HCl | 50 mg | Lösen des Thiamins und Auffüllen auf ein Volumen von 500 ml. <u>Bezeichnung</u>: <u>MS-Thiamin</u>. Verwendung von 4.0 ml/Liter des fertigen Mediums. |
| m-Inosit<br>Glycin | 10 g<br>0,2 g | Lösen der Bestandteile und Auffüllen auf ein Endvolumen von 1000 ml. Bezeichnung: <u>MS-Glycin/Inosit</u>. Verwendung von 10 ml/Liter des fertigen Mediums. |

Beispiel 14: Pflanzenkeimungsmedien

[Zusammensetzung einer Stammlösung nach Beasley und Ting (1973)]

| Bestandteil | Konzentration pro 1000 ml. | Anmerkungen |
|---|---|---|
| KH$_2$PO$_4$<br>H$_3$BO$_3$<br>Na$_2$MoO$_4$•2 H$_2$O | 2,72 g<br>61,83 mg<br>2,42 mg | Lösen der Bestandteile und Auffüllen auf ein Volumen von 100 ml. Bezeichnung: B&T-Stammlösung A. Verwendung von 10 ml/Liter des fertigen Mediums. |
| CaCl$_2$•2 H$_2$O<br>KI<br>CoCl$_2$•6 H$_2$O | 2,6 g<br>8,3 mg<br>0,24 mg | Lösen der Bestandteile und Auffüllen auf ein Volumen von 100 ml. Bezeichnung: B&T-Stammlösung B. Verwendung von 10 ml/Liter des fertigen Mediums. |
| MgSO$_4$•7 H$_2$O<br>MnSO$_4$•H$_2$O<br>ZnSO$_4$•7 H$_2$O<br>CuSO$_4$•5 H$_2$O | 4,93 g<br>169,02 mg<br>86,27 mg<br>0,25 mg | Lösen der Bestandteile und Auffüllen auf ein Volumen von 100 ml. Bezeichnung: B&T-Stammlösung C. Verwendung von 10 ml/Liter des fertigen Mediums. |
| KNO$_3$ | 25,275 g | Lösen der Bestandteile und Auffüllen auf ein Volumen von 200 ml. Bezeichnung: B&T-Stammlösung D. Verwendung von 40 ml/Liter des fertigen Mediums. |
| Nicotinsäure<br>Pyridoxin HCl<br>Thiamin HCl | 4,92 mg<br>8,22 mg<br>13,49 mg | Lösen der Bestandteile und Auffüllen auf ein Endvolumen von 100 ml. Bezeichnung: B&T-organisch. Verwendung von 10 ml/Liter des fertigen Mediums. |
| Fe-EDTA | | Verwendung von 10 ml/Liter von MSFe/EDTA |
| Inosit | | 100 mg/Liter des fertigen Mediums |
| NH$_4$NO$_3$ (15 μM) | | 1200.6 mg/Liter des fertigen Mediums |

Beispiel 15: Regeneration ganzer Pflanzen ausgehend von Keimblatt-Explantaten

Baumwollsamen der Varietät Acala SJ2 von *Gossypium hirsutum* werden drei Minuten mit 95 % Alkohol sterilisiert, zweimal mit sterilem Wasser gewaschen, anschliessend für die Dauer von 15 Minuten in eine 15 %ige Natriumhypochlorit-Lösung eingebracht und erneut in sterilem Wasser gewaschen. Zur Herstellung von Keimlingen werden die so sterilisierten Samen für einen Zeitraum von etwa 14 Tagen im Dunkeln auf einem der gebräuchlichen Agar-Medien zur Keimung gebracht. Die Keimblätter dieser Keimlinge werden in etwa 2 bis 4 mm$^2$ grosse Segmente zerschnitten und anschliessend unter sterilen Bedingungen auf ein Kallus-induzierendes Medium transferiert, das sich aus den Makro- und Mikrosalzen des Murashige und Skoog Medium (MS) zusammensetzt und zusätzlich Thiamin HCl [0,4 mg/Liter], Glucose [30 g/Liter], Naphthyl-1-essigsäure (NAA) 2 mg/Liter], Kinetin [1 mg/Liter], m-Inosit [100 mg/Liter] sowie Agar [0.8 %] enthält.

Die Kulturen werden bei etwa 30°C mit einer Photoperiode von 16 Stunden Licht/8 Stunden Dunkelheit in einem "Percival" Inkubator inkubiert. Die Beleuchtung erfolgt mit Hilfe von fluoreszierendem Licht (kaltes Tageslicht), bei einer Beleuchtungsstärke von etwa 2000 bis 4000 lx.

An den kultivierten Gewebefragmenten entwickeln sich innerhalb von drei bis vier Wochen Kalli, die eine weisse bis grau-grüne Färbung aufweisen. Die gebildeten Kalli werden alle drei bis vier Wochen auf ein Kallus-Wachstumsmedium überimpft, das m-Inosit [100 mg/Liter], Saccharose [20 g/Liter], Naphthyl-1-essigsäure [2 mg/Liter] und Agar enthält, und dort erneut kultiviert. Vier bis sechs Monate nach dem Transfer der Gewebeexplantate auf das Kallus-induzierende Medium erfolgt die Ausbildung somatischer Embryonen. Der Kallus und die Embryonen werden durch drei- bis vierwöchiges Ueberimpfen auf frisches Kallus-Wachstumsmedium und erneutes Kultivieren am Leben erhalten.

Somatische Embryonen, die sich an Gewebestücken entwickeln, werden entweder auf frisches Kallus-Wachstumsmedium überimpft oder aber auf ein spezifisches Keimungsmedium für Embryonen (Beasley & Ting's Medium) übertragen.

Die Pflänzchen, die sich aus den somatischen Embryonen entwickeln, werden auf ein Beasley and Ting's Medium transferiert, das als Zusatz Ammoniumnitrat [1200 mg/Liter] sowie Caseinhydrolysat [500 mg/Liter] als organische Stickstoffquelle enthält. Das Medium wird durch ein Verfestigungsmittel (Gelrit) stabilisiert und die Pflänzchen werden in "Magenta" Behälter plaziert.

Die somatischen Embryonen entwickeln sich innerhalb von etwa drei Monaten zu Pflänzchen. Die Pflänzchen sind mit Erreichen des 6- bis 8-Blatt-Stadiums [die Grösse der Pflanzen beträgt dann zwischen 7,5 und 10 cm] bewurzelt und werden in Erde übertragen. Die Kultivierung erfolgt für einen Zeitraum von drei bis vier Wochen in einem Inkubator bei hoher Luftfeuchtigkeit. Danach werden die Pflänzchen ins Gewächshaus transferiert. Wenn die Pflänzchen ausgehärtet sind, werden sie in offene, bearbeitete Ackererde ausgepflanzt.

Beispiel 16: Regeneration von ganzen Pflanzen ausgehend von Keimblattexplantaten - Variation 1

Beispiel 15 wird wiederholt, wobei in diesem Fall ein halbkonzentriertes MS-Medium verwendet wird, in dem alle Medienbestandteile in ihrer angegebenen Konzentration auf die Hälfte reduziert sind. Man erhält im wesentlichen die gleichen Resultate wie bei Verwendung des voll-konzentrierten MS-Mediums.

Beispiel 17: Regeneration verschiedener Baumwoll-Varietäten ausgehend von Keimblattexplantaten

Die in den Beispielen 15 und 16 beschriebenen Verfahrensmassnahmen werden unter Verwendung der Acala Baumwoll-Varietäten SJ4, SJ2C-1, GC510, B1644, B2724, B1810, der "Picker" Varietät Siokra und der "Stripper" Varietät FC2017 durchgeführt. Alle zuvor aufgezählten Varietäten können erfolgreich regeneriert werden.

Beispiel 18: Regeneration von Baumwollpflanzen ausgehend von Keimblattexplantaten unter Zwischenschaltung einer Zell-Suspensionskultur.

Die in Beispiel 15 beschriebenen Verfahrensmassnahmen werden bis zur Herstellung eines Kallus, der zur Ausbildung somatischer Embryonen befähigt ist, wiederholt.

Stücke von 750 mg bis 1000 mg des aktiv wachsenden embryogenen Kallus werden in 8 ml Aliquots eines flüssigen Suspensionskultur-Mediums, das sich aus den Makro- und Mikro-Salzen des MS-Mediums zusammensetzt und darüberhinaus Thiamin HCl [0,4 mg/Liter], Saccharose [20 mg/Liter], m-Inosit [100 mg/Liter] und Naphthyl-1-essigsäure [2 mg/Liter] als Zusatz enthält, in "T"-Röhrchen suspendiert und anschliessend auf einer rotierenden Trommel bei einer Umdrehungsgeschwindigkeit von 1,5 Upm und einem Licht/Dunkel Rhythmus von 16 Stunden/8 Stunden inkubiert. Das Licht stammt wiederum aus fluoreszierenden Lampen (kaltes Tageslicht) und hat eine Intensität zwischen 2000 und 4000 1x.

Nach vier Wochen wird die Suspensionskultur durch ein Nylonnetz mit einer Maschenweite von 840 μm filtriert, um grössere Zellklumpen zu entfernen. Die Fraktion, die nur Partikel mit einer Grösse von unter 840 μm umfasst, lässt man absetzen und wäscht anschliessend einmal mit 20 bis 25 ml eines frischen Suspensionskultur-Mediums. Diese Zellsuspension wird in "T"-Röhrchen überführt (2 ml pro Röhrchen) und jedes Röhrchen wird mit 6 ml frischem Suspensionskultur-Medium verdünnt. Die Kulturen werden durch Wiederholung der oben beschriebenen Verfahrensschritte in Intervallen von 10 bis 12 Tagen am Leben erhalten.

Bei jeder Wiederholung des zuvor beschriebenen Kultivierungsschrittes wird die Suspensionskultur erneut durch das 840 μm Netz filtriert und nur diejenige Fraktion, die Zellaggregate von weniger als 840 μm enthält, wird auf frisches Suspensionskultur-Medium überführt. In allen Fällen wird die Fraktion, die Zellklumpen von mehr als 840 μm aufweist, auf ein Kallus-Wachstumsmedium gegeben, um auf diese Weise somatische Embryonen zu erhalten.

Die somatischen Embryonen, die sich auf dem Kallus-Wachstumsmedium entwickeln, werden entfernt und auf ein Keimungsmedium für Embryonen übertragen. Unter Verwendung des in Beispiel 15 beschriebenen Versuchsprotokolls findet zunächst die Keimung der Embryonen statt, die sich dann zu Pflänzchen und anschliessend zu im Freiland wachsenden Pflanzen entwickeln.

Beispiel 19: Regeneration von Baumwollpflanzen ausgehend von Keimblattexplantaten unter Zwischenschaltung von Zell-Suspensions-Kulturen - Variante 1.

Die in Beispiel 18 beschriebenen Verfahrensschritte werden wiederholt, mit der Ausnahme, dass in diesem Fall die 750 bis 1000 mg embryogenen Kallusgewebes in eine DeLong Flasche übertragen werden, die 15 bis 20 ml eines flüssigen MS-Mediums mit 2 mg/Liter NAA enthält.

Die Flasche mit der Kultur wird auf einer Rundschüttelmaschine bei einer Umdrehungsgeschwindigkeit von 100 bis 110 Upm inkubiert. Nach drei Wochen wird die Suspensionskultur durch ein Nylonnetz mit einer Maschenweite von 840 μm filtriert, um auf diese Weise grosse Zellklumpen für die Entwicklung ganzer Pflanzen zu gewinnen, wie in Beispiel 18 beschrieben.

Diejenige Fraktion, die Zellen mit einer Grösse von weniger als 840 μm aufweist, lässt man zunächst absetzen, wäscht diese anschliessend einmal in einem flüssigen MS-Medium und resuspendiert dann in 2 ml bis 5 ml eines flüssigen MS-Mediums.

Die Zellsuspension wird erneut kultiviert und zwar durch Uebertragung von 1 ml bis 2 ml der Suspension in eine DeLong Flasche, die frisches MS-Medium enthält.

Die Kulturen werden durch Wiederholen der oben beschriebenen Verfahrensschritte in Intervallen von sieben bis zehn Tagen am Leben erhalten.

Für jede erneute Kultivierung werden lediglich diejenigen Suspensionen verwendet, die Zellen mit einer Grösse von weniger als 840 μm aufweisen. Die grösseren Zellklumpen (840 μm und grösser) werden für die Entwicklung ganzer Pflanzen verwendet.

Beispiel 20: Herstellung ganzer Pflanzen aus grossen Zellklumpen aus Suspensionskulturen

Nach drei- bis viermaliger Wiederholung der Kultivierung unter Verwendung der in den Beispielen 18 und 19 beschriebenen Versuchsprotokolle, werden 1,5 ml bis 2 ml der Zellsuspension von den "T"-Röhrchen bzw. den DeLong Flaschen entnommen und auf mit Agar verfestigtes MS-Medium übertragen, das 2 mg/Liter NAA enthält, sowie ein Beasley & Ting Medium, das mit 500 mg/Liter Caseinhydrolysat angereichert ist. Nach drei bis vier Wochen werden embryogene Kalli mit sich entwickelnden Embryonen sichtbar.

Auch in diesem Fall werden Zellklumpen mit 840 μm und mehr auf ein Kallus-Wachstumsmedium übertragen. Aus diesen Zellklumpen erhält man embryogene Klumpen mit sich entwickelnden Embryonen, die zuletzt zu ganzen Pflanzen heranwachsen.

Beispiel 21: Transformation von Baumwoll-Suspensionskulturzellen mit Hilfe von *Agrobacterium* LBA 4434

### 21.1. Wachstum der pflanzlichen Suspensionskultur

Eine Acala Baumwoll-Suspensionskultur (wie in Beispiel 18 beschrieben) wird in "T"-Röhrchen wiederholt kultiviert, wobei das Medium (MS-Medium mit 2 mg/Liter NAA) alle sieben bis zehn Tage gewechselt wird. Nach dem Mediumwechsel wird das "T"-Röhrchen jeweils um 90° gedreht, und man erlaubt den Zellen, sich abzusetzen. Vor der Transformation wird der Ueberstand mit Hilfe einer Pipette abgehoben und die resultierenden Zellen werden wie zuvor beschrieben behandelt.

### 21.2. Beschreibung des *Agrobacterium* Vektors

Der *Agrobacterium* Stamm LBA 4434 (Hoekema et al, 1983) enthält ein binäres, auf dem Ti-Plasmid basierendes Pflanzen-Transformationssystem. In einem solchen binären Transformationssystem enthält eines der Plasmide die T-DNA Region des Ti-Plasmids, während das zweite Plasmid die vir-Region des Ti-Plasmids aufweist. Die Transformation der Pflanze wird durch das Zusammenwirken beider Plasmide gewährleistet.

Im *Agrobacterium* Stamm LBA 4434 enthält das T-DNA Plasmid pAL1050 die $T_L$ Sequenz von pTiAch5, einem Octopin-Ti-Plasmid. Das vir-Plasmid im Stamm LBA 4434, pAL4404, enthält dagegen die intakte Virulenzregion von pTiAch5 (Ooms et al, 1982). Der *Agrobacterium* Stamm 4434 kann von Dr. Robert Schilperoort, Abteilung für Biochemie, Universität Leiden, Niederlande, bezogen werden.

### 21.3 Kultivierung der Agrobakterien; Wachstumsbedingungen

Der transformierende *Agrobacterium* Stamm wird einer Glycerin-Stammlösung entnommen und in Form einer Uebernachtkultur kultiviert. Am nächsten Tag wird aus dieser Uebernachtkultur ein Aliquot entnommen und damit eine 50 ml-Kultur angeimpft. Die Agrobakterien werden auf YEB Medium kultiviert, das die folgende Zusammensetzung aufweist:

| | |
|---|---|
| Rindfleischextrakt | 5 g/Liter |
| Hefeextrakt | 1 g/Liter |
| Pepton | 5 g/Liter |
| Saccharose | 5 g/Liter |

Die Lösung wird mit Hilfe von NaOH auf einen pH von 7,2 eingestellt. Nach dem Autoklavieren wird 1 ml einer 2 M $MgCl_2$-Lösung zugegeben.

Zu dem YEB Medium werden geeignete Antibiotika in einer geeigneten Konzentration zugegeben.

Man bestimmt die optische Dichte der 50 ml Uebernachtkultur bei 600 nm ($OD_{600}$), zentrifugiert die Kultur und resuspendiert das Pellet in einem Wachstumsmedium für Pflanzenzellen (MS-Medium plus NAA in einer Konzentration von 2 mg/ml) bis eine $OD_{600}$ von 0,5 erreicht ist.

8 ml dieser bakteriellen Suspension wird zu jedem "T"-Röhrchen, das die pflanzlichen Zellen gemäss Abschnitt 21.1. enthält, zugegeben.

### 21.4. Infektion

Die "T"-Röhrchen mit den Pflanzen- und Bakterienzellen werden zunächst geschüttelt, um alle Zellen zu resuspendieren, und anschliessend erneut für einen Zeitraum von 3 Stunden auf einer rotierenden Trommel inkubiert, um den Agrobakterien die Möglichkeit zu geben, sich an die Pflanzenzellen anzuheften. Man lässt dann die Zellen absetzen und entfernt den restlichen Ueberstand.

Anschliessend wird ein Aliquot frisches Wachstumsmedium zu den "T"-Röhrchen zugegeben. Diese werden auf einer Schüttelvorrichtung für einen Zeitraum von 18 bis 20 Stunden in Gegenwart eventuell noch vorhandener Agrobakterien inkubiert. Danach lässt man die Zellen erneut absetzen, entfernt den Ueberstand und wäscht die Zellen zweimal mit einer Lösung aus Wachstumsmedium und Cefotaxim (200 µg/ml).

Nach dem Waschen werden die Zellen aus den einzelnen "T"-Röhrchen in jeweils 10 ml Wachstumsmedium mit Cefotaxim (jeweils 200 µg/ml) resuspendiert, und jeweils 1 ml Aliquots davon werden auf Petrischalen ausplattiert.

### 21.5. Kultivierung des transformierten Gewebes

Die mit Agrobakterien infizierten Zellen wachsen auf dem Wachstumsmedium, das keine Hormonzusätze enthält, und demonstrieren dadurch, dass das Gewebe das Wild-Typ Phytohormongen in Form einer T-DNA erhält.

Die entsprechenden Zellen entwickeln sich zu Tumoren und bieten damit ein weiteres Indiz für die erfolgreiche Transformation der Kulturen.

Beispiel 22: Transformation von Baumwoll-Suspensionskulturzellen zu einem Kanamycin-resistenten, nicht-tumorösen Phänotyp

Es werden die gleichen Verfahrensmassnahmen, die zuvor in Beispiel 21 beschrieben wurden, angewendet, abgesehen davon, dass andere transformierende Agrobacterien verwendet werden und dass das Pflanzen-Selektionsmedium ein Antibiotikum für die Selektionierung transformierter pflanzlicher Gewebe enthält.

## 22.1. Kultivierung des pflanzlichen Gewebes

Die Kultivierung des pflanzlichen Gewebes erfolgt analog der in Beispiel 21, Abschnitt 21.1. angegebenen Verfahrensmassnahmen.

## 22. 2. Beschreibung des *Agrobacterium*-Vektors

Die transformierenden Agrobakterien besitzen den T-DNA-enthaltenden binären Vektor pCIB10 (Rothstein et al, 1987) sowie das vir-Plasmid pAL4404. Die T-DNA von pCIB10 enthält ein chimäres Gen, das aus dem Promotor des Nopalinsynthasegens, der kodierenden Region von Tn5 (die das Enzym Neomycinphosphotransferase kodiert) und dem Terminator des Nopalinsynthasegens zusammengesetzt ist. Der *Agrobacterium* Stamm LBA44O4, der das vir-Hefeplasmid pAL4404 (gemäss obiger Beschreibung) enthält, kann ebenfalls von Dr. R. Schilperoort bezogen werden.

## 22.3. Kultivierung der Agrobacterien, Wachstumsbedingungen

Agrobakterien mit dem Plasmid pCIB10 werden auf YEB Medium mit 50 μg/ml Kanamycin kultiviert. Die übrigen Kultivierungsbedingungen entsprechen denjenigen, die in Beispiel 21, Abschnitt 21.3. beschrieben sind.

## 22.4. Infektion

Die Transformation pflanzlicher Zellen erfolgt gemäss den in Beispiel 21 beschriebenen Verfahrensmassnahmen. In diesem Fall werden jedoch die aus Abschnitt 21.3 resultierenden Aliquots (1 ml) direkt auf mit selektiven Antibiotika angereicherten Medien ausplattiert.

Das Selektionsmedium enthält entweder Kanamycin (50 μg/ml) oder G418 (25 μg/ml). Die Expression des chimären nos/neo/nos Gens in transformiertem pflanzlichem Gewebe erlaubt die Selektion dieses Gewebes auf eines der zuvor genannten Antibiotika.

## 22.5. Kultivierung des transformierten Gewebes

In diesem und in allen folgenden Beispielen enthalten die Pflanzen-Wachstumsmedien Phytohormone gemäss den in Beispiel 15 gemachten Angaben.

Nach 2 bis 4 Wochen wird das transformierte Gewebe auf den Selektionsplatten sichtbar. Nicht-infiziertes sowie Kontrollgewebe zeigt keine Anzeichen von Wachstum. Es wird schliesslich braun und stirbt ab. Transformiertes Gewebe wächst dagegen auch in Gegenwart von Kanamycin und G418 sehr gut. Zu diesem Zeitpunkt werden gut wachsende Gewebestücke auf frisches Selektionsmedium überimpft und dort weiter kultiviert.

## 22. 6. Kultivierung somatischer Embryonen

Aus den Gewebestücken bilden sich somatische Embryonen aus. Sie werden auf frisches Medium überimpft (nicht-selektives Medium).

## 22.7. Keimung

Wenn die Embryonen beginnen, auszudifferenzieren und zu keimen, d.h. zu dem Zeitpunkt, da sie mit der Bildung von Wurzeln beginnen und 2 bis 3 Blätter aufweisen, werden sie auf 'Magenta'-Behälter mit Wachstumsmedium übertragen. Die Pflänzchen werden noch bis zum 6 bis 8 Blattstadium weiterkultiviert, bevor sie von dem Agar-Medium entfernt werden.

## 22.8. Kultivierung der Pflänzchen

Die Pflänzchen werden jetzt in Topferde gepflanzt und zur Aufrechterhaltung einer hohen Luftfeuchtigkeit mit einem Becherglas abgedeckt und in einem "percival" Inkubator für 4 bis 8 Wochen kultiviert. Zu diesem Zeitpunkt wird das Becherglas entfernt und die Pflanzen werden ins Gewächshaus gebracht.

## 22.9. Kultivierung der Pflanzen im Gewächshaus

Die Pflanzen wachsen im Gewächshaus heran, entwickeln Blüten und bilden schliesslich Samen.

## Beispiel 23: Transformation von Zellen aus einer Baumwoll Suspensionskultur zu einem Glyphosate-toleranten Phänotyp

Sofern im folgenden keine anderslautenden Angaben gemacht werden, erfolgt die Durchführung der Experimente gemäss den Angaben in Beispiel 22.

Im Unterschied zu Beispiel 22 werden in diesem Fall andere transformierende Agrobakterien verwendet. Darüberhinaus wird nach der Selektion des pflanzlichen Gewebes auf einem für die Selektion von transformiertem pflanzlichem Material geeigneten Antibiotikum-haltigen Medium erneut selektioniert, diesmal auf Herbizid-Toleranz.

### 23.1. Beschreibung des *Agrobacterium*-Vektors

Die transformierenden Agrobakterien enthalten den T-DNA Vektor pPMG85/587 (Fillatti et al, 1987) sowie das vir-Plasmid pAL4404. Das Plasmid pPMG85/587 enthält drei chimäre Gene, die zu einer Expression in Pflanzen in der Lage sind. Zwei dieser Gene kodieren Neomycinphosphotransferase (NPT), ein Enzym, das eine Resistenz gegenüber dem Antibiotikum Kanamycin bzw. G418 verleiht. Das dritte chimäre Gen, welches die kodierende Sequenz eines mutierten aroA Gens aus *Salmonella typhimurium* besitzt, verleiht eine Toleranz gegenüber dem Herbizid Glyphosate (Comai et al, 1983).

### 23.2. Kultivierung des pflanzlichen Gewebes

Die Kultivierung des pflanzlichen Gewebes erfolgt analog der in Beispiel 21, Abschnitt 21.1. angegebenen Verfahrensmassnahmen.

### 23.3. Kultivierung der Agrobakterien

Die Agrobakterien mit dem T-DNA Vektor pPMG85/587 werden auf Kanamycinhaltigen Kulturmedien (100 µg/ml) herangezogen.

### 23.4. Infektion

Die Transformation wird gemäss den in Beispiel 21 im Einzelnen ausgeführten Verfahrensschritten durchgeführt. In diesem Fall werden jedoch die in Abschnitt 21.3. resultierenden Aliquots (je 1 ml) direkt auf die Medien mit den selektiven Antibiotika ausplattiert.

Diese Selektionsmedien enthalten entweder Kanamycin (50 µg/ml) oder G418 (25 µg/ml). Die Expression des chimären NPT Gens im pflanzlichen Gewebe erlaubt die Selektion des transformierten Pflanzengewebes auf einem dieser beiden Medien.

### 23.5. Kultivierung des transformierten Gewebes

Nach 2 bis 4 Wochen wird das transformierte Gewebe auf den Selektionsplatten sichtbar. Die Selektion des pflanzlichen Materials erfolgt in der Regel auf Kanamycin-haltigen Medien.

Das Pflanzengewebe (entweder einzelne Embryonen oder Kallus) wird anschliessend auf Medien überführt, die das Herbizid Glyphosate als Zusatz enthalten. Das transformierte Gewebe zeigt weiterhin gutes Wachstum.

### Beispiel 24: Transformation von Zellen aus einer Baumwoll Suspensionskultur zu einem Hygromycin-resistenten, nicht-tumorösen Phänotyp

Sofern im folgenden keine anderslautenden Angaben gemacht werden, erfolgt die Durchführung der Experimente gemäss den Angaben in Beispiel 22.

Im Unterschied zu Beispiel 22 werden in diesem Fall andere transformierende Agrobakterien verwendet. Darüberhinaus enthält das Pflanzenselektionsmedium ein Antibiotikum, das für die Selektion von transformiertem pflanzlichem Gewebe geeignet ist.

### 24.1. Kultivierung des pflanzlichen Gewebes

Die Kultivierung des pflanzlichen Gewebes erfolgt analog der in Beispiel 21, Abschnitt 21.1. angegebenen Verfahrensmassnahmen.

### 24.2. Beschreibung des *Agrobacterium*-Vektors

Die transformierenden Agrobakterien enthalten den binären T-DNA Vektor pCIB2115 (Rothstein et al, 1987) sowie das vir-Plasmid. Die T-DNA von pCIB2115 enthält ein chimäres Gen, das aus dem Promotor und dem Terminator des 35S Transkripts von CaMV (Odell et al, 1985) sowie der kodierenden Sequenz der Hygromycinphosphotransferase (Gritz und Davies, 1983) zusammengesetzt ist.

### 24.3. Kultivierung der Agrobakterien

Die Agrobakterien, welche das Plasmid pCIB2115 enthalten, werden auf YEB Medium, das 50 µg/ml Kanamycin enthält, kultiviert.

### 24.4. Infektion

Die Transformation wird gemäss den in Beispiel 21 im Einzelnen ausgeführten Verfahrensschritten durchgeführt. In diesem Fall werden jedoch die in Abschnitt 21.3 resultierenden Aliquots (je 1 ml) direkt auf die Medien mit den selektiven Antibiotika ausplattiert.

Das Selektionsmedium enthält 50 µg/ml Hygromycin. Die Expression des chimären Hygromycin Gens in transformiertem pflanzlichem Gewebes erlaubt die Selektion dieses Gewebes auf Hygromycin-haltigen Medien.

### 24.5.: Kultivierung des transformierten Gewebes

Die Kultivierung erfolgt analog der in Beispiel 22.5 beschriebenen Verfahrensmassnahmen. Das in dem Pflanzen-Selektionsmedium verwendete Antibiotikum ist in diesem Fall Hygromycin.

### Beispiel 25: Pflanzenextraktionsverfahren

Das pflanzliche Gewebe wird in einem Extraktionspuffer homogenisiert (ca. 100 mg Gewebe in 0,1 ml Extraktionspuffer)

Extraktionspuffer für
Blattgewebe

| | |
|---|---|
| Na$_2$CO$_3$ (pH 9,5) | 50 mM |
| EDTA | 10 mM |
| Triton X-100 | 0,05 % |
| Tween | 0,05 % |
| NaCl | 100 mM |
| PMSF (erst kurz vor Gebrauch zugeben) | 1 mM |
| Leupeptin (erst kurz vor Gebrauch zugeben) | 1 mM |

Nach der Extraktion wird der pH-Wert des Extraktes durch Zugabe von 2 M Tris-Puffer auf einen Wert von pH 8,0 bis 8,5 eingestellt. Der Extrakt wird dann 10 Minuten in einer Beckman Mikrozentrifuge zentrifugiert und der so gewonnene Ueberstand für die ELISA Analyse verwendet.

### Beispiel 26: ELISA Analyse des Pflanzenextraktes

ELISA ("enzyme-linked immunosorbent assay") ist ein sehr sensitiver und spezifischer Assay für antigenwirksames Material. Die ELISA Assays sind für den Nachweis der Expression von Polypeptid-Genprodukten sehr gut geeignet.

Die Entwicklung des ELISA Assays als generellem Hilfsmittel ist bei Clark et al (1986) beschrieben. Diese Beschreibung ist in Form einer Referenz ein Bestandteil dieser Erfindung.

Ein ELISA-Assay für das Bt Toxin wird unter Verwendung von Standardverfahren entwickelt und wird für die Analyse von transgenem Pflanzenmaterial auf die Expression von Bt Sequenzen benützt. Dabei werden die folgenden Verfahrensschritte angewendet:

Medien und Puffer

EPBS ("ELISA Phosphate Buffered Saline")

| | | |
|---|---|---|
| 10 mM NaPhosphat: | Na$_2$HPO$_4$ | 4,68 g/4 Liter |
| | NaH$_2$PO$_4$•H$_2$O | 0,976 g/4 Liter |
| 140 mM NaCl | NaCl | 32,7 g/4 Liter |
| pH ca. 7,4 | | |

Borat-gepufferte Salzlösung
100 mM Borsäure
25 mM Na-Borat
75 mM NaCl
Einstellen des pH-Wertes auf 8,4 bis 8,5 mit HCl oder NaOH, je nach Bedarf.

ELISA Blockadepuffer
In EPBS:
1 % BSA ("Bovine Serum Albumine")
0,02 % Na-Azid

ELISA Waschpuffer
10 mM Tris-HCl; pH 8,0
0,05 % Tween 20
0,02 % Na-Azid

2,5 M TRIS

ELISA Verdünner
In EPBS:
0,05 % Tween 20
1 % BSA

0,02 % Na-Azid

ELISA Substratpuffer
In 500 ml:
48 ml Diethanolamin,
24,5 mg $MgCl_2$;
Einstellen des pH Wertes auf 9,8 mit HCl.

ELISA Substrat
15 mg p-Nitrophenylphosphat in 25 ml ELISA Substratpuffer

Verfahren

1. Eine ELISA-Platte wird mit Ethanol vorbehandelt.

2. Ein über eine Affinitätschromatographie gereinigtes Kaninchen-anti-*Bt*-Toxin Antiserum (50 µl) wird in einer Konzentration von 3 µg/ml in einer Borat-gepufferten Salzlösung auf die Platte gegeben und über Nacht bei einer Temperatur von 4°C inkubiert. Das Antiserum wird als Antwort auf die Immunisierung von Kaninchen mit über einen Gradienten gereinigten *Bt* Toxinkristallen (Ang und Nickerson, 1978), die mit Natriumdodecylsulfat solubilisiert werden, gebildet.

3. Waschen mit ELISA Waschpuffer.

4. Behandlung der Platte mit Blockadepuffer für 1 Stunde bei Raumtemperatur.

5. Waschen mit ELISA Waschpuffer.

6. Hinzufügen des Pflanzenextraktes in einer Menge, die einer Proteinmenge von 50 µg entspricht (dies entspricht typischerweise ca. 5 µl Pflanzenextrakt). Die Zusammensetzung des Blattextraktionspuffers ist in Beispiel 25 beschrieben. Der Proteinanteil wird mit Hilfe der Bradford-Methode ermittelt (Bradford, 1976), unter Verwendung einer kommerziell erhältlichen Testpackung (Bio-Rad, Richmond, Kalifornien). Sofern eine Verdünnung des Blattextraktes notwendig ist, verwendet man einen ELISA Verdünner. Dieser Ansatz wird über Nacht bei 4°C inkubiert.

7. Waschen mit ELISA Waschpuffer.

8. Zugabe von 50 µl eines über eine Affinitätschromatographie gereinigten Ziegen-anti-*Bt*-Toxin Antiserums, in einer Konzentration von 3 µg Protein/ml in ELISA Verdünner. Inkubation für eine Stunde bei einer Temperatur von 37°C.

9. Waschen mit ELISA Waschpuffer.

10. Zugabe von 50 µl Kaninchen-anti-Ziegen-Antikörpern gebunden an alkalische Phosphatase (kommerziell erhältlich von Sigma Chemicals, St. Louis, Mo.). Dieser Ansatz wird im Verhältnis 1:500 in ELISA Verdünner verdünnt. Inkubation für eine Stunde bei 37°C.

11. Waschen mit ELISA Waschpuffer.

12. Zugabe von 50 µl Substrat (0,6 mg/ml p-Nitrophenylphosphat in ELISA Substratpuffer). 30 Minuten bei Raumtemperatur inkubieren.

13. Beendigen der Reaktion durch Zugabe von 50 µl 3 M NaOH.

14. Messen der Absorption bei 405 nm in einem modifizierten "ELISA-Reader" (Hewlett Packard, Stanford, Ca.).

Pflanzliches Gewebe, das mit dem pCIB10/35Sbt(BclI) Konstrukt (Fig. 16) transformiert ist, zeigt bei der Analyse unter Verwendung des ELISA Assays eine positive Reaktion, was auf eine Expression des *Bt* Gens hinweist.

Beispiel 27: Bioassay transformierter Baumwolle

Eier von *Heliothis virescens* werden vom "Tobacco Insect Control Laboratory at North Carolina State University, Raleigh, North Carolina" bezogen.

Die Eier befinden sich auf einer Mullunterlage, die in grosse gedeckte Bechergläser überführt wird. Das Becherglas ist mit feuchtem Filterpapier ausgekleidet, um eine gleichbleibende Feuchtigkeit zu garantieren. Die Inkubation der *Heliothis*-Eier erfolgt bei einer Temperatur von 29°C.

Unter diesen Bedingungen schlüpfen die Larven innerhalb von drei Tagen. Nach dem Schlüpfen werden die Larven sobald wie möglich in kleine gedeckte Plastikbehälter überführt (1 Larve/Behälter), die jeweils eine Baumwollblattscheibe enthalten.

Die Uebertragung der Larven erfolgt mit Hilfe eines feinborstigen Pinsels.

Die Blattscheiben, die einen Durchmesser von einem Zentimeter aufweisen, werden zuvor aus den Blätter von Baumwollpflanzen ausgestanzt und in dem Plastikbehälter auf ein rundes angefeuchtetes Filterpapier aufgebracht. Von jeder Pflanze werden mindestens 6 bis 8 Blattscheiben, die sowohl von jungen als auch von alten Blätter stammen, getestet. Die Blattscheiben werden in 2 Tagesintervallen oder aber in Abhängigkeit von der Nahrungsaufnahme nach Bedarf ersetzt.

Die Wachstumsraten (die Grösse oder das Gesamtgewicht aller innerhalb einer Versuchsgruppe befindlichen Larven) sowie die Sterblichkeitsrate der Larven, die sich von Blättern transformierter Pflanzen ernähren, werden mit denjenigen der Kontrolltiere, die sich von nichttransformierten Baumwollblättern ernähren, verglichen.

Larven, die sich von Blattscheiben ernähren, die von Baumwollpflanzen stammen, welche mit pCIB10/35Sbt(BclI) transformiert sind, zeigen eine verminderte Wachstumsrate sowie eine erhöhte Sterblichkeit im Vergleich zu den Kontrollen.

## Beispiel 28: Konstruktion des Plasmids pCIB1300, das zu hohen Expressionsraten in Pflanzen führt

Zur Erzielung hoher Expressionsraten des *Bt* Toxingens in Pflanzen wird das Plasmid pCIB1300 konstruiert. Diese Plasmid enthält ein nicht-translatierte Leader-Sequenz 5' zu dem *Bt* Toxingen, die zu einer Steigerung der *Bt* Toxin-Genexpression führt.

Bei der nicht translatierten Leader-Sequenz handelt es sich um eine 40 Bp umfassende Sequenz, die 5' zum Initiationskodon des *Bt* Toxingens und 3' zum nicht-translatierten CaMV Leader liegt.

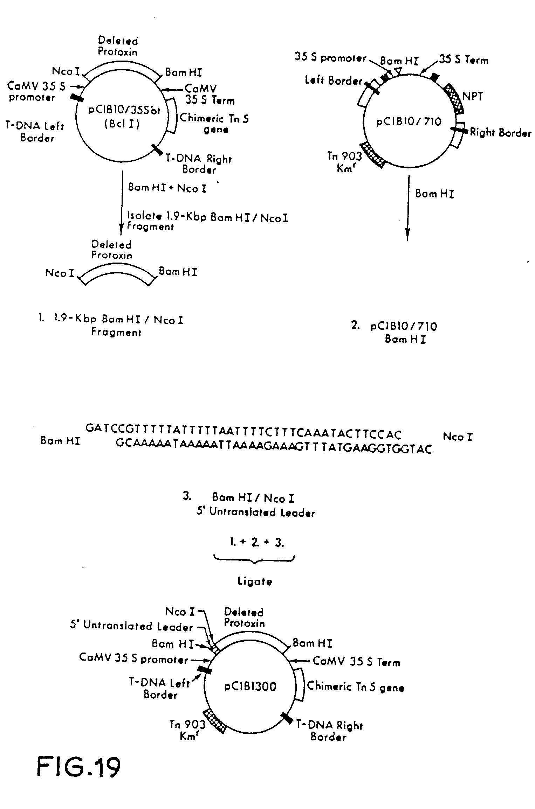

Das endgültige pCIB1300 Plasmid wird konstruiert, indem die 40 Bp umfassende Leader-Sequenz und ein deletiertes *Bt* Toxingen in die BamHI Restriktionsschnittstelle des Plasmids pCIB10/710 eingespleisst werden, wie dies in Fig. 19 gezeigt wird.

Ein 1.9 kBp NcoI-BamHI Fragment des Plasmids pCIB20/35Sbt(Bcl) wird mit Hilfe einer bei niedrigen Temperaturen gelierenden Agarose gereinigt.
Die 40 Bp Leader Sequenz wird auf chemischem Wege mit Hilfe eines "applied Biosystems DNA Synthesizer" synthetisiert und zwar in Form eines doppelsträngigen Oligonukleotids mit einer überhängenden BamHI Schnittstelle am 5' Ende und einer überhängenden Ncol-Schnittstelle am 3' Ende. Die Sequenz der nicht-translatierten Leadersequenz, die in der Mitte der Fig. 19 wiedergegeben ist, leitet sich vom nicht-translatierten Leader des Hüllproteins des Alfalfa Mosaik Virus (AMV) ab, der bei Koper-Zwarthoff et al. (1977) beschrieben ist.

Der 40 Bp Leader, ein 1.9 kBp *Bt* Fragment, sowie der mit BamHI linearisierte pCIB710 Vektor werden unter Verwendung von T4 DNA-Ligase miteinander verknüpft ("three-part ligation"), wobei der Vektor pCIB1300 entsteht.

## Beispiel 29: Isolierung eines CDNA Klons, der die kleine Untereinheit der RuBPCase in Baumwolle kodiert

*Gossypium hirsutum* (Funk line RF522) Pflanzen werden im Gewächshaus bei einer täglichen Lichtperiode von 14 Stunden aus Samen herangezogen. Aus jungen grünen Blättern wird anschliessend die RNA gemäss dem bei Newbury und Possingham (1977) beschriebenen Verfahren isoliert. Die Reinigung der PolyA+ RNA ist bei Maniatis et al. (1982) auf Seite 197 beschrieben.

Doppelsträngige cDNA < komplementäre DNA) wird gemäss dem Verfahren von Okayama und Berg (1982) synthetisiert, wobei die folgenden Modifikationen durchgeführt werden:

    a) die Erststrang cDNA wird mit einem oligo-dT Starter versehen,

    b) nach dem Anfügen von oligo-dG Schwänzen an die doppelsträngige cDNA unter Verwendung von Polynukleotidyltransferase wird sie in das Plasmid pUC9 (PstI Schnittstelle, von Pharmacia) kloniert, das zuvor mit oligo-dC Anhängen versehen wurde, und über die komplementären Basen verknüpft, und

    c) mit der resultierenden DNA wird *E. coli* Stamm HB101 transformiert.

Da RuBPCase (Ribulosebisphosphatcarboxylase) zusammen mit dem Chlorophyll a/b Bindungsprotein (Cab) zu den Proteinen gehört, die am häufigsten in grünen Blättern vorkommen, wird die Genbibliothek auf die cDNA Klone der am häufigsten vorliegenden mRNA hin untersucht.

Die auf Nitrocellulosefiltern vorliegenden Abdrücke der cDNA Klone werden mit dem ersten cDNA Strang gescreent, der mit Hilfe von dCT32P und reverser Transkriptase radioaktiv markiert wird, wobei die gleiche polyA+ RNA als Vorlage ("Template") dient, die auch zuvor bereits für die Konstruktion der cDNA Bibliothek verwendet wurde.

Auf diese Weise werden 6 von insgesamt 275 Klonen selektioniert und anschiessend weiter analysiert.

Die Northern-Analyse, die gemäss der bei Maniatis et al. (1982) auf Seite 202 gemachten Beschreibung durchgeführt wird, zeigt, dass zwei dieser cDNA Klone mit einer Klasse von mRNA Molekülen hybridisieren, die eine Länge von 1100 nt (Nukleotiden) aufweisen. Diese zeigen eine Kreuz-Hybridisierung mit einer Cab-Gensonde aus Tabak. Die anderen 4 cDNA Klone hybridisieren mit einer Klasse von mRNA-Molekülen mit einer Grösse von 900 bis 1000 nt, was der Grösse der rbcs (mRNA der kleinen Untereinheit der Ribulosebisphosphatcarboxylase) entspricht.

Nach Durchführung einer Hybridselektion mit Hilfe dieser 4 cDNA Klone wird mRNA aus Baumwollblättern freigesetzt und in-vitro [wie bei Maniatis et al (1982) beschrieben] unter Verwendung eines in-vitro Retikulocyten-Translationskits (Promega Biotec) translatiert.

Eine anschliessend durchgeführte Elektrophorese an einem Polyacrylamidgel zeigt eine Hauptpolypeptidbande bei ungefähr 20 Kd. Dies entspricht dem Molekulargewicht der RuBPCase Vorstufe (Precursor).

Die anderen drei cDNA Klone zeigen eine Kreuzhybridisierung mit dem Klon, der für das Hybrid-Freisetzungsexperiment verwendet wurde. Grosse Teile dieser cDNA Klone werden nach einer Klonierung im Phagen M13 unter Verwendung der Didesoxy-Kettenabbruch Technik (Sanger et al, 1977) sequenziert. Ein Vergleich dieser Sequenzen mit zuvor bereits aus anderen Spezies isolierten und publizierten rbcS Sequenzen macht deutlich, dass es sich auch in diesem Fall um rbcS cDNA Klone handelt.

### Beispiel 30: Isolation genomischer Klone der kleinen Untereinheit der Baumwoll RuBPCase

#### 30.1. Genomische "Southern Blot" Analyse von Baumwolle

Genomische "Southern Blots" werden mit Hilfe von Standardverfahren unter Verwendung von Nitrocellulosefiltern hergestellt. Die Prähybridisierungs-, Hybridisierungs- und Waschbedingungen entsprechen denjenigen, die bei Klessig et al (1983) beschrieben sind.

Die genomische "Southern Blot" Analse unter Verwendung des zuvor beschriebenen rbcS cDNA Klones als Sonde liefert 4 bis 5 genomische Fragmente, je nach dem welche Restriktionsenzyme für die Verdauung der DNA verwendet werden.

Die RuBPCase in Baumwolle wird durch eine kleine Genfamilie kodiert, wie dies auch in anderen, früher bereits untersuchten Spezies der Fall ist. Die rbcS Multigenfamilie der Baumwolle umfasst schätzungsweise mindestens 5 Mitglieder.

#### 30.2. Isolation von genomischen rbcS Klonen

Für die Konstruktion einer genomischen Baumwoll-Genbibliothek wird die mit Sau3a teilweise verdaute Baumwoll-DNA über einen l0%igen bis 40%igen Saccharose-Gradienten nach der Grösse aufgetrennt und anschliessend in die λ EMBL3 Arme (Stratagene) eingespleisst.

Die Verpackung der λ Rekombinanten erfolgt unter Verwendung eines "Packagene Kits" (Stratagene) Es schliess sich eine Transfektion in den *E. coli*-Stamm K802 an.

Duplizierte Nitrocellulosefilter-Abdrücke werden, wie bei Maniatis et al. (1982) beschrieben (Seite 320), unter Verwendung des oben beschriebenen rbcS cDNA Klons als Probe gescreent.

Zwölf positive Klone werden aus einer Gesamtzahl von 450'000 Plaques herausgereinigt. Die DNA wird aus Plattenlysaten dieser rekombinanten Phagen isoliert, wie dies bei Maniatis et al (1982) auf Seite 80 beschrieben ist.

Nach einem Vergleich dieser genomischen Klone anhand ihrer Restriktionsmuster, die mit verschiedenen Restriktionsenzymen erhalten werden, können insgesamt 5 verschiedene rbcS Gene identifiziert werden. Jeder dieser Klone wird erneut in dem Plasmidvektor pBSM13 (Stratagene) kloniert.

Die erhaltenen Subklone werden anschliessend vermessen ("mapped") und teilweise sequenziert, um auf diese Weise das 5' Ende sowie das erste ATG-Kodon (Translations-Startsignal) des Gens aufzufinden.

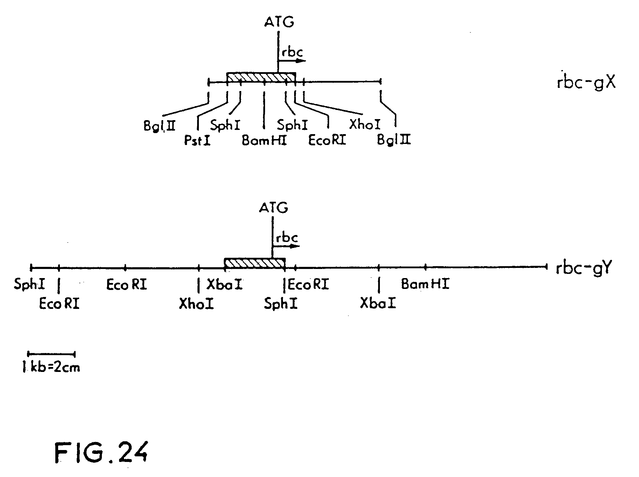

Eine Gen-Karte von zwei dieser genomischen Subklone, rbc-gX und rbc-gY ist in Fig. 24 wiedergegeben.

Die λ Phagen EMBL3, die die genomische DNA der rbc-gX sowie der rbc-gY Subklone enthalten, sind bei der als Internationale Hinterlegungsstelle anerkannten "American Type Cultur Collection" in Rockville, Maryland hinterlegt.

#### 30.3. Untersuchung der Expressionsrate von rbcS Genfragmenten in Baumwollblättern

41 weitere rbcS cDNA Klone werden aus der cDNA-Bibliothek von Baumwollblättern isoliert. Die Restriktionsanalyse, Sequenzierung und Hybridisierung dieser cDNA Klone mit Gen-spezifischen Sonden erlaubt die Schlussfolgerung, dass das Gen, welches in dem genomischen rbc-gX Klon enthalten ist, für ca. 17 % der rbcs Transkripte im Baumwollblatt verantwortlich ist.

### Beispiel 31: Konstruktion chimärer Gene unter Verwendung von rbcS Promotoren aus Baumwolle

#### 31.1. Insertion einer NcoI Schnittstelle am ersten ATG Kodon der rbcs Transitpeptide kodierenden Gene

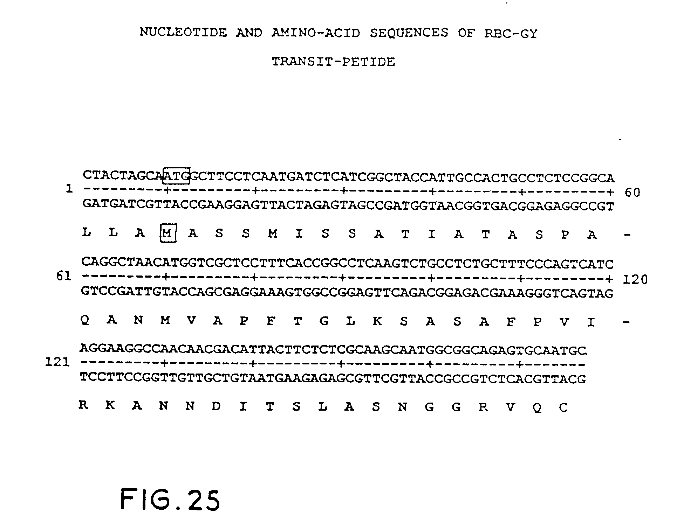

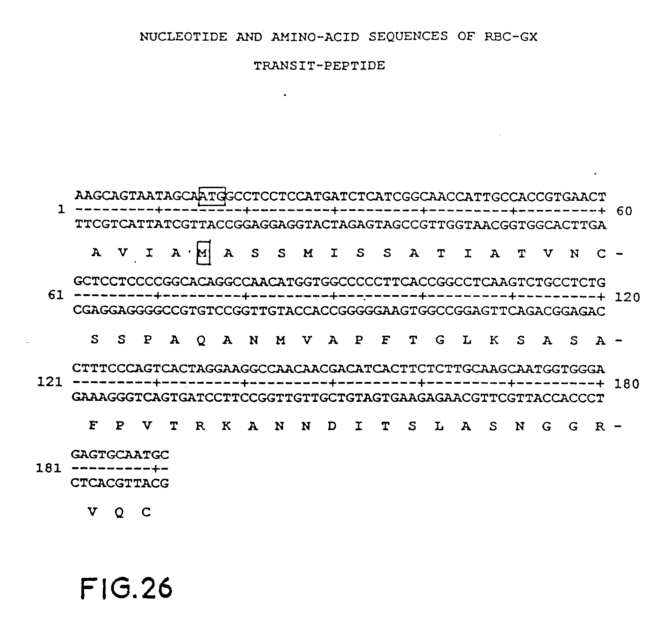

Die Sequenzen der Transitpeptide rbc-gX und rbc-gY sowie der kodierenden Gene sind in den Fig. 26 und 25 wiedergegeben.

Am ersten ATG Startkodon dieser Transitpeptid-kodierenden Gene wird eine NcoI Schnittstelle (CCATGG) eingefügt.

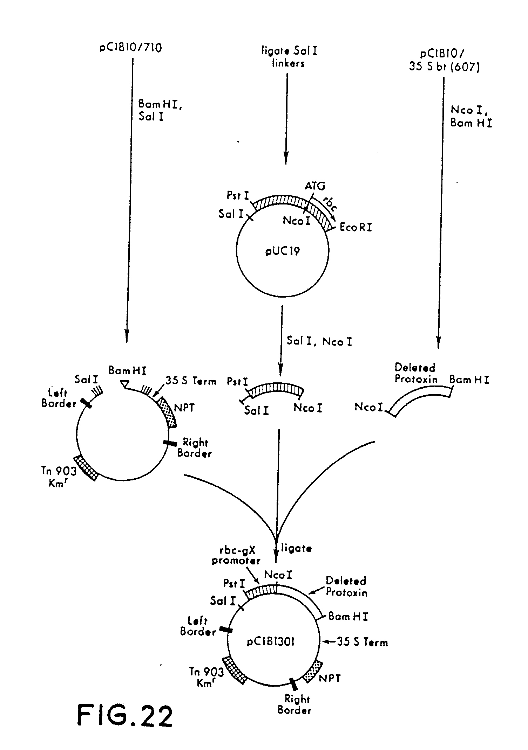

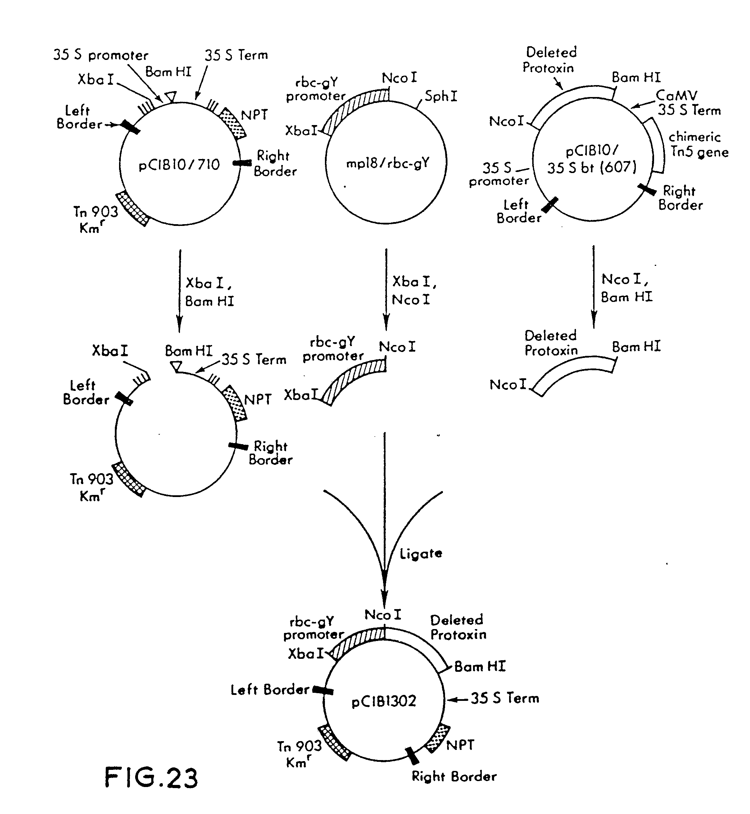

Dies wird erreicht durch Klonierung des PstI-EcoRI Fragmentes des rbc-gX Gens und des XbaI-SPhI Fragmentes des rbc-gY Gens (gestreifte Fragmente in Fig. 22 und Fig. 23) in mp18 bzw. mp19 und Verwendung von Standardverfahren im Rahmen einer Oligonukleotid-vermittelten gerichteten Mutagenese ("site-directed mutagenesis") für die Einführung der NcoI Schnittstelle.

#### 31.2. Konstruktion von pCIB301, einem Plasmid mit einem chimären Gen, das ein deletiertes *Bt* Protoxingen (607 Deletion) unter der Kontrolle eines rbc-gX Promotors enthält

Nach der Schnittstelle-spezifischen Mutagenese wird doppelsträngige, replikative (ds rf) DNA aus dem M13 Klon isoliert und anschliessend mit HindIII und EcoRI verdaut. Das HindIII-EcoRI Fragment, das den rbc-gX Promotor enthält, wird zusammen mit dem HindIII und EcoRI verdauten Plasmid pUC19 verknüpft. Mit dem Verknüpfungsgemisch ("ligation mix") wird dann *E. coli* Stamm HB101 transformiert.

Aus Ampicillin-selektionierten Transformanten wird Plasmid DNA isoliert und mit HindIII verdaut. Die Enden der resultierenden Moleküle werden mit glatten Enden versehen, indem sie mit der Klenow-Untereinheit der DNA Polymerase I behandelt werden. Anschliessend werden an diese glatten Enden SalI Linker angehängt. Das resultierende lineare Molekül wird mit SalI und NcoI verdaut und über ein Gel gereinigt.

In einer Dreifachverknüpfung ("three-part ligation") wird das Gelgereinigte SalI-NcoI Fragment mit einem Gel-gereinigten BamHI-SalI Fragment des Plasmids pCIB770, einem Replikon, das einen weiten Wirtsbereich besitzt und als ein *Agrobacterium* Ti-plasmid Klonierungsvektor verwendet wird (Rothstein et al, 1987), sowie mit einem Gel-gereinigten Ncol-BamHl Fragment, das das verkürzte, 607 Aminosäuren kodierende *Bt* Toxingen enthält, verknüpft. Das Verknüpfungsgemisch ("ligation mix") wird in *E. coli* Stamm HB101 transformiert.

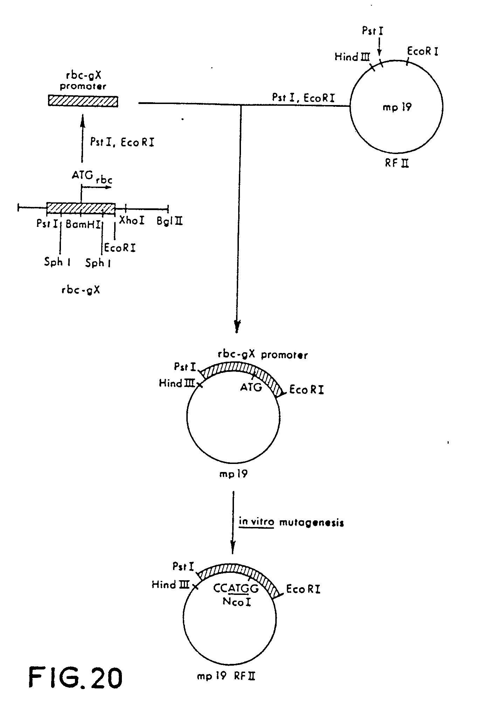

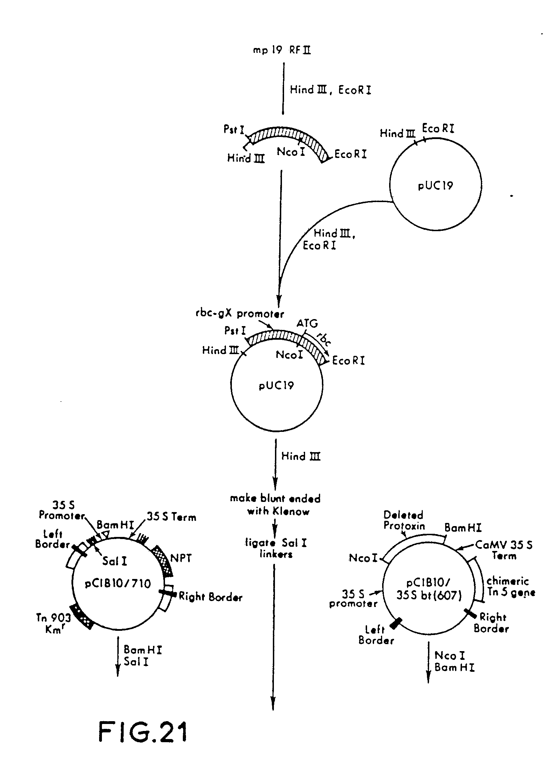

Das resultierende Plasmid pCIB1301, das in den Fig. 20, 21 und 22 graphisch dargestellt ist, wird auf Kanamycin selektioniert.

31.3. Konstruktion von pCIB1302, einem Plasmid mit einem chimären Gen, das ein deletiertes *Bt* Protoxingen (607 Deletion) unter der Kontrolle eines rbc-gY Promotors enthält.

Nach der erfolgen Mutagenese wird doppelsträngige, replikative (ds rf) DNA aus den M13 Klonen isoliert und anschliessend mit Xbal-Ncol verdaut. Das ca. 1.97 kBp umfassende Ncol-BamHl Fragment, welches das deletierte Gen enthält, wird dann zusammen mit dem Xbal-Ncol rbc-gY Promotorfragment in einer Dreifachverknüpfung in das Xbal-BamHl geschnittene Plasmid pCIB10/710 eingespleisst.

Das resultierende Plasmid pCIB1302, dessen Struktur in Fig. 23 wiedergegeben ist, wird auf Kanamycin selektioniert.

Literaturverzeichnis

An, G., Watson, B.D., Stachel, S., Gordon, M.P., Nester E.W., EMBO J. **4**, 277, 1985

Ang, B.J., Nickerson, K.W., Appl. Environ. Microbiol. **36**, 625, 1978

Barton, K.A., Chilton, M.-D., in: Wu, R., Grossmann, L., Moldave, K., Methods in Enzymology **101**, 527, 1983

Beasley, Ting, Am. J. Bot. **60**, 130, 1973

Bevan, M., Barnes, W.M., Chilton, M.-D., Nucl. Acids Res. **11**, 369, 1983

Bevan, M.W., Flavell, R.B., Chilton, M.-D., Nature **304**, 184, 1983

Bevan, M., Nucl. Acids Res. **12**, 8711, 1984

Bolivar, F., Rodriguez, R.L. Greene, P.J., Betlach, M.C., Heyneker, H.L., Boyer, H.W., Crosa, J.H., Falkow, S., Gene **2**, 95, 1977

Bradford, M., Anal. Biochem. **72**, 248, 1976

Caplan, A., Herrera-Estrella, L., Inzé, D., van Haute, E., van Montagu, M., Schell, J., Zambryski, P., Science **222**, 815, 1983

Chaleff, R.S., Ray, T.B., Science **223**, 1148, 1984

Cheng et al., Plant Sci. Lett. **19**, 91, 1980

Chilton, M.-D. et al., Proc. Natl. Acad. Sci., USA **77**, 7347, 1974

Chilton, M.-D., Farrand, S.K., Levin, R., Nester, E.W., Genetics **83**, 609, 1976

Chilton, M.-D., Bevan, M.W., Yadav, N., Matzke, A.J.M., Byrne, M., Grula, M., Barton, K., Vanderleyden, J., de Framond, A., Barnes, W.M., Stadler Genetics Symposia Series **13**, 39, 1981

Chilton, M.-D., in: the Role of Plant Biotechnology in Plant Breeding, Bericht des 1984 Plant Breeding Research Forum, 21.-23. August 1984, 177, 1985

Clark M.F. et al., Methods in Enzymology **118**, 742, 1986

Comai, L., Schilling-Cordaro, C., Mergia, A., Houck, C.M., Plasmid **10**, 21, 1983

Covey, S.N., Lomonossoff, G.P., Hull, R., Nucl. Acids Res. **9**, 6735, 1981

Deacon, J.W., Aspects of Microbiology **7**, ed. Cole et al., American Society of Microbiology, 1983

DeBlock et al., EMBO Journal **3**, 1681, 1984

van den Elzen, P.J.M., Townsend, J., Lee, K.Y., Bedbrook, J.R., Plant. Mol. Biol. **5**, 299, 1985

Fillatti, J. et al., Mol. Gen. Genet **206**, 192, 1987

Fraley, R.T., Rogers, S.G., Horsch, R.B., Sanders, P.R., Flick, J.S., Adams, S.P., Bittner, M.L., Brand, L.A., Fink, C.L., Fry, J.S., Galluppi, G.R., Goldberg, S.B., Hoffmann, N.L., Woo, S.C., Proc. Natl. Acad. Sci. USA **80**, 4803, 1983

Fraley, R.T., Rogers, S.G., Horsch, R.B., Eichholtz, D.A., Flick, J.S., Fink, C.L., Hoffmann, N.L., Sanders, P.R., Biotechnology **3**, 629, 1985

de Framond, A.J., Barton, K.A., Chilton, M.-D., Biotechnology **1**, 262, 1983

Gamborg, O.L., Miller, R.A., Ojima, K., Exptl. Cell Res. **50**, 151, 1968

Geiser, M., Schweitzer, S., Grimm, C., Gene **48**, 109, 1986

Gritz, L., Davies, J., Gene **25**, 179, 1983

Hernalsteens, J.P., van Vliet, F., de Beuckeleer, M., Depicker, A., Engler, G., Lemmers, M., Holsters, M., van Montagu, M., Schell, J., Nature **287**, 654, 1980

Hinnen, A., Hicks, J.B., Fink, G.R., Proc. Natl. Acad. Sci. USA **75**, 1929, 1978

Hoekema, A., Hirsch, P.R., Hooykas, P.J.J., Schilperoort, R.A., Nature **303**, 179, 1983

Hohn, T., Richards, K., Lebeurier, G., in: Gene cloning in organisms other than E.coli, Current Topics in Microbiology and Immunology **96**, 193, 1982

Holsters, M., de Waele, D., Depicker, A., Messens, E., van Montagu, M., Schell, J., Mol. Gen. Genet. **163**, 181, 1978

Klausner, A., Biotechnology **2**, 408, 1984

Klee, H.J., Yanofsky, M.F., Nester, E.W., Biotechnology **3**, 637, 1985

Klessig et al., Plant Mol. Biol. Reporter. **1**, 12, 1983

Koper-Zwarthoff, E.C., Lockard, R.E., Alzner-Deweerd, B., RajBhandary, U.L., Bol, J.F., Proc. Natl. Acad. Sci. USA **74**, 5504, 1977

Maniatis, T., Fritsch, E.F., Sambrook, J., Molecular cloning, a laboratory manual, Cold Spring Harbor Laboratory, Cold Spring Harbor, 1982

Matzke, A.J.M., Chilton, M.D., J. Mol. Appl. Genet. **1**, 39, 1981

Messing, J., in: Wu, R., Grossmann, L., Moldave, K., Methods in Enzymology **101**, 20, 1983

Miller, L.K., Lingg, A.J., Bulla, L.A., Science **219**, 715, 1983

Morelli, G., Nagy, F., Fraley, R.T., Rogers, S.G., Chua, N.H., Nature **315**, 200, 1985

Murashige, T., Skoog, F., Physiol. Plant. **15**, 473, 1962

Nagy, I.J, Maliga, P., Z. Pflanzenphysiol. **78**, 453, 1976

Newbury, Possingham, Plant Physiol. **60**, 543, 1977

Norrander, J., Kempe, T., Messing, J., Gene **26**, 101, 1983

Odell et al., 1985

Oka, A., Sugisaki, H., Takanami, M., J. Mol. Biol. **147**, 217, 1981

Okayama, Berg, Mol. Cell. Biol. **2**, 161, 1982

Ooms, G., Regensburg-Tuink, T.J.G., Hofker, M.H., Hoekema, A., Hooykaas, P.J.J., Schilperoort, R.A., Plant Molecular Biology **1**, 265, 1982

Paszkowski, J., Shillito, R.D., Saul, M., Mandak, V., Hohn, T., Hohn, B., Potrykus, I., EMBO J. **3**, 2717, 1984

Rothstein, S.J., Lahners, K.N., Lotstein, R.J., Carozzi, N.B., Jayne, S.M., Rice, D.A., Gene **53**, 153, 1987

Sanger et al., 1977

Sekar, V., et al., Proc. Natl. Acad. Sci, USA **84**, 7036, 1987

Simon, R., Priefer, U., Pühler, A., in: Pühler, A. (Hrsg.), Molecular Genetics of the Bacteria-Plant Interaction, Springer Verlag, Berlin, 98, 1983

Velten, 3., Velten, L., Hain, R., Schell, J., EMBO J. **3**, 2723, 1984

Wang, K., Herrera-Estrella, L., van Montagu, M., Zambryski, P., Cell **38**, 455, 1984

White, Phytomorphology **11**, 19, 1961

Wong, H.C., Schnepf, H.E., Whiteley, H.R., J. Biol. Chem. **258**, 1960, 1983

Yadav, N.S., Vanderleyden, J., Bennett, D.R., Barnes, W.M., Chilton, M.D., Proc. Natl. Acad. Sci. USA **79**, 6322, 1982

Yanish-Perron, C., Vieira, J., Messing, J., Gene **33**, 103, 1985

Zambryski, P., Joos, H. Genetello, C., Leemans, J., van Montagu, M., Schell, J., EMBO J. **2**, 2143, 1983

Zoller, M.J., Smith, M., in: Wu, R., Grossmann, L., Moldave, K., Methods in Enzymology **100**, 468, 1983

**Patentansprüche**

1. Eine Baumwoll-Zelle, die ein chimäres Gen enthält, welches ein Polypeptid exprimiert, das im wesentlichen die Insektentoxizitätseigenschaften des *Bacillus thuringiensis* Kristallproteins aufweist.

2. Eine Zelle gemäss Anspruch 1, dadurch gekennzeichnet, dass es sich um Zellen von Baumwollpflanzen, ausgewählt aus der Gruppe bestehend aus *Gossypium hirsutum, Gossypium arboreum* oder *Gossypium barbadense* handelt.

3. Eine Zelle gemäss Anspruch 2, dadurch gekennzeichnet, dass es sich um Zellen von *Gossypium hirsutum* handelt.

4. Eine Zelle gemäss Anspruch 1, dadurch gekennzeichnet, dass es sich um Zellen der Baumwollvarietäten Acala SJ-2, Acala GC 510, Acala B-1644 oder Siokra handelt.

5. Eine Zelle gemäss Anspruch 1, dadurch gekennzeichnet, dass der Promotor, die 5'-nichttranslatierte Region und gegebenenfalls die 3'-nichttranslatierte Region des chimären Gens aus Pflanzen- oder Pflanzenvirusgenen stammen.

6. Eine Zelle gemäss Anspruch 5, dadurch gekennzeichnet, dass der Promotor, die 5'-nichttranslatierte Region und gegebenenfalls die 3'-nichttranslatierte Region des chimären Gens aus einem pflanzlichen Gen stammen, das die kleine Untereinheit der Ribulose-1,5-bisphosphatcarboxylase oder das Chlorophyll a/b-Bindungsprotein kodiert.

7. Eine Zelle gemäss Anspruch 5, dadurch gekennzeichnet, dass der Promotor, die 5'-nichttranslatierte Region und gegebenenfalls die 3'-nichttranslatierte Region aus einem Gen eines DNA-Pflanzenvirus stammen.

8. Eine Zelle gemäss Anspruch 7, dadurch gekennzeichnet, dass es sich bei besagtem Pflanzenvirus um Cauliflower Mosaik Virus handelt.

9. Eine Zelle gemäss Anspruch 8, dadurch gekennzeichnet, dass es sich beim Cauliflower Mosaik Virus Promotor um den 35S Promotor des CaMV Gens VI handelt.

10. Eine Zelle gemäss Anspruch 1, dadurch gekennzeichnet, dass der Promotor, die 5'-nichttranslatierte Region und gegebenenfalls die 3'-nichttranslatierte Region des chimären Gens aus DNA-Sequenzen stammen, die in den Plasmiden von *Agrobacterium* vorliegen und die zu einer Expression in Pflanzen führen.

11. Eine Zelle gemäss Anspruch 10, dadurch gekennzeichnet, dass der Promotor aus dem Ti-Plasmid von *Agrobacterium tumefaciens* stammt.

12. Eine Zelle gemäss Anspruch 10, dadurch gekennzeichnet, dass besagte DNA Sequenzen aus einem Gen stammen, das Octopinsynthase kodiert.

13. Eine Zelle gemäss Anspruch 10, dadurch gekennzeichnet, dass besagte DNA Sequenzen aus einem Gen stammen, das Nopalinsynthase kodiert.

14. Eine Zelle gemäss Anspruch 1, dadurch gekennzeichnet, dass das Polypeptid ein Mr von etwa 130 000 bis etwa 140 000 hat oder insektizide Fragmente davon umfasst.

15. Eine Zelle gemäss Anspruch 14, dadurch gekennzeichnet, dass das Polypeptid mit einem anderen Molekül verknüpft ist.

16. Eine Zelle gemäss Anspruch 1, dadurch gekennzeichnet, das das chimäre Gen in wesentlichen Teilen zu der Nukleotidsequenz komplementär ist, die das kristalline δ-Endotoxin Protein von *B. thuringiensis* kodiert.

17. Eine Zelle gemäss Anspruch 1, dadurch gekennzeichnet, dass das chimäre Gen in der Lage ist, mit der kodierenden Sequenz desjenigen Gens, welches das kristalline δ-Endotoxin Protein von *B. thuringiensis* kodiert, zu hybridisieren.

18. Eine Zelle gemäss Anspruch 14, dadurch gekennzeichnet, dass das Polypeptid im wesentlichen die selben immunologischen Eigenschaften hat wie das kristalline Protein von *B. thuringiensis*.

19. Eine Zelle gemäss einem der Ansprüche 16, 17 oder 18, dadurch gekennzeichnet, dass es sich bei besagtem *B. thuringiensis* um eine Unterart handelt, ausgewählt aus der Gruppe bestehend aus *Bt* var. *berliner*, *Bt* var. *alesti*, *Bt* var. *tolworthi*, *Bt* var. *sotto*, *Bt* var. *dendrolimus*, *Bt* var. *tenebrionis*, *Bt* var. *san diego* und *Bt* var. *aizawai*.

20. Eine Zelle gemäss Anspruch 19, dadurch gekennzeichnet, dass es sich um die *B. thuringiensis* Varietät *kurstaki* HD1 handelt.

21. Eine Zelle gemäss Anspruch 20, dadurch gekennzeichnet, dass das chimäre Gen ein Polypeptid exprimiert, welches die folgende Aminosäuresequenz aufweist:

```
Met Asp Asn Asn Pro Asn Ile Asn Glu Cys          10
Ile Pro Tyr Asn Cys Leu Ser Asn Pro Glu          20
Val Glu Val Leu Gly Gly Glu Arg Ile Glu          30
Thr Gly Tyr Thr Pro Ile Asp Ile Ser Leu          40
Ser Leu Thr Gln Phe Leu Leu Ser Glu Phe          50
Val Pro Gly Ala Gly Phe Val Leu Gly Leu          60
Val Asp Ile Ile Trp Gly Ile Phe Gly Pro          70
Ser Gln Trp Asp Ala Phe Leu Val Gln Ile          80
Glu Gln Leu Ile Asn Gln Arg Ile Glu Glu          90
Phe Ala Arg Asn Gln Ala Ile Ser Arg Leu          100
Glu Gly Leu Ser Asn Leu Tyr Gln Ile Tyr          110
Ala Glu Ser Phe Arg Glu Trp Glu Ala Asp          120
Pro Thr Asn Pro Ala Leu Arg Glu Glu Met          130
Arg Ile Gln Phe Asn Asp Met Asn Ser Ala          140
Leu Thr Thr Ala Ile Pro Leu Phe Ala Val          150
Gln Asn Tyr Gln Val Pro Leu Leu Ser Val          160
Tyr Val Gln Ala Ala Asn Leu His Leu Ser          170
Val Leu Arg Asp Val Ser Val Phe Gly Gln          180
Arg Trp Gly Phe Asp Ala Ala Thr Ile Asn          190
```

```
Ser Arg Tyr Asn Asp Leu Thr Arg Leu Ile        200
Gly Asn Tyr Thr Asp His Ala Val Arg Trp         210
Tyr Asn Thr Gly Leu Glu Arg Val Trp Gly         220
Pro Asp Ser Arg Asp Trp Ile Arg Tyr Asn         230
Gln Phe Arg Arg Glu Leu Thr Leu Thr Val         240
Leu Asp Ile Val Ser Leu Phe Pro Asn Tyr         250
Asp Ser Arg Thr Tyr Pro Ile Arg Thr Val         260
Ser Gln Leu Thr Arg Glu Ile Tyr Thr Asn         270
Pro Val Leu Glu Asn Phe Asp Gly Ser Phe         280
Arg Gly Ser Ala Gln Gly Ile Glu Gly Ser         290
Ile Arg Ser Pro His Leu Met Asp Ile Leu         300
Asn Ser Ile Thr Ile Tyr Thr Asp Ala His         310
Arg Gly Glu Tyr Tyr Trp Ser Gly His Gln         320
Ile Met Ala Ser Pro Val Gly Phe Ser Gly         330
Pro Glu Phe Thr Phe Pro Leu Tyr Gly Thr         340
Met Gly Asn Ala Ala Pro Gln Gln Arg Ile         350
Val Ala Gln Leu Gly Gln Gly Val Tyr Arg         360
Thr Leu Ser Ser Thr Leu Tyr Arg Arg Pro         370
Phe Asn Ile Gly Ile Asn Asn Gln Gln Leu         380
Ser Val Leu Asp Gly Thr Glu Phe Ala Tyr         390
Gly Thr Ser Ser Asn Leu Pro Ser Ala Val         400
Tyr Arg Lys Ser Gly Thr Val Asp Ser Leu         410
Asp Glu Ile Pro Pro Gln Asn Asn Asn Val         420
Pro Pro Arg Gln Gly Phe Ser His Arg Leu         430
Ser His Val Ser Met Phe Arg Ser Gly Phe         440
Ser Asn Ser Ser Val Ser Ile Ile Arg Ala         450
Pro Met Phe Ser Trp Ile His Arg Ser Ala         460
Glu Phe Asn Asn Ile Ile Pro Ser Ser Gln         470
Ile Thr Gln Ile Pro Leu Thr Lys Ser Thr    .    480
Asn Leu Gly Ser Gly Thr Ser Val Val Lys         490
Gly Pro Gly Phe Thr Gly Gly Asp Ile Leu         500
Arg Arg Thr Ser Pro Gly Gln Ile Ser Thr         510
Leu Arg Val Asn Ile Thr Ala Pro Leu Ser         520
Gln Arg Tyr Arg Val Arg Ile Arg Tyr Ala         530
Ser Thr Thr Asn Leu Gln Phe His Thr Ser         540
Ile Asp Gly Arg Pro Ile Asn Gln Gly Asn         550
Phe Ser Ala Thr Met Ser Ser Gly Ser Asn         560
```

Leu Gln Ser Gly Ser Phe Arg Thr Val Gly                570

Phe Thr Thr Pro Phe Asn Phe Ser Asn Gly                580

Ser Ser Val Phe Thr Leu Ser Ala His Val                590

Phe Asn Ser Gly Asn Glu Val Tyr Ile Asp                600

Arg Ile Glu Phe Val Pro Ala Glu Val Thr                610

Phe Glu Ala Glu Tyr Asp Leu Glu Arg Ala                620

Gln Lys Ala Val Asn Glu Leu Phe Thr Ser                630

Ser Asn Gln Ile Gly Leu Lys Thr Asp Val                640

Thr Asp Tyr His Ile Asp Gln Val Ser Asn                650

Leu Val Glu Cys Leu Ser Asp Glu Phe Cys                660

Leu Asp Glu Lys Lys Glu Leu Ser Glu Lys                670

Val Lys His Ala Lys Arg Leu Ser Asp Glu                680

Arg Asn Leu Leu Gln Asp Pro Asn Phe Arg                690

Gly Ile Asn Arg Gln Leu Asp Arg Gly Trp                700

Arg Gly Ser Thr Asp Ile Thr Ile Gln Gly                710

Gly Asp Asp Val Phe Lys Glu Asn Tyr Val                720

Thr Leu Leu Gly Thr Phe Asp Glu Cys Tyr                730

Pro Thr Tyr Leu Tyr Gln Lys Ile Asp Glu                740

Ser Lys Leu Lys Ala Tyr Thr Arg Tyr Gln                750

Leu Arg Gly Tyr Ile Glu Asp Ser Gln Asp                760

Leu Glu Ile Tyr Leu Ile Arg Tyr Asn Ala                770

Lys His Glu Thr Val Asn Val Pro Gly Thr                780

Gly Ser Leu Trp Pro Leu Ser Ala Pro Ser                790

Pro Ile Gly Lys Cys Ala His His Ser His                800

His Phe Ser Leu Asp Ile Asp Val Gly Cys                810

Thr Asp Leu Asn Glu Asp Leu Gly Val Trp                820

Val Ile Phe Lys Ile Lys Thr Gln Asp Gly                830

His Ala Arg Leu Gly Asn Leu Glu Phe Leu                840

Glu Glu Lys Pro Leu Val Gly Glu Ala Leu                850

Ala Arg Val Lys Arg Ala Glu Lys Lys Trp                860

Arg Asp Lys Arg Glu Lys Leu Glu Trp Glu                870

Thr Asn Ile Val Tyr Lys Glu Ala Lys Glu                880

Ser Val Asp Ala Leu Phe Val Asn Ser Gln                890

Tyr Asp Arg Leu Gln Ala Asp Thr Asn Ile                900

Ala Met Ile His Ala Ala Asp Lys Arg Val                910

His Ser Ile Arg Glu Ala Tyr Leu Pro Glu                920

Leu Ser Val Ile Pro Gly Val Asn Ala Ala                930

```
Ile Phe Glu Glu Leu Glu Gly Arg Ile Phe        940

Thr Ala Phe Ser Leu Tyr Asp Ala Arg Asn        950

Val Ile Lys Asn Gly Asp Phe Asn Asn Gly        960

Leu Ser Cys Trp Asn Val Lys Gly His Val        970

Asp Val Glu Glu Gln Asn Asn His Arg Ser        980

Val Leu Val Val Pro Glu Trp Glu Ala Glu        990

Val Ser Gln Glu Val Arg Val Cys Pro Gly        1000

Arg Gly Tyr Ile Leu Arg Val Thr Ala Tyr        1010

Lys Glu Gly Tyr Gly Glu Gly Cys Val Thr        1020

Ile His Glu Ile Glu Asn Asn Thr Asp Glu        1030

Leu Lys Phe Ser Asn Cys Val Glu Glu Glu        1040

Val Tyr Pro Asn Asn Thr Val Thr Cys Asn        1050

Asp Tyr Thr Ala Thr Gln Glu Glu Tyr Glu        1060

Gly Thr Tyr Thr Ser Arg Asn Arg Gly Tyr        1070

Asp Gly Ala Tyr Glu Ser Asn Ser Ser Val        1080

Pro Ala Asp Tyr Ala Ser Ala Tyr Glu Glu        1090

Lys Ala Tyr Thr Asp Gly Arg Arg Asp Asn        1100

Pro Cys Glu Ser Asn Arg Gly Tyr Gly Asp        1110

Tyr Thr Pro Leu Pro Ala Gly Tyr Val Thr        1120

Lys Glu Leu Glu Tyr Phe Pro Glu Thr Asp        1130

Lys Val Trp Ile Glu Ile Gly Glu Thr Glu        1140

Gly Thr Phe Ile Val Asp Ser Val Glu Leu        1150

Leu Leu Met Glu Glu End                        1156
```

22. Eine Zelle gemäss Anspruch 1, dadurch gekennzeichnet, dass die kodierende Region folgende DNA Sequenz aufweist:

```
         10         20         30         40         50         60
GTTAACACCC TGGGTCAAAA ATTGATATTT AGTAAAATTA GTTGCACTTT GTGCATTTTT

         70         80         90        100        110        120
TCATAAGATG AGTCATATGT TTTAAATTGT AGTAATGAAA AACAGTATTA TATCATAATG

        130        140        150        160        170        180
AATTGGTATC TTAATAAAAG AGATGGAGGT AACTTATGGA TAACAATCCG AACATCAATG

        190        200        210        220        230        240
AATGCATTCC TTATAATTGT TTAAGTAACC CTGAAGTAGA AGTATTAGGT GGAGAAAGAA

        250        260        270        280        290        300
TAGAAACTGG TTACACCCCA ATCGATATTT CCTTGTCGCT AACGCAATTT CTTTTGAGTG
```

```
        310        320        330        340        350        360
AATTTGTTCC CGGTGCTGGA TTTGTGTTAG GACTAGTTGA TATAATATGG GGAATTTTTG

        370        380        390        400        410        420
GTCCCTCTCA ATGGGACGCA TTTCTTGTAC AAATTGAACA GTTAATTAAC CAAAGAATAG

        430        440        450        460        470        480
AAGAATTCGC TAGGAACCAA GCCATTTCTA GATTAGAAGG ACTAAGCAAT CTTTATCAAA

        490        500        510        520        530        540
TTTACGCAGA ATCTTTTAGA GAGTGGGAAG CAGATCCTAC TAATCCAGCA TTAAGAGAAG

        550        560        570        580        590        600
AGATGCGTAT TCAATTCAAT GACATGAACA GTGCCCTTAC AACCGCTATT CCTCTTTTTG

        610        620        630        640        650        660
CAGTTCAAAA TTATCAAGTT CCTCTTTTAT CAGTATATGT TCAAGCTGCA AATTTACATT

        670        680        690        700        710        720
TATCAGTTTT GAGAGATGTT TCAGTGTTTG GACAAAGGTG GGGATTTGAT GCCGCGACTA

        730        740        750        760        770        780
TCAATAGTCG TTATAATGAT TTAACTAGGC TTATTGGCAA CTATACAGAT CATGCTGTAC

        790        800        810        820        830        840
GCTGGTACAA TACGGGATTA GAGCGTGTAT GGGGACCGGA TTCTAGAGAT TGGATAAGAT

        850        860        870        880        890        900
ATAATCAATT TAGAAGAGAA TTAACACTAA CTGTATTAGA TATCGTTTCT CTATTTCCGA

        910        920        930        940        950        960
ACTATGATAG TAGAACGTAT CCAATTCGAA CAGTTTCCCA ATTAACAAGA GAAATTTATA

        970        980        990       1000       1010       1020
CAAACCCAGT ATTAGAAAAT TTTGATGGTA GTTTTCGAGG CTCGGCTCAG GGCATAGAAG

       1030       1040       1050       1060       1070       1080
GAAGTATTAG GAGTCCACAT TTGATGGATA TACTTAACAG TATAACCATC TATACGGATG

       1090       1100       1110       1120       1130       1140
CTCATAGAGG AGAATATTAT TGGTCAGGGC ATCAAATAAT GGCTTCTCCT GTAGGGTTTT

       1150       1160       1170       1180       1190       1200
CGGGGCCAGA ATTCACTTTT CCGCTATATG GAACTATGGG AAATGCAGCT CCACAACAAC

       1210       1220       1230       1240       1250       1260
GTATTGTTGC TCAACTAGGT CAGGGCGTGT ATAGAACATT ATCGTCCACT TTATATAGAA

       1270       1280       1290       1300       1310       1320
GACCTTTTAA TATAGGGATA AATAATCAAC AACTATCTGT TCTTGACGGG ACAGAATTTG

       1330       1340       1350       1360       1370       1380
CTTATGGAAC CTCCTCAAAT TTGCCATCCG CTGTATACAG AAAAAGCGGA ACGGTAGATT
```

```
          1390       1400       1410       1420       1430       1440
     CGCTGGATGA AATACCGCCA CAGAATAACA ACGTGCCACC TAGGCAAGGA TTTAGTCATC

          1450       1460       1470       1480       1490       1500
     GATTAAGCCA TGTTTCAATG TTTCGTTCAG GCTTTAGTAA TAGTAGTGTA AGTATAATAA

          1510       1520       1530       1540       1550       1560
     GAGCTCCTAT GTTCTCTTGG ATACATCGTA GTGCTGAATT TAATAATATA ATTCCTTCAT

          1570       1580       1590       1600       1610       1620
     CACAAATTAC ACAAATACCT TTAACAAAAT CTACTAATCT TGGCTCTGGA ACTTCTGTCG

          1630       1640       1650       1660       1670       1680
     TTAAAGGACC AGGATTTACA GGAGGAGATA TTCTTCGAAG AACTTCACCT GGCCAGATTT

          1690       1700       1710       1720       1730       1740
     CAACCTTAAG AGTAAATATT ACTGCACCAT TATCACAAAG ATATCGGGTA AGAATTCGCT

          1750       1760       1770       1780       1790       1800
     ACGCTTCTAC CACAAATTTA CAATTCCATA CATCAATTGA CGGAAGACCT ATTAATCAGG

          1810       1820       1830       1840       1850       1860
     GGAATTTTTC AGCAACTATG AGTAGTGGGA GTAATTTACA GTCCGGAAGC TTTAGGACTG

          1870       1880       1890       1900       1910       1920
     TAGGTTTTAC TACTCCGTTT AACTTTTCAA ATGGATCAAG TGTATTTACG TTAAGTGCTC

          1930       1940       1950       1960       1970       1980
     ATGTCTTCAA TTCAGGCAAT GAAGTTTATA TAGATCGAAT TGAATTTGTT CCGGCAGAAG

          1990       2000       2010       2020       2030       2040
     TAACCTTTGA GGCAGAATAT GATTTAGAAA GAGCACAAAA GGCGGTGAAT GAGCTGTTTA

          2050       2060       2070       2080       2090       2100
     CTTCTTCCAA TCAAATCGGG TTAAAAACAG ATGTGACGGA TTATCATATT GATCAAGTAT

          2110       2120       2130       2140       2150       2160
     CCAATTTAGT TGAGTGTTTA TCTGATGAAT TTTGTCTGGA TGAAAAAAAA GAATTGTCCG

          2170       2180       2190       2200       2210       2220
     AGAAAGTCAA ACATGCGAAG CGACTTAGTG ATGAGCGGAA TTTACTTCAA GATCCAAACT

          2230       2240       2250       2260       2270       2280
     TTAGAGGGAT CAATAGACAA CTAGACCGTG GCTGGAGAGG AAGTACGGAT ATTACCATCC

          2290       2300       2310       2320       2330       2340
     AAGGAGGCGA TGACGTATTC AAAGAGAATT ACGTTACGCT ATTGGGTACC TTTGATGAGT

          2350       2360       2370       2380       2390       2400
     GCTATCCAAC GTATTTATAT CAAAAAATAG ATGAGTCGAA ATTAAAAGCC TATACCCGTT

          2410       2420       2430       2440       2450       2460
     ACCAATTAAG AGGGTATATC GAAGATAGTC AAGACTTAGA AATCTATTTA ATTCGCTACA

          2470       2480       2490       2500       2510       2520
     ATGCCAAACA CGAAACAGTA AATGTGCCAG GTACGGGTTC CTTATGGCCG CTTTCAGCCC
```

```
       2530       2540       2550       2560       2570       2580
CAAGTCCAAT CGGAAAATGT GCCCATCATT CCCATCATTT CTCCTTGGAC ATTGATGTTG

       2590       2600       2610       2620       2630       2640
GATGTACAGA CTTAAATGAG GACTTAGGTG TATGGGTGAT ATTCAAGATT AAGACGCAAG

       2650       2660       2670       2680       2690       2700
ATGGCCATGC AAGACTAGGA AATCTAGAAT TTCTCGAAGA GAAACCATTA GTAGGAGAAG

       2710       2720       2730       2740       2750       2760
CACTAGCTCG TGTGAAAAGA GCGGAGAAAA AATGGAGAGA CAAACGTGAA AAATTGGAAT

       2770       2780       2790       2800       2810       2820
GGGAAACAAA TATTGTTTAT AAAGAGGCAA AAGAATCTGT AGATGCTTTA TTTGTAAACT

       2830       2840       2850       2860       2870       2880
CTCAATATGA TAGATTACAA GCGGATACCA ACATCGCGAT GATTCATGCG GCAGATAAAC

       2890       2900       2910       2920       2930       2940
GCGTTCATAG CATTCGAGAA GCTTATCTGC CTGAGCTGTC TGTGATTCCG GGTGTCAATG

       2950       2960       2970       2980       2990       3000
CGGCTATTTT TGAAGAATTA GAAGGGCGTA TTTTCACTGC ATTCTCCCTA TATGATGCGA

       3010       3020       3030       3040       3050       3060
GAAATGTCAT TAAAAATGGT GATTTTAATA ATGGCTTATC CTGCTGGAAC GTGAAAGGGC

       3070       3080       3090       3100       3110       3120
ATGTAGATGT AGAAGAACAA AACAACCACC GTTCGGTCCT TGTTGTTCCG GAATGGGAAG

       3130       3140       3150       3160       3170       3180
CAGAAGTGTC ACAAGAAGTT CGTGTCTGTC CGGGTCGTGG CTATATCCTT CGTGTCACAG

       3190       3200       3210       3220       3230       3240
CGTACAAGGA GGGATATGGA GAAGGTTGCG TAACCATTCA TGAGATCGAG AACAATACAG

       3250       3260       3270       3280       3290       3300
ACGAACTGAA GTTTAGCAAC TGTGTAGAAG AGGAAGTATA TCCAAACAAC ACGGTAACGT

       3310       3320       3330       3340       3350       3360
GTAATGATTA TACTGCGACT CAAGAAGAAT ATGAGGGTAC GTACACTTCT CGTAATCGAG

       3370       3380       3390       3400       3410       3420
GATATGACGG AGCCTATGAA AGCAATTCTT CTGTACCAGC TGATTATGCA TCAGCCTATG

       3430       3440       3450       3460       3470       3480
AAGAAAAAGC ATATACAGAT GGACGAAGAG ACAATCCTTG TGAATCTAAC AGAGGATATG

       3490       3500       3510       3520       3530       3540
GGGATTACAC ACCACTACCA GCTGGCTATG TGACAAAAGA ATTAGAGTAC TTCCCAGAAA

       3550       3560       3570       3580       3590       3600
CCGATAAGGT ATGGATTGAG ATCGGAGAAA CGGAAGGAAC ATTCATCGTG GACAGCGTGG
```

```
        3610        3620        3630        3640        3650        3660
AATTACTTCT TATGGAGGAA TAATATATGC TTTATAATGT AAGGTGTGCA AATAAAGAAT


        3670        3680        3690        3700        3710        3720
GATTACTGAC TTGTATTGAC AGATAAATAA GGAAATTTTT ATATGAATAA AAAACGGGCA


        3730        3740        3750        3760        3770        3780
TCACTCTTAA AAGAATGATG TCCGTTTTTT GTATGATTTA ACGAGTGATA TTTAAATGTT


        3790        3800        3810        3820        3830        3840
TTTTTTGCGA AGGCTTTACT TAACGGGGTA CCGCCACATG CCCATCAACT TAAGAATTTG


        3850        3860        3870        3880        3890        3900
CACTACCCCC AAGTGTCAAA AAACGTTATT CTTTCTAAAA AGCTAGCTAG AAAGGATGAC


        3910        3920        ·3930        3940        3950        3960
ATTTTTATG AATCTTTCAA TTCAAGATGA ATTACAACTA TTTTCTGAAG AGCTGTATCG


        3970        3980        3990        4000        4010        4020
TCATTTAACC CCTTCTCTTT TGGAAGAACT CGCTAAAGAA TTAGGTTTTG TAAAAAGAAA


        4030        4040        4050        4060        4070        4080
ACGAAAGTTT TCAGGAAATG AATTAGCTAC CATATGTATC TGGGGCAGTC AACGTACAGC


        4090        4100        4110        4120        4130        4140
GAGTGATTCT CTCGTTCGAC TATGCAGTCA ATTACACGCC GCCACAGCAC TCTTATGAGT


        4150        4160        4170        4180        4190        4200
CCAGAAGGAC TCAATAAACG CTTTGATAAA AAAGCGGTTG AATTTTTGAA ATATATTTTT


        4210        4220        4230        4240        4250        4260
TCTGCATTAT GGAAAAGTAA ACTTTGTAAA ACATCAGCCA TTTCAAGTGC AGCACTCACG


        4270        4280        4290        4300        4310        4320
TATTTTCAAC GAATCCGTAT TTTAGATGCG ACGATTTTCC AAGTACCGAA ACATTTAGCA


        4330        4340        4350        4360
CATGTATATC CTGGGTCAGG TGGTTGTGCA CAAACTGCAG
```

23. Eine Zelle gemäss Anspruch 20, dadurch gekennzeichnet, dass das chimäre Gen ein insektizides Polypeptid exprimiert, das der in Anspruch 21 wiedergegebenen Aminosäuresequenz im wesentlichen homolog ist.

24. Eine Zelle gemäss Anspruch 20, dadurch gekennzeichnet, dass das chimäre Gen der in Anspruch 22 wiedergegebenen DNA-Sequenz im wesentlichen homolog ist.

25. Eine Zellkultur bestehend aus Baumwollzellen gemäss einem der Ansprüche 1 bis 4.

26. Eine Zellkultur gemäss Anspruch 25, dadurch gekennzeichnet, dass es sich bei besagten Baumwollzellen um Zellen von *Gossypium hirsutum, Gossypium arboreum* und *Gossypium barbadense* handelt.

27. Eine Zellkultur gemäss Anspruch 5, dadurch gekennzeichnet, dass es sich um Zellen von *Gossypium hirsutum* handelt.

28. Eine Zellkultur gemäss einem der Ansprüche 25 bis 27, dadurch gekennzeichnet, dass es sich bei den Zellen um Protoplasten handelt.

29. Eine Baumwollpflanze, die ein chimäres Gen enthält, welches ein Polypeptid exprimiert, das die wesentlichen Insektentoxizitätseigenschaften des *Bacillus thuringiensis* Kristallproteins aufweist.

30. Eine Baumwollpflanze gemäss Anspruch 29, die ein chimäres Gen enthält, welches ein Polypeptid exprimiert, das die wesentlichen Insektentoxizitätseigenschaften des *Bacillus thuringiensis* var. *kurstaki* HD1 Kristallproteins aufweist.

31. Eine Baumwollpflanze gemäss Anspruch 30, die ein chimäres Gen enthält, welches ein Polypeptid exprimiert, das im wesentlichen die Insektentoxizitätseigenschaften des *Bacillus thuringiensis* Kristallproteins aufweist und zwar in einer Menge, die ausreicht, die Pflanze unattraktiv und/oder toxisch für Lepidopterenlarven zu machen .

32. Eine Baumwollpflanze gemäss Anspruch 30, dadurch gekennzeichnet, dass es sich um eine Pflanze ausgewählt aus der Gruppe bestehend aus *Gossypium hirsutum, Gossypium arboreum* oder *Gossypium barbadense* handelt.

33. Eine Baumwollpflanze gemäss Anspruch 31, dadurch gekennzeichnet, dass es sich um *Gossypium hirsutum* handelt.

34. Vermehrungsgut einer transgenen Baumwollpflanze gemäss einem der Ansprüche 29 bis 33.

35. Vermehrungsgut gemäss Anspruch 34, ausgewählt aus der Gruppe bestehend aus Protoplasten, Zellen, Kalli, Gewebe, Embryonen, Organe, Samen, Pollen, Eizellen, Zygoten oder anderem, von einer transgenen Baumwollpflanze erhältlichen Vermehrungsgut.

36. Vermehrungsgut gemäss Anspruch 34, welches sich sexuell, asexuell, in-vitro oder in-vivo vermehren lässt.

37. Nachkommen einer transformierten Baumwollpflanze gemäss einem der Ansprüche 29 bis 33 oder Mutanten und Varianten davon, die noch die charakteristischen Eigenschaften des Ausgangsmaterials aufweisen, welche diese aufgrund der Transformation mit exogener DNA erhalten hat.

38. Ein Verfahren zur Herstellung eines transformierten embryogenen Baumwollkallus, das dadurch gekennzeichnet, ist, dass man:

(a) einen *Agrobacterium*-Vektor, der ein Gen enthält, welches Baumwollzellen eine Resistenz gegenüber dem Antibiotikum Hygromycin verleiht, mit einem Baumwollexplantat für einen Zeitraum in Kontakt bringt, der ausreicht, um das Gen in das Explantat zu transferieren;

(b) das transformierte Explantat in einem Kallus-Wachstumsmedium für einen Zeitraum von etwa 15 bis 200 Stunden bei einer Temperatur von 25° C bis 35° C und einem Hell-/Dunkel-Rhythmus von 16 Stunden Licht/8 Stunden Dunkelheit inkubiert, um auf diese Weise aus den Explantaten einen Kallus zu entwickeln;

(c) die inkubierten Explantate mit einem Kallus-Wachstumsmedium, das ein für *Agrobacterium* toxisch wirkendes Antibiotikum enthält, für einen Zeitraum in Kontakt bringt, der ausreicht die Agrobakterien abzutöten;

(d) den *Agrobacterium*-freien Kallus auf einem Kallus-Wachstumsmedium kultiviert;

(e) den resultierenden embryogenen Kallus mit dem Antibiotikum Hygromycin in Kontakt bringt und zwar in einer Konzentration, die ausreicht für eine Selektionierung eines Hygromycin-resistenten Kallus; und

(f) embryogenen Kallus selektioniert.

39. Verfahren gemäss Anspruch 38, dadurch gekennzeichnet, dass man in einem weiteren Verfahrensschritt den transformierten Kallus zur Keimung bringt und daraus ganze Pflänzchen entwickelt.

40. Verfahren gemäss Anspruch 38, dadurch gekennzeichnet, dass man den transformierten Kallus, bevor man ihn in Verfahrensschritt (c) mit dem Kallus-Wachstumsmedium in Kontakt bringt, abspült und zwar mit einem Kallus-Wachstumsmedium, das kein für *Agrobacterium* toxisches Antibiotikum enthält.

41. Verfahren gemäss Anspruch 38, dadurch gekennzeichnet, dass besagtes Explantat aus dem Hypokotyl oder den Keimblättern von Baumwollkeimlingen stammt oder aber, dass es sich um ein Gemisch davon handelt.

42. Verfahren gemäss Anspruch 38, dadurch gekennzeichnet, dass das Kallus-Wachstumsmedium ein Murashige und Skoog-Medium ist, das mit ca. 1 mg/Liter bis ca. 10 mg/Liter Naphthyl-1-essigsäure angereichert ist.

43. Verfahren gemäss Anspruch 38, dadurch gekennzeichnet, dass es sich bei dem für *Agrobacterium* toxischen Antibiotikum um Cefotaxim handelt.

44. Verfahren zur Transformation von Baumwollzellen, die einer Suspensionskultur in einem Kallus-Wachstumsmedium unterzogen werden, das, nachdem ein erster Wachstumszyklus im Rahmen der Suspensionskultur durchlaufen wurde, durch die folgenden Verfahrensmassnahmen gekennzeichnet ist:

(a) Rückgewinnung der Zellen sowie von jeglichem embryogenen Kallus aus dem Kallus-Wachstumsmedium;

(b) Resuspendierung der Zellen und des embryogenen Kallus in einem Kallus-Wachstumsmedium, das einen *Agrobacterium*-Vektor enthält, der ein Gen besitzt, welches Baumwollzellen eine Resistenz gegenüber dem Antibiotikum Hygromycin verleiht, wobei die Bedingungen für das Wachstum in Suspension für einen Zeitraum aufrechterhalten werden, der für die Transformation der suspendierten Zellen ausreicht;

(c) Rückgewinnung der suspendierten Zellen aus dem *Agrobacterium*-haltigen Kallus-Wachstumsmedium;

(d) Behandlung der transformierten Zellen und des embryogenen Kallus mit einem Antibiotikum in einer Konzentration, die ausreicht die Agrobakterien abzutöten;

(e) in Kontaktbringen der Zellen und des embryogenen Kallus mit dem Antibiotikum Hygromycin

56

zur Selektionierung der transformierten Zellen und des transformierten embryogenen Kallus;

(f) Filtrieren der Suspension zur Entfernung von embryogenem Kallus der eine Grösse von ca. 600 μm überschreitet.

45. Verfahren gemäss Anspruch 44, dadurch gekennzeichnet, dass die Verfahrensschritte (d) und (e) vor dem Verfahrensschritt (f) durchgeführt werden.

46. Verfahren gemäss Anspruch 44, dadurch gekennzeichnet, dass die Verfahrensschritte (d) und (e) nach dem Verfahrensschritt (f) durchgeführt werden.

47. Verfahren gemäss Anspruch 44, dadurch gekennzeichnet, dass der Verfahrensschritt (d) vor dem Verfahrensschritt (f) und dass der Verfahrensschritt (e) nach dem Verfahrensschritt (f) durchgeführt wird.

48. Verfahren gemäss Anspruch 44, dadurch gekennzeichnet, dass der Verfahrensschritt (e) vor dem Verfahrensschritt (f) und der Verfahrensschritt (d) nach dem Verfahrensschritt (f) durchgeführt wird.

49. Verfahren gemäss Anspruch 44, dadurch gekennzeichnet, dass es sich bei dem in Verfahrensschritt (d) verwendeten Antibiotikum um Cefotaxim handelt.

50. Verfahren gemäss Anspruch 44, dadurch gekennzeichnet, das der Wachstumszyklus im Rahmen der Suspensionskultur zwischen ca. 7 und 14 Tagen dauert.

51. Verfahren gemäss Anspruch 44, dadurch gekennzeichnet, dass in einem weiteren Verfahrensschritt die transformierten Baumwollzellen zu Pflänzchen entwickelt werden.

52. Baumwollpflanzen, die aufgrund einer Transformation eine Resistenz gegenüber dem Antibiotikum Hygromycin aufweisen.

53. Verfahren zum Schutz von Baumwollpflanzen vor Insektenfrass, dadurch gekennzeichnet, dass man innerhalb der die Pflanze aufbauenden Pflanzenzellen ein *Bt* Kristallprotein oder ein Protein, das im wesentlichen die Toxizitätseigenschaften des *Bt* Kristallproteins aufweist, in einer Menge exprimiert, die ausreicht, die Insektenlarven abzutöten oder aber diese unter Kontrolle zu halten.

54. Verfahren gemäss Anspruch 53, dadurch gekennzeichnet, dass es sich um Lepidopteren-, Coleopteren- oder um Dipteren-Larven handelt.

55. Verfahren gemäss Anspruch 54, dadurch gekennzeichnet, dass es sich um Lepidopteren-Larven handelt.

56. Verfahren zur Abtötung oder zur Kontrolle von Schadinsekten, dadurch gekennzeichnet, dass man sie mit Baumwollpflanzenzellen füttert, die chimäre Gene enthalten, welche eine insektizide Menge eines Toxins exprimieren, das im wesentlichen die Insektentoxizität des kristallinen Proteins von *Bt* aufweist.

57. Verfahren gemäss einem der Ansprüche 53 oder 56, dadurch gekennzeichnet, dass es sich um ein Kristallprotein der *Bt*-Varietät *kurstaki* HD1 handelt.

FIG.1

FIG.2

FIG.3

FIG.4

FIG.5

FIG.6

FIG.7

FIG.8

FIG.9

FIG.10

FIG.11

FIG.12

FIG.13

FIG.14

FIG.15

FIG.16

FIG.17

FIG.19

FIG.20

FIG.21

FIG.22

FIG.23

ATG

rbc

rbc-gX

BglII · SphI · SphI · XhoI

PstI · BamHI · EcoRI · BglII

ATG

rbc

rbc-gY

SphI · EcoRI · XbaI · EcoRI · BamHI

EcoRI · XhoI · SphI · XbaI

1 kb=2cm

FIG.24

NUCLEOTIDE AND AMINO-ACID SEQUENCES OF RBC-GY

TRANSIT-PETIDE

```
    CTACTAGCAATGGCTTCCTCAATGATCTCATCGGCTACCATTGCCACTGCCTCTCCGGCA
  1 ---------+---------+---------+---------+---------+---------+ 60
    GATGATCGTTACCGAAGGAGTTACTAGAGTAGCCGATGGTAACGGTGACGGAGAGGCCGT

    L  L  A  M  A  S  S  M  I  S  S  A  T  I  A  T  A  S  P  A   -

    CAGGCTAACATGGTCGCTCCTTTCACCGGCCTCAAGTCTGCCTCTGCTTTCCCAGTCATC
 61 ---------+---------+---------+---------+---------+---------+ 120
    GTCCGATTGTACCAGCGAGGAAAGTGGCCGGAGTTCAGACGGAGACGAAAGGGTCAGTAG

    Q  A  N  M  V  A  P  F  T  G  L  K  S  A  S  A  F  P  V  I   -

    AGGAAGGCCAACAACGACATTACTTCTCTCGCAAGCAATGGCGGCAGAGTGCAATGC
121 ---------+---------+---------+---------+---------+-------
    TCCTTCCGGTTGTTGCTGTAATGAAGAGAGCGTTCGTTACCGCCGTCTCACGTTACG

    R  K  A  N  N  D  I  T  S  L  A  S  N  G  G  R  V  Q  C
```

# FIG.25

NUCLEOTIDE AND AMINO-ACID SEQUENCES OF RBC-GX

TRANSIT-PEPTIDE

```
    AAGCAGTAATAGCAATGGCCTCCTCCATGATCTCATCGGCAACCATTGCCACCGTGAACT
  1 ---------+---------+---------+---------+---------+---------+ 60
    TTCGTCATTATCGTTACCGGAGGAGGTACTAGAGTAGCCGTTGGTAACGGTGGCACTTGA

      A    V    I    A ·  M    A    S    S    M    I    S    S    A    T    I    A    T    V    N    C  -

    GCTCCTCCCCGGCACAGGCCAACATGGTGGCCCCCTTCACCGGCCTCAAGTCTGCCTCTG
 61 ---------+---------+---------+---------+---------+---------+ 120
    CGAGGAGGGGCCGTGTCCGGTTGTACCACCGGGGGAAGTGGCCGGAGTTCAGACGGAGAC

      S    S    P    A    Q    A    N    M    V    A    P    F    T    G    L    K    S    A    S    A  -

    CTTTCCCAGTCACTAGGAAGGCCAACAACGACATCACTTCTCTTGCAAGCAATGGTGGGA
121 ---------+---------+---------+---------+---------+---------+ 180
    GAAAGGGTCAGTGATCCTTCCGGTTGTTGCTGTAGTGAAGAGAACGTTCGTTACCACCCT

      F    P    V    T    R    K    A    N    N    D    I    T    S    L    A    S    N    G    G    R  -

    GAGTGCAATGC
181 ---------+-
    CTCACGTTACG

      V    Q    C
```

# FIG.26